# EUROPEAN PATENT APPLICATION

(11) **EP 2 530 165 A1**
(43) Date of publication of application: **05.12.2012**
(21) Application number: 11736901.7
(22) Date of filing: 19.01.2011
(51) Int. Cl.: C12P 17/18, C07D 493/04, C12N 15/09

(54) **METHOD FOR PRODUCING PYRIPYROPENE DERIVATIVE BY ENZYMATIC PROCESS**

(30) Priority: 26.01.2010 JP 2010014727; 26.01.2010 JP 2010014336
(71) Applicant: Meiji Seika Pharma Co., Ltd., Chuo-ku Tokyo (JP)
(72) Inventor: YAMAMOTO Kentaro, Yokohama-shi Kanagawa 222-8567 (JP); TSUCHIDA Mariko, Yokohama-shi Kanagawa 222-8567 (JP); OYAMA Kazuhiko, Yokohama-shi Kanagawa 222-8567 (JP); GOTO Kimihiko, Yokohama-shi Kanagawa 222-8567 (JP); MITOMI Masaaki, Yokohama-shi Kanagawa 222-8567 (JP)
(74) Representative: Regimbeau
(86) International application number: PCT/JP2011/050853
(87) International publication number: WO 2011/093187

(57) **Abstract**

There is provided a method for producing a pyripyropene derivative represented by the following formula A by an enzyme method. The production method of the present invention allows for production of a pyripyropene derivative under simpler conditions and in shorter steps. 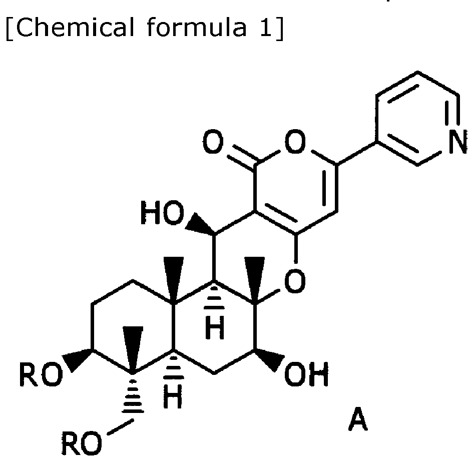 [wherein R represents a linear, branched or cyclic C₂₋₆ alkylcarbonyl group (when the alkyl moiety of this group is branched or cyclic, C₃₋₆ alkyl carbonyl group)].

SEQUENCE LISTING

<110>  Meiji Seika Kaisha Ltd.

<120>  Process for producing Pyripyropene derivatives by the enzymatic method

<130>  187413PX

<150>  JP2010-14727
<151>  2010-01-26

<150>  JP2010-14336
<151>  2010-01-26

<160>  296

<170>  PatentIn version 3.1

<210>  1
<211>  21
<212>  DNA
<213>  Filamentous fungi

<220>
<221>  modified_base
<222>  (6)..(6)
<223>  I

<220>
<221>  modified_base
<222>  (9)..(9)
<223>  I

<400>  1
gayccnmgnt tyttyaayat g                                                21

<210>  2
<211>  20
<212>  DNA
<213>  Filamentous fungi

<220>
<221>  modified_base
<222>  (3)..(3)
<223>  I

<220>
<221>  modified_base
<222>  (6)..(6)
<223>  I

<220>
<221>  modified_base
<222>  (9)..(9)
<223>  I

<400>  2
gtnccngtnc crtgcatytc                                                  20

<210>  3
<211>  500

<212>  DNA
<213>  Filamentous fungi

<400>  3
cattaccgag tgagggccct ctgggtccaa cctcccaccc gtgtttattt accttgttgc      60

ttcggcgggc ccgccttaac tggccgccgg ggggcttacg cccccgggcc cgcgcccgcc     120

gaagacaccc tcgaactctg tctgaagatt gtagtctgag tataaatata aattatttaa     180

aactttcaac aacggatctc ttggttccgg catcgatgaa gaacgcagcg aaatgcgata     240

cgtaatgtga attgcaaatt cagtgaatca tcgagtcttt gaacgcacat tgcgccccct      300

ggtattccgg ggggcatgcc tgtccgagcg tcattgctgc cctcaagccc ggcttgtgtg     360

ttgggccccg tcctccgatt ccgggggacg ggcccgaaag gcagcggcgg caccgcgtcc     420

ggtcctcgag cgtatggggc tttgtcaccc gctctgtagg cccggccggc gcttgccgat     480

caacccaaat tttatccag                                                  500


<210>  4
<211>  28
<212>  PRT
<213>  Penicillium coprobium PF1169

<400>  4

Gln Pro Trp Lys Asp Ser Ile Trp Ala Gly Asp Val Tyr Met Phe Glu
1               5                   10                  15


Gly Asp Asp Ile Val Ala Val Tyr Gly Gly Val Lys
            20                  25


<210>  5
<211>  36
<212>  PRT
<213>  Penicillium coprobium PF1169

<400>  5

His Asn Ser Ile Phe Gln Ala Leu Ala Arg Lys Ile Leu Asp Met Ala
1               5                   10                  15


Leu Pro Pro Gly Gly Gly Ala Pro Ala Pro Ala Pro Ala Ala Lys Arg
            20                  25                  30


Pro Ala Pro Ile
        35


<210>  6
<211>  70
<212>  PRT
<213>  Penicillium coprobium PF1169

<400>  6

Gly Arg Phe Leu Ser Ser Asp Gly Arg Cys His Thr Phe Asp Glu Lys

1                   5                   10                  15

Ala Asn Gly Tyr Ala Arg Gly Glu Ala Val Gly Cys Leu Ile Leu Lys
            20                  25                  30

Pro Leu Ala Lys Ala Leu His Asp Gln Asn Lys Ile Arg Ala Val Ile
        35                  40                  45

Arg Gly Thr Gly Ser Asn Gln Asp Gly Arg Thr Ala Gly Ile Thr Val
    50                  55                  60

Pro Asn Gly Ala Ala Gln
65                  70

<210>  7
<211>  74
<212>  PRT
<213>  Penicillium coprobium PF1169

<400>  7

Arg Ile Ser Tyr Tyr Phe Asp Trp Gln Gly Pro Ser Met Ala Val Asp
1                   5                   10                  15

Thr Gly Cys Ser Ser Ser Leu Leu Ala Val His Leu Gly Val Glu Ala
            20                  25                  30

Leu Gln Asn Asp Asp Cys Ser Met Ala Val Ala Val Gly Ser Asn Leu
        35                  40                  45

Ile Leu Ser Pro Asn Ala Tyr Ile Ala Asp Ser Lys Thr Arg Met Leu
    50                  55                  60

Ser Pro Thr Gly Arg Ser Arg Met Trp Asp
65                  70

<210>  8
<211>  51
<212>  PRT
<213>  Penicillium coprobium PF1169

<400>  8

Ser Ser Phe Leu Thr Ser Thr Val Gln Gln Ile Val Glu Glu Thr Ile
1                   5                   10                  15

Gln Gly Gly Thr Gly Gln Val Val Met Glu Ser Asp Leu Met Gln Thr
            20                  25                  30

Glu Phe Leu Glu Ala Ala Asn Gly His Arg Met Asn Asp Cys Gly Val
        35                  40                  45

Val Thr Ser
    50

<210>  9
<211>  79
<212>  PRT
<213>  Penicillium coprobium PF1169

<400>  9

Phe Asn Ala Ala His Arg Val Leu Pro Leu Pro Ser Tyr Lys Trp Asp
1               5                   10                  15

Leu Lys Asn Tyr Trp Ile Pro Tyr Thr Asn Asn Phe Cys Leu Leu Lys
            20                  25                  30

Gly Ala Pro Ala Ala Pro Val Ala Glu Ala Thr Pro Ile Ser Val Phe
        35                  40                  45

Leu Ser Ser Ala Ala Gln Arg Val Leu Glu Thr Ser Gly Asp Asn Ser
    50                  55                  60

Ser Ala Phe Ile Val Ile Glu Asn Asp Ile Ala Asp Pro Asp Leu
65                  70                  75

<210>  10
<211>  84
<212>  PRT
<213>  Penicillium coprobium PF1169

<400>  10

Val Ile Arg Gly Thr Gly Ser Asn Gln Asp Gly Arg Thr Ala Gly Ile
1               5                   10                  15

Thr Val Pro Asn Gly Ala Ala Gln Glu Ser Leu Ile Arg Ser Val Tyr
            20                  25                  30

Ala Gln Ala Asp Leu Asp Pro Ser Glu Thr Asp Phe Val Glu Ala His
        35                  40                  45

Gly Thr Gly Thr Leu Ala Gly Asp Pro Val Glu Thr Gly Ala Ile Ala
    50                  55                  60

Arg Val Phe Gly Thr Asp Arg Pro Pro Gly Asp Pro Val Arg Ile Gly
65                  70                  75                  80

Ser Ile Lys Thr

<210>  11
<211>  81
<212>  PRT

<213>  Penicillium coprobium PF1169

<400>  11

Gln Glu Ala Lys Ala Met Asp Pro Gln Gln Arg Met Leu Leu Glu Cys
1               5                   10                  15

Thr Tyr Glu Ala Leu Glu Asn Gly Gly Ile Ser Lys Glu Ser Leu Lys
            20                  25                  30

Gly Gln Asn Val Gly Val Phe Val Gly Ser Ala Phe Pro Asp Tyr Glu
        35                  40                  45

Met Tyr Asn Arg Arg Asp Leu Glu Thr Ala Pro Met His Gln Ser Thr
    50                  55                  60

Gly Asn Ala Leu Ala Leu Gln Ser Asn Arg Ile Ser Tyr Tyr Phe Asp
65                  70                  75                  80

Phe

<210>  12
<211>  66
<212>  PRT
<213>  Penicillium coprobium PF1169

<400>  12

Asn His Thr Gly Arg Ala Glu Gln Ser Lys Ile Ala Ile Ile Gly Leu
1               5                   10                  15

Ser Gly Arg Phe Pro Glu Ala Pro Asp Thr Glu Ala Phe Trp Asp Leu
            20                  25                  30

Leu Lys Lys Gly Leu Asp Val His Arg Glu Val Pro Pro Glu Arg Trp
        35                  40                  45

Asp Val Lys Ala His Val Asp Pro Glu Gly Lys Lys Arg Thr Pro Ala
    50                  55                  60

Lys Leu
65

<210>  13
<211>  14
<212>  PRT
<213>  Penicillium coprobium PF1169

<400>  13

Glu Lys Asn Thr Ser Gln Val Glu Tyr Gly Cys Trp Tyr Asn
1               5                   10

<210>  14
<211>  71
<212>  PRT
<213>  Penicillium coprobium PF1169

<400>  14

Ala Gly Gly Asn Thr Thr Val Ala Leu Glu Asp Ala Pro Ile Arg Thr
1               5                   10                  15

Arg Ser Gly Ser Asp Pro Arg Ser Leu His Pro Ile Ala Ile Ser Ala
            20                  25                  30

Lys Ser Lys Val Ser Leu Arg Gly Asn Leu Glu Asn Leu Leu Ala Tyr
        35                  40                  45

Leu Asp Thr His Pro Asp Val Ser Leu Ser Asp Leu Ser Tyr Thr Thr
    50                  55                  60

Thr Ala Arg Arg His His His
65                  70

<210>  15
<211>  77
<212>  PRT
<213>  Penicillium coprobium PF1169

<400>  15

Ala Thr Asp Thr Glu Lys Phe Trp Asp Leu Leu Ala Ser Gly Val Asp
1               5                   10                  15

Val His Arg Lys Ile Pro Ala Asp Arg Phe Asp Val Glu Thr His Tyr
            20                  25                  30

Asp Pro Asn Gly Lys Arg Met Asn Ala Ser His Thr Pro Tyr Gly Cys
        35                  40                  45

Phe Ile Asp Glu Pro Gly Leu Phe Asp Ala Ala Phe Phe Asn Met Ser
    50                  55                  60

Pro Arg Glu Ala Gln Gln Thr Asp Pro Met Gln Arg Leu
65                  70                  75

<210>  16
<211>  39
<212>  PRT
<213>  Penicillium coprobium PF1169

<400>  16

Pro Glu Tyr Ser Gln Pro Leu Cys Thr Ala Ile Gln Ile Ala Leu Val
1               5                   10                  15

Glu Leu Leu Glu Ser Phe Gly Val Val Pro Lys Ala Val Val Gly His
            20                  25                  30

Ser Ser Gly Glu Ile Ala Ala
        35

<210>  17
<211>  71
<212>  PRT
<213>  Penicillium coprobium PF1169

<400>  17

Arg Arg Thr Phe Leu Pro Trp Arg Leu Thr Ser Ser Ala Leu Ser Gly
1               5                   10                  15

Gln Glu Leu Thr Gln Ser Leu Ala Ile Asp Ala Val Pro Ile Arg Ser
            20                  25                  30

Ser Lys Glu Pro Thr Val Gly Phe Val Phe Thr Gly Gln Gly Ala Gln
        35                  40                  45

Trp His Gly Met Gly Lys Glu Leu Leu Ser Thr Tyr Pro Ile Phe Arg
    50                  55                  60

Gln Thr Met Gln Asp Val Asp
65                  70

<210>  18
<211>  75
<212>  PRT
<213>  Penicillium coprobium PF1169

<400>  18

Leu Arg Arg Leu Leu His Ala Lys Asn Asp Ser Leu Val Ala Ala Phe
1               5                   10                  15

Phe Gln Lys Thr Tyr Cys Ala Leu Arg Lys Glu Ile Thr Ser Leu Pro
            20                  25                  30

Pro Ser Glu Arg Gln Val Phe Pro Arg Phe Thr Ser Ile Val Asp Leu
        35                  40                  45

Leu Ala Arg Phe Lys Glu Phe Gly Pro Asn Pro Ala Leu Glu Ser Ala
    50                  55                  60

Leu Thr Thr Ile Tyr Gln Leu Gly Cys Phe Ile
65                  70                  75

<210>  19
<211>  81

<212>  PRT
<213>  Penicillium coprobium PF1169

<400>  19

Phe Asp Ala Ala Phe Phe Asn Met Ser Pro Arg Glu Ala Gln Gln Thr
1               5                   10                  15

Asp Pro Met Gln Arg Leu Ala Ile Val Thr Ala Tyr Glu Ala Leu Glu
            20                  25                  30

Arg Ala Gly Tyr Val Ala Asn Arg Thr Ala Ala Thr Asn Leu His Arg
        35                  40                  45

Ile Gly Thr Phe Tyr Gly Gln Ala Ser Asp Asp Tyr Arg Glu Val Asn
    50                  55                  60

Thr Ala Gln Glu Ile Ser Thr Tyr Phe Ile Pro Gly Gly Cys Arg Ala
65                  70                  75                  80

Phe

<210>  20
<211>  38
<212>  PRT
<213>  Penicillium coprobium PF1169

<400>  20

Asp Thr Ala Cys Ser Ser Ser Leu Val Ala Leu His Tyr Ala Val Gln
1               5                   10                  15

Ser Leu Arg Asn Gly Glu Ser Thr Glu Ala Leu Ile Ala Gly Cys His
            20                  25                  30

Leu Asn Ile Val Pro Asp
        35

<210>  21
<211>  75
<212>  PRT
<213>  Penicillium coprobium PF1169

<400>  21

Ala Lys His Pro Pro Ala Thr Ser Ile Leu Leu Gln Gly Asn Pro Lys
1               5                   10                  15

Thr Ala Thr Gln Ser Leu Phe Leu Phe Pro Asp Gly Ser Gly Ser Ala
            20                  25                  30

Thr Ser Tyr Ala Thr Ile Pro Gly Ile Ser Pro Asp Val Cys Val Tyr
        35                  40                  45

Gly Leu Asn Cys Pro Tyr Met Arg Thr Pro Glu Lys Leu Lys Phe Ser
    50                  55                  60

Leu Asp Glu Leu Thr Ala Pro Tyr Val Ala Glu
65                  70                  75

<210>  22
<211>  38
<212>  PRT
<213>  Penicillium coprobium PF1169

<400>  22

Gly Asn Gly Ser Ala Met Ile Ser Asn Arg Ile Ser Trp Phe Phe Asp
1               5                   10                  15

Leu Lys Gly Pro Ser Leu Ser Leu Asp Thr Ala Cys Ser Ser Ser Leu
            20                  25                  30

Val Ala Leu His Leu Ala
        35

<210>  23
<211>  57
<212>  PRT
<213>  Penicillium coprobium PF1169

<400>  23

Ala Ile Arg Asp Glu Val Arg Gln Leu Pro Thr Pro Leu Arg Ala Leu
1               5                   10                  15

Val Pro Ala Phe Glu Asn Val Leu Glu Leu Ala Asn Tyr Thr Asp Leu
            20                  25                  30

Arg Lys Gly Pro Leu Ser Gly Ser Ile Asp Gly Val Leu Leu Cys Val
        35                  40                  45

Val Gln Leu Ser Ser Leu Ile Gly Tyr
    50                  55

<210>  24
<211>  74
<212>  PRT
<213>  Penicillium coprobium PF1169

<400>  24

Ala Val Ala Trp Asp Pro Gln Gln Arg Ile Leu Leu Glu Val Val Tyr
1               5                   10                  15

Glu Ala Leu Glu Ser Ala Gly Tyr Phe Arg Ala Gly Ile Lys Pro Glu
            20                  25                  30

Leu Asp Asp Tyr Gly Cys Tyr Ile Gly Ala Val Met Asn Asn Tyr Tyr
        35                  40                  45

Asp Asn Met Ser Cys Gln Pro Thr Thr Ala Tyr Ala Thr Val Gly Thr
    50                  55                  60

Ser Arg Cys Phe Leu Ser Gly Cys Val Ser
65                  70

<210>  25
<211>  52
<212>  PRT
<213>  Penicillium coprobium PF1169

<400>  25

Gly Val Ile Val Gly Ser Ala Ala Asn Gln Asn Leu Asn Leu Ser His
1               5                   10                  15

Ile Thr Val Pro His Ser Gly Ser Gln Val Lys Leu Tyr Gln Asn Val
            20                  25                  30

Met Ser Gln Ala Gly Val His Pro His Ser Val Thr Tyr Val Glu Ala
        35                  40                  45

His Gly Thr Gly
    50

<210>  26
<211>  57
<212>  PRT
<213>  Penicillium coprobium PF1169

<400>  26

Trp Arg Ile Thr Val Ala Ile Val Gly Gly Val Asn Ala Leu Cys Gly
1               5                   10                  15

Pro Gly Leu Thr Arg Val Leu Asp Lys Ala Gly Ala Ile Ser Ser Asp
            20                  25                  30

Gly Ser Cys Lys Ser Phe Asp Asp Asp Ala His Gly Tyr Ala Arg Gly
        35                  40                  45

Glu Gly Ala Gly Ala Leu Val Leu Lys
    50                  55

<210>  27
<211>  78
<212>  PRT
<213>  Penicillium coprobium PF1169

<400>  27

Leu Ile Asp Asp Thr Thr Val Trp Ile Glu Ile Gly Pro His Pro Val
1               5                   10                  15

Cys Leu Gly Phe Val Lys Ala Thr Leu Glu Ser Val Ala Val Ala Val
            20                  25                  30

Pro Ser Leu Arg Arg Gly Glu Asn Ala Trp Cys Thr Leu Ala Gln Ser
        35                  40                  45

Leu Thr Thr Leu His Asn Ala Gly Val Pro Val Gly Trp Ser Glu Phe
    50                  55                  60

His Arg Pro Phe Glu Arg Ala Leu Cys Leu Leu Asp Leu Pro
65                  70                  75

<210>  28
<211>  65
<212>  PRT
<213>  Penicillium coprobium PF1169

<400>  28

Val Trp Ile Glu Ile Gly Pro His Pro Val Cys Leu Gly Phe Val Lys
1               5                   10                  15

Ala Thr Leu Glu Ser Val Ala Val Ala Val Pro Ser Leu Arg Arg Gly
            20                  25                  30

Glu Asn Ala Trp Cys Thr Leu Ala Gln Ser Leu Thr Thr Leu His Asn
        35                  40                  45

Ala Gly Val Pro Val Gly Trp Ser Glu Phe His Arg Pro Phe Glu Arg
    50                  55                  60

Ala
65

<210>  29
<211>  83
<212>  PRT
<213>  Penicillium coprobium PF1169

<400>  29

Thr Ser Asp Asp Tyr Arg Glu Val Asn Ser Gly Gln Asp Ile Asp Thr
1               5                   10                  15

Tyr Phe Ile Pro Gly Gly Asn Arg Ala Phe Thr Pro Gly Arg Ile Asn
            20                  25                  30

Tyr Tyr Phe Lys Phe Ser Gly Pro Ser Val Ser Val Asp Thr Ala Cys

35                  40                  45

Ser Ser Ser Leu Ala Ala Ile His Val Ala Cys Asn Ser Leu Trp Arg
    50                  55                  60

Asn Glu Ser Asp Ser Ala Val Ala Gly Gly Val Asn Ile Leu Thr Asn
65                  70                  75                  80

Pro Asp Asn

<210>  30
<211>  54
<212>  PRT
<213>  Penicillium coprobium PF1169

<400>  30

Gly Arg Phe Leu Ser Ser Asp Gly Arg Cys His Thr Phe Asp Glu Lys
1               5                   10                  15

Ala Asn Gly Tyr Ala Arg Gly Glu Ala Val Gly Cys Leu Ile Leu Lys
            20                  25                  30

Pro Leu Ala Lys Ala Leu His Asp Gln Asn Lys Ile Arg Ala Val Ile
        35                  40                  45

Arg Gly Thr Gly Ser Asn
    50

<210>  31
<211>  63
<212>  PRT
<213>  Penicillium coprobium PF1169

<400>  31

Asp Thr Ala Cys Ser Ser Ser Leu Tyr Ala Leu His Ser Ala Cys Leu
1               5                   10                  15

Ala Leu Asp Ser Arg Asp Cys Asp Gly Ala Val Val Ala Ala Ala Asn
            20                  25                  30

Leu Ile Gln Ser Pro Glu Gln Gln Met Ile Ala Val Lys Ala Gly Ile
        35                  40                  45

Leu Ser Pro Asp Ser Met Cys His Thr Phe Asp Glu Ser Ala Asn
    50                  55                  60

<210>  32
<211>  55
<212>  PRT
<213>  Penicillium coprobium PF1169

<400>  32

Lys Gln Thr Thr Ser Arg Gly Tyr Phe Leu Asp His Leu Glu Asp Phe
1               5                   10                  15

Asp Cys Gln Phe Phe Gly Ile Ser Pro Lys Glu Ala Glu Gln Met Asp
            20                  25                  30

Pro Gln Gln Arg Val Ser Leu Glu Val Ala Ser Glu Ala Leu Glu Asp
        35                  40                  45

Ala Gly Ile Pro Ala Lys Ser
    50                  55

<210>  33
<211>  38
<212>  PRT
<213>  Penicillium coprobium PF1169

<400>  33

Pro Val Gly Cys Arg Ala Phe Gly Pro Gly Arg Ile Asn Tyr Phe Phe
1               5                   10                  15

Lys Phe Ser Gly Pro Ser Phe Ser Ile Asp Thr Ala Cys Ser Ser Ser
            20                  25                  30

Leu Ala Thr Ile Gln Val
        35

<210>  34
<211>  18
<212>  PRT
<213>  Penicillium coprobium PF1169

<400>  34

Ala Cys Thr Ser Leu Trp Asn Gly Glu Thr Asp Thr Val Val Ala Gly
1               5                   10                  15

Gly Met

<210>  35
<211>  12
<212>  PRT
<213>  Penicillium coprobium PF1169

<400>  35

Thr Ala Gln Glu Ile Ser Thr Tyr Phe Ile Pro Gly
1               5                   10

<210>  36

<211>  39
<212>  PRT
<213>  Penicillium coprobium PF1169

<400>  36

Pro Glu Tyr Ser Gln Pro Leu Cys Thr Ala Ile Gln Ile Ala Leu Val
1               5                   10                  15

Glu Leu Leu Glu Ser Phe Gly Val Val Pro Lys Ala Val Val Gly His
            20                  25                  30

Ser Ser Gly Glu Ile Ala Ala
        35

<210>  37
<211>  36
<212>  PRT
<213>  Penicillium coprobium PF1169

<400>  37

Ile Ser Gln Pro Ala Cys Thr Ala Leu Gln Ile Ala Leu Val Asp Leu
1               5                   10                  15

Leu Ala Glu Trp Ser Ile Thr Pro Ser Val Val Val Gly His Ser Ser
            20                  25                  30

Gly Glu Ile Ala
        35

<210>  38
<211>  39
<212>  PRT
<213>  Penicillium coprobium PF1169

<400>  38

Pro Glu Tyr Ser Gln Pro Leu Cys Thr Ala Ile Gln Ile Ala Leu Val
1               5                   10                  15

Glu Leu Leu Glu Ser Phe Gly Val Val Pro Lys Ala Val Val Gly His
            20                  25                  30

Ser Ser Gly Glu Ile Ala Ala
        35

<210>  39
<211>  76
<212>  PRT
<213>  Penicillium coprobium PF1169

<400>  39

Glu Glu Phe Trp Asp Leu Cys Ser Arg Gly Arg Gly Ala Trp Ser Pro
1               5                   10                  15

Val Pro Lys Asp Arg Phe Asn Ala Gly Ser Phe Tyr His Pro Asn Ala
            20                  25                  30

Asp Arg Pro Gly Ser Phe Asn Ala Ala Gly Ala His Phe Leu Thr Glu
        35                  40                  45

Asp Ile Gly Leu Phe Asp Ala Pro Phe Phe Asn Ile Thr Leu Gln Glu
    50                  55                  60

Ala Gln Thr Met Asp Pro Gln Gln Arg Ile Phe Leu
65                  70                  75

<210> 40
<211> 77
<212> PRT
<213> Penicillium coprobium PF1169

<400> 40

Ile Asn Glu Pro Arg Asp Arg Pro Gln Phe Phe His Ala His Gly Thr
1               5                   10                  15

Gly Thr Gln Ala Gly Asp Pro Gln Glu Ala Glu Ala Val Ser Thr Ala
            20                  25                  30

Leu Phe Pro Asp Gly Ser Asn Ile Glu Thr Lys Leu Phe Val Gly Ser
        35                  40                  45

Ile Lys Thr Val Ile Gly His Thr Glu Gly Ser Ala Gly Leu Ala Ser
    50                  55                  60

Leu Ile Gly Ser Ser Leu Ala Met Lys His Gly Val Ile
65                  70                  75

<210> 41
<211> 43
<212> PRT
<213> Penicillium coprobium PF1169

<400> 41

Lys Leu Ala Phe Val Phe Thr Gly Gln Gly Gly Gln Trp Ala Gly Met
1               5                   10                  15

Gly Arg Glu Leu Leu Ser Ile Ser Thr Phe Arg Glu Ser Met Ala Arg
            20                  25                  30

Ser Gln Glu Ile Leu Ala Ser Leu Gly Cys Pro
        35                  40

<210> 42

<211>  71
<212>  PRT
<213>  Penicillium coprobium PF1169

<400>  42

Lys Ser Phe Leu Asp Asp Leu Ala Phe Thr Val Asn Glu Arg Arg Ser
1               5                   10                  15

Ile Phe Pro Trp Lys Ala Ala Val Val Gly Asp Thr Met Glu Gly Leu
            20                  25                  30

Ala Ala Ser Leu Ala Gln Asn Ile Lys Pro Arg Ser Val Leu Arg Met
        35                  40                  45

Pro Thr Leu Gly Phe Val Phe Thr Gly Gln Gly Ala Gln Trp Pro Gly
    50                  55                  60

Met Gly Lys Glu Leu Leu Gln
65                  70

<210>  43
<211>  55
<212>  PRT
<213>  Penicillium coprobium PF1169

<400>  43

Ser Val Ala Cys Ile Asn Ser Pro Phe Asn Cys Thr Leu Ser Gly Pro
1               5                   10                  15

Glu Glu Asp Ile Asp Ala Val Lys Ala Gln Ala Asp Gln Asp Gly Leu
            20                  25                  30

Phe Ala Gln Lys Leu Lys Thr Gly Val Ala Tyr His Ser Thr Ala Met
        35                  40                  45

Ser Ala Ile Ala Asn Asp Tyr
    50                  55

<210>  44
<211>  68
<212>  PRT
<213>  Penicillium coprobium PF1169

<400>  44

Met Leu Ala Val Gly Ala Ser Ala Ser Asp Ile Gln Gln Ile Leu Asp
1               5                   10                  15

Ala Met Arg Gly Asn Lys Ala Val Ile Ala Cys Val Asn Ser Glu Ser
            20                  25                  30

Ser Val Thr Leu Ser Gly Asp Leu Asp Val Ile Ala Asn Leu Gln Thr

35                  40                  45

Ala Leu Asp Lys Glu Gly Ile Phe Thr Arg Lys Leu Lys Val Asp Val
    50                  55                  60

Ala Tyr His Ser
65

<210>  45
<211>  38
<212>  PRT
<213>  Penicillium coprobium PF1169

<400>  45

Gly Asn Gly Ser Ala Met Ile Ser Asn Arg Ile Ser Trp Phe Phe Asp
1               5                   10                  15

Leu Lys Gly Pro Ser Leu Ser Leu Asp Thr Ala Cys Ser Ser Ser Leu
            20                  25                  30

Val Ala Leu His Leu Ala
        35

<210>  46
<211>  77
<212>  PRT
<213>  Penicillium coprobium PF1169

<400>  46

Gly Pro Ser Met Thr Ile Asp Thr Ala Cys Ser Ser Ser Leu Ile Ala
1               5                   10                  15

Leu His Gln Ala Val Gln Ser Leu Arg Ser Gly Glu Thr Asp Val Ala
            20                  25                  30

Val Ala Ala Gly Thr Asn Leu Leu Leu Gly Pro Glu Gln Tyr Ile Ala
        35                  40                  45

Glu Ser Lys Leu Lys Met Leu Ser Pro Asn Gly Arg Ser Arg Met Trp
    50                  55                  60

Asp Lys Asp Ala Asp Gly Tyr Ala Arg Gly Asp Gly Ile
65                  70                  75

<210>  47
<211>  61
<212>  PRT
<213>  Penicillium coprobium PF1169

<400>  47

Ile Gly Ser Ile Lys Pro Asn Ile Gly His Leu Glu Ala Gly Ala Gly

1               5                   10                  15

Val Met Gly Phe Ile Lys Ala Ile Leu Ser Ile Gln Lys Gly Val Leu
            20                  25                  30

Ala Pro Gln Ala Asn Leu Thr Lys Leu Asn Ser Arg Ile Asp Trp Lys
        35                  40                  45

Thr Ala Gly Val Lys Val Val Gln Glu Ala Thr Pro Trp
    50                  55                  60

<210>  48
<211>  37
<212>  PRT
<213>  Penicillium coprobium PF1169

<400>  48

Gly Leu Phe Asp Ala Pro Phe Phe Asn Ile Thr Leu Gln Glu Ala Gln
1               5                   10                  15

Thr Met Asp Pro Gln Gln Arg Ile Phe Leu Glu Cys Val Tyr Glu Ala
            20                  25                  30

Leu Glu Asn Gly Gly
        35

<210>  49
<211>  70
<212>  PRT
<213>  Penicillium coprobium PF1169

<400>  49

Gly Arg Phe Leu Ser Ser Asp Gly Arg Cys His Thr Phe Asp Glu Lys
1               5                   10                  15

Ala Asn Gly Tyr Ala Arg Gly Glu Ala Val Gly Cys Leu Ile Leu Lys
            20                  25                  30

Pro Leu Ala Lys Ala Leu His Asp Gln Asn Lys Ile Arg Ala Val Ile
        35                  40                  45

Arg Gly Thr Gly Ser Asn Gln Asp Gly Arg Thr Ala Gly Ile Thr Val
    50                  55                  60

Pro Asn Gly Ala Ala Gln
65                  70

<210>  50
<211>  37
<212>  PRT
<213>  Penicillium coprobium PF1169

<400>  50

Ser Phe Asp Ser Arg Ala Glu Gly Tyr Ala Arg Gly Glu Gly Val Gly
1               5                   10                  15

Thr Val Val Val Lys Pro Leu Ser Thr Ala Ile Arg Asp Gly Asp Thr
            20                  25                  30

Ile Arg Ala Val Ile
        35

<210>  51
<211>  83
<212>  PRT
<213>  Penicillium coprobium PF1169

<400>  51

Trp Pro Arg Leu Pro Glu Arg Arg Arg Ile Ala Val Val Asn Asn Phe
1               5                   10                  15

Ser Ala Ala Gly Gly Asn Thr Thr Val Ala Leu Glu Asp Ala Pro Ile
            20                  25                  30

Arg Thr Arg Ser Gly Ser Asp Pro Arg Ser Leu His Pro Ile Ala Ile
        35                  40                  45

Ser Ala Lys Ser Lys Val Ser Leu Arg Gly Asn Leu Glu Asn Leu Leu
    50                  55                  60

Ala Tyr Leu Asp Thr His Pro Asp Val Ser Leu Ser Asp Leu Ser Tyr
65                  70                  75                  80

Thr Thr Thr

<210>  52
<211>  59
<212>  PRT
<213>  Penicillium coprobium PF1169

<400>  52

Val Tyr Ser Gly Ser Met Thr Asn Asp Tyr Glu Leu Leu Ser Thr Arg
1               5                   10                  15

Asp Ile Tyr Asp Met Pro His Asn Ser Ala Thr Gly Asn Gly Arg Thr
            20                  25                  30

Met Leu Ala Asn Arg Leu Ser Trp Phe Phe Asp Leu Gln Gly Pro Ser
        35                  40                  45

Ile Met Met Asp Thr Ala Cys Ser Ser Ser Leu
    50                  55

<210>  53
<211>  67
<212>  PRT
<213>  Penicillium coprobium PF1169

<400>  53

Leu Ser Pro Gln Asn Asn Pro Glu Asp Arg Cys Gln Tyr Phe Glu Ala
1               5                   10                  15

His Gly Thr Gly Thr Gln Ala Gly Asp Pro Gln Glu Ala Ala Ala Ile
            20                  25                  30

Asn Ser Ser Phe Phe Gly Pro Glu Ser Val Pro Asp Ser Thr Asp Arg
        35                  40                  45

Leu Tyr Val Gly Ser Ile Lys Thr Ile Ile Gly His Thr Glu Ala Thr
    50                  55                  60

Ala Gly Leu
65

<210>  54
<211>  83
<212>  PRT
<213>  Penicillium coprobium PF1169

<400>  54

Asp Gly Tyr Gly Arg Gly Glu Gly Val Ala Ser Val Val Leu Lys Arg
1               5                   10                  15

Leu Gln Asp Ala Ile Asn Asp Gly Asp Pro Ile Glu Cys Val Ile Arg
            20                  25                  30

Ala Ser Gly Ala Asn Ser Asp Gly Arg Thr Met Gly Ile Thr Met Pro
        35                  40                  45

Asn Pro Lys Ala Gln Gln Ser Leu Ile Leu Ala Thr Tyr Ala Arg Ala
    50                  55                  60

Gly Leu Ser Pro Gln Asn Asn Pro Glu Asp Arg Cys Gln Tyr Phe Glu
65                  70                  75                  80

Ala His Gly

<210>  55
<211>  38
<212>  PRT

<213>  Penicillium coprobium PF1169

<400>  55

Met Leu Ala Val Gly Ala Ser Ala Ser Asp Ile Gln Gln Ile Leu Asp
1               5                   10                  15

Ala Met Arg Gly Asn Lys Ala Val Ile Ala Cys Val Asn Ser Glu Ser
            20                  25                  30

Ser Val Thr Leu Ser Gly
        35

<210>  56
<211>  29
<212>  PRT
<213>  Penicillium coprobium PF1169

<400>  56

Ser Gly Cys Tyr Arg Glu Leu Ala Asp Cys Pro Gly Gln Arg Gly Ile
1               5                   10                  15

Phe Thr Arg Lys Leu Lys Val Asp Val Ala Tyr His Ser
            20                  25

<210>  57
<211>  38
<212>  PRT
<213>  Penicillium coprobium PF1169

<400>  57

Gly Asn Gly Ser Ala Met Ile Ser Asn Arg Ile Ser Trp Phe Phe Asp
1               5                   10                  15

Leu Lys Gly Pro Ser Leu Ser Leu Asp Thr Ala Cys Ser Ser Ser Leu
            20                  25                  30

Val Ala Leu His Leu Ala
        35

<210>  58
<211>  59
<212>  PRT
<213>  Penicillium coprobium PF1169

<400>  58

Ile Ser Glu Cys Val Thr Val Tyr Trp Lys Ala Ile Lys Ser Ala Gln
1               5                   10                  15

Pro Asp Gly Pro Tyr Ala Leu Ala Gly Tyr Ser Tyr Gly Ser Met Leu
            20                  25                  30

Ala Phe Glu Val Ala Lys Leu Leu Ile Lys Asn Gly Asp Lys Val Asp
        35                  40                  45

Phe Leu Gly Cys Phe Asn Leu Pro Pro His Ile
    50                  55

<210>  59
<211>  72
<212>  PRT
<213>  Penicillium coprobium PF1169

<400>  59

Gly Ala Ala Val Gln Leu Val Ile Glu Gly Gly Asn Gln Pro Lys Gly
1               5                   10                  15

Ala Met Met Ala Val Gly Ala Asn Ala Ser Thr Val Gln Pro Leu Leu
            20                  25                  30

Asp Ala Met Lys Asp Lys His Ala Val Val Ala Cys Ile Asn Ser Asp
        35                  40                  45

Ser Ser Ile Thr Val Ser Gly Asp Glu Thr Ala Ile Glu Asp Leu Glu
    50                  55                  60

Ser Val Leu Lys Arg Gln Asp Ile
65                  70

<210>  60
<211>  79
<212>  PRT
<213>  Penicillium coprobium PF1169

<400>  60

Ser Val Pro Ile Glu Glu His Ser Pro Val Val Thr Gln Leu Gly Thr
1               5                   10                  15

Thr Cys Val Gln Met Ala Leu Thr Lys Tyr Trp Thr Ser Leu Gly Val
            20                  25                  30

Thr Pro Ser Phe Val Met Gly His Ser Leu Gly Glu Phe Ala Ala Leu
        35                  40                  45

Asn Ala Ala Gly Val Leu Thr Ile Ser Asp Thr Ile Tyr Leu Ala Gly
    50                  55                  60

Arg Arg Ala Gln Leu Leu Thr Glu Gln Ile Lys Val Gly Thr His
65                  70                  75

<210>  61
<211>  67
<212>  PRT

<213>  Penicillium coprobium PF1169

<400>  61

Phe Ile Glu Asp Ser Ile Ser Lys Glu His Lys Pro Thr Arg Val Pro
1               5                   10                  15

Ile His Gly Pro Tyr His Ala Ser His Leu Tyr Asn Asp Arg Asp Ile
            20                  25                  30

Asp Arg Ile Met Glu Ser Trp Pro Thr Glu Gln Leu Trp Ala Tyr Val
        35                  40                  45

Pro Gln Ile Pro Val Leu Ser Thr Gln Thr Gly Lys Ala Phe Gln Ala
    50                  55                  60

Asp Ser Leu
65

<210>  62
<211>  76
<212>  PRT
<213>  Penicillium coprobium PF1169

<400>  62

Gly Pro Ser Met Thr Ile Asp Thr Ala Cys Ser Ser Ser Leu Ile Ala
1               5                   10                  15

Leu His Gln Ala Val Gln Ser Leu Arg Ser Gly Glu Thr Asp Val Ala
            20                  25                  30

Val Ala Ala Gly Thr Asn Leu Leu Leu Gly Pro Glu Gln Tyr Ile Ala
        35                  40                  45

Glu Ser Lys Leu Lys Met Leu Ser Pro Asn Gly Arg Ser Arg Met Trp
    50                  55                  60

Asp Lys Asp Ala Asp Gly Tyr Ala Arg Gly Asp Gly
65                  70                  75

<210>  63
<211>  31
<212>  PRT
<213>  Penicillium coprobium PF1169

<400>  63

Leu Phe Leu Phe Pro Asp Gly Ser Gly Ser Ala Thr Ser Tyr Ala Thr
1               5                   10                  15

Ile Pro Gly Ile Ser Pro Asp Val Cys Val Tyr Gly Leu Asn Cys
            20                  25                  30

<210>  64
<211>  26
<212>  PRT
<213>  Penicillium coprobium PF1169

<400>  64

Ala Lys His Pro Pro Ala Thr Ser Ile Leu Leu Gln Gly Asn Pro Lys
1               5                   10                  15

Thr Ala Thr Gln Ser Phe Ile Phe Val Pro
            20                  25

<210>  65
<211>  46
<212>  PRT
<213>  Penicillium coprobium PF1169

<400>  65

Tyr Gln Ala Thr Gly Cys Ala Ala Ser Leu Gln Ser Asn Arg Ile Ser
1               5                   10                  15

Tyr Phe Phe Asp Leu Arg Gly Pro Ser Ile Thr Ile Asp Thr Ala Cys
            20                  25                  30

Ser Ser Ser Leu Val Ala Leu His Tyr Ala Val Gln Ser Leu
        35                  40                  45

<210>  66
<211>  66
<212>  PRT
<213>  Penicillium coprobium PF1169

<400>  66

Tyr Ser Ala Thr Gly Ser Gly Leu Thr Val Leu Ala Asn Arg Ile Thr
1               5                   10                  15

His Cys Phe Asp Leu Arg Gly Pro Ser His Val Val Asp Thr Ala Cys
            20                  25                  30

Ser Ser Ser Leu Tyr Ala Leu His Ser Ala Cys Leu Ala Leu Asp Ser
        35                  40                  45

Arg Asp Cys Asp Gly Ala Val Val Ala Ala Ala Asn Leu Ile Gln Ser
    50                  55                  60

Pro Glu
65

<210>  67
<211>  76
<212>  PRT

<213>  Penicillium coprobium PF1169

<400>  67

Ser Val Pro Ile Glu Glu His Ser Pro Val Val Thr Gln Leu Gly Thr
1               5                   10                  15

Thr Cys Val Gln Met Ala Leu Thr Lys Tyr Trp Thr Ser Leu Gly Val
            20                  25                  30

Thr Pro Ser Phe Val Met Gly His Ser Leu Gly Glu Phe Ala Ala Leu
        35                  40                  45

Asn Ala Ala Gly Val Leu Thr Ile Ser Asp Thr Ile Tyr Leu Ala Gly
    50                  55                  60

Arg Arg Ala Gln Leu Leu Thr Glu Gln Ile Lys Val
65                  70                  75

<210>  68
<211>  71
<212>  PRT
<213>  Penicillium coprobium PF1169

<400>  68

His Leu Asn Leu Met Gly Pro Ser Thr Ala Val Asp Ala Ala Cys Ala
1               5                   10                  15

Ser Ser Leu Val Ala Ile His His Gly Val Gln Ala Ile Lys Leu Gly
            20                  25                  30

Glu Ser Arg Val Ala Ile Val Gly Gly Val Asn Ala Leu Cys Gly Pro
        35                  40                  45

Gly Leu Thr Arg Val Leu Asp Lys Ala Gly Ser Ile Ser Ser Asp Gly
    50                  55                  60

Ser Cys Lys Ser Phe Asp Asp
65                  70

<210>  69
<211>  84
<212>  PRT
<213>  Penicillium coprobium PF1169

<400>  69

Ser Phe Arg Arg Gln Glu Asp Thr Trp Lys Val Leu Ser Asn Ala Thr
1               5                   10                  15

Ser Thr Leu Tyr Leu Ala Gly Ile Glu Ile Lys Trp Lys Glu Tyr His
            20                  25                  30

Gln Asp Phe Asn Ala Ala His Arg Val Leu Pro Leu Pro Ser Tyr Lys
        35                  40                  45

Trp Asp Leu Lys Asn Tyr Trp Ile Pro Tyr Thr Asn Asn Phe Cys Leu
    50                  55                  60

Leu Lys Gly Ala Pro Ala Ala Pro Val Ala Glu Ala Thr Pro Ile Ser
65                  70                  75                  80

Val Phe Leu Ser

<210>  70
<211>  78
<212>  PRT
<213>  Penicillium coprobium PF1169

<400>  70

Lys Thr Ser Cys Phe Val Gly Ser Phe Ser Ala Asp Tyr Thr Asp Leu
1               5                   10                  15

Leu Leu Arg Asp Pro Glu Cys Val Pro Met Tyr Gln Cys Thr Asn Ala
            20                  25                  30

Gly Gln Ser Arg Ala Met Thr Ala Asn Arg Leu Ser Tyr Phe Phe Asp
        35                  40                  45

Leu Lys Gly Pro Ser Val Thr Val Asp Thr Ala Cys Ser Gly Ser Leu
    50                  55                  60

Val Ala Leu His Leu Ala Cys Gln Ser Leu Arg Thr Gly Asp
65                  70                  75

<210>  71
<211>  67
<212>  PRT
<213>  Penicillium coprobium PF1169

<400>  71

Tyr Ser Ala Thr Gly Ser Gly Leu Thr Val Leu Ala Asn Arg Ile Thr
1               5                   10                  15

His Cys Phe Asp Leu Arg Gly Pro Ser His Val Val Asp Thr Ala Cys
            20                  25                  30

Ser Ser Ser Leu Tyr Ala Leu His Ser Ala Cys Phe Gly Pro Leu Asn
        35                  40                  45

Ser Arg Asp Cys Asp Gly Ala Val Val Ala Ala Ala Asn Leu Ile Gln
    50                  55                  60

Ser Pro Glu
65

<210> 72
<211> 79
<212> PRT
<213> Penicillium coprobium PF1169

<400> 72

Ser Val Pro Ile Glu Glu His Ser Pro Val Val Thr Gln Leu Gly Thr
1               5                   10                  15

Thr Cys Val Gln Met Ala Leu Thr Lys Tyr Trp Thr Ser Leu Gly Val
            20                  25                  30

Thr Pro Ser Phe Val Met Gly His Ser Leu Gly Glu Phe Ala Ala Leu
        35                  40                  45

Asn Ala Ala Gly Val Leu Thr Ile Ser Asp Thr Ile Tyr Leu Ala Gly
    50                  55                  60

Arg Arg Ala Gln Leu Leu Thr Glu Gln Ile Glu Gly Gly Thr His
65                  70                  75

<210> 73
<211> 40
<212> PRT
<213> Penicillium coprobium PF1169

<400> 73

Glu Ala Asn Leu His Val Pro Leu Glu Pro Thr Pro Trp Pro Ala Gly
1               5                   10                  15

Arg Pro Glu Arg Ile Ser Val Asn Ser Phe Gly Ile Gly Gly Ser Asn
            20                  25                  30

Ala His Ala Ile Leu Glu Ser Ala
        35                  40

<210> 74
<211> 70
<212> PRT
<213> Penicillium coprobium PF1169

<220>
<221> misc_feature
<222> (5)..(5)
<223> Xaa can be any naturally occurring amino acid

<400> 74

Ile Gly His Thr Xaa Gly Ser Ala Gly Leu Ala Ser Leu Ile Gly Ser
1               5                   10                  15

Ser Leu Ala Met Lys His Gly Val Ile Pro Pro Asn Leu His Phe Gly
            20                  25                  30

Gln Leu Ser Glu Lys Val Ala Pro Phe Tyr Thr His Leu Asn Ile Pro
        35                  40                  45

Thr Glu Pro Val Pro Trp Pro Asn Ser Thr Ser Ser Gln Val Lys Arg
    50                  55                  60

Ala Ser Ile Asn Ser Phe
65                  70

<210>  75
<211>  45
<212>  PRT
<213>  Penicillium coprobium PF1169

<400>  75

Pro Val Cys Ser Gly Met Val Lys Ala Thr Phe Gly Pro Gln Ala Thr
1               5                   10                  15

Thr Val Ala Ser Phe Arg Arg Gln Glu Asp Thr Trp Lys Val Leu Ser
            20                  25                  30

Asn Ala Thr Ser Thr Leu Tyr Leu Ala Gly Ile Glu Ile
        35                  40                  45

<210>  76
<211>  19
<212>  PRT
<213>  Penicillium coprobium PF1169

<220>
<221>  misc_feature
<222>  (13)..(13)
<223>  Xaa can be any naturally occurring amino acid

<400>  76

Leu Leu Gly Leu Arg Leu Lys Trp Lys Glu Tyr His Xaa Asp Phe Asn
1               5                   10                  15

Ala Ala His

<210>  77
<211>  69
<212>  PRT
<213>  Penicillium coprobium PF1169

<400>  77

Val Tyr Ser Gly Ser Met Thr Asn Asp Tyr Glu Leu Leu Ser Thr Arg
1               5                   10                  15

Asp Ile Tyr Asp Met Pro His Asn Ser Ala Thr Gly Asn Gly Arg Thr
            20                  25                  30

Met Leu Ala Asn Arg Leu Ser Trp Phe Phe Asp Leu Gln Gly Pro Ser
        35                  40                  45

Ile Met Met Asp Thr Ala Cys Ser Ser Ser Leu Thr Ala Val His Leu
    50                  55                  60

Ala Ala Gln Ser Leu
65

<210>  78
<211>  85
<212>  PRT
<213>  Penicillium coprobium PF1169

<400>  78

Asp Ala Gln Phe Phe Gly Thr Lys Pro Val Glu Ala Asn Ser Ile Asp
1               5                   10                  15

Pro Gln Gln Arg Leu Leu Leu Glu Thr Val Tyr Glu Gly Leu Glu Thr
            20                  25                  30

Ser Gly Ile Pro Met Glu Arg Leu Gln Gly Ser Asn Thr Ala Val Tyr
        35                  40                  45

Val Gly Leu Met Thr Asn Asp Tyr Ala Asp Met Leu Gly Arg Asp Met
    50                  55                  60

Gln Asn Phe Pro Thr Tyr Phe Ala Ser Gly Thr Ala Arg Ser Ile Leu
65                  70                  75                  80

Ser Asn Arg Val Ser
                85

<210>  79
<211>  28
<212>  PRT
<213>  Penicillium coprobium PF1169

<400>  79

Asp Pro Ala Tyr Phe Asp Ser Ser Phe Phe Asn Ile Thr Lys Thr Glu
1               5                   10                  15

Leu Leu Thr Leu Asp Pro Gln Gln Arg Leu Val Leu

20                  25

<210>  80
<211>  51
<212>  PRT
<213>  Penicillium coprobium PF1169

<400>  80

Val Ala Cys Val Asn Ser Pro Ala Ser Thr Thr Leu Ser Gly Asp Val
1               5                   10                  15

Asp Tyr Ile Asn Gln Leu Glu Ala Arg Leu Gln Gln Asp Gly His Phe
            20                  25                  30

Ala Arg Lys Leu Arg Ile Asp Thr Ala Tyr His Ser Pro His Met Glu
        35                  40                  45

Glu Leu Val
    50

<210>  81
<211>  24
<212>  PRT
<213>  Penicillium coprobium PF1169

<400>  81

Leu Lys Ser Ile Ser Pro Val Val Thr Gln Leu Gly Thr Thr Cys Val
1               5                   10                  15

Gln Met Ala Leu Thr Lys Tyr Trp
            20

<210>  82
<211>  59
<212>  PRT
<213>  Penicillium coprobium PF1169

<400>  82

Gly Cys Phe Tyr Gly Met Thr Ser Asp Asp Tyr Arg Glu Val Asn Ser
1               5                   10                  15

Gly Gln Asp Ile Asp Thr Tyr Phe Ile Pro Gly Gly Asn Arg Ala Phe
            20                  25                  30

Thr Pro Gly Arg Ile Asn Tyr Tyr Phe Lys Phe Ser Gly Pro Ser Val
        35                  40                  45

Ser Val Asp Thr Ala Cys Ser Ser Ser Leu Ala
    50                  55

<210>  83

<211>  16
<212>  PRT
<213>  Penicillium coprobium PF1169

<400>  83

Leu Glu Met Ala Gly Phe Ile Pro Asp Ser Ile Pro Leu Arg Arg Arg
1               5                   10                  15


<210>  84
<211>  53
<212>  PRT
<213>  Penicillium coprobium PF1169

<400>  84

Ala Ile Val Gly Gly Val Asn Ala Leu Cys Gly Pro Gly Leu Thr Arg
1               5                   10                  15


Val Leu Asp Lys Ala Gly Ala Ile Ser Ser Asp Gly Ser Cys Lys Ser
            20                  25                  30


Phe Asp Asp Asp Ala His Gly Tyr Ala Arg Gly Glu Gly Ala Gly Ala
        35                  40                  45


Leu Val Thr Lys Lys
    50


<210>  85
<211>  60
<212>  PRT
<213>  Penicillium coprobium PF1169

<400>  85

Ile Ala Ile Val Gly Ile Gly Gly Arg Phe Pro Gly Glu Ala Thr Asn
1               5                   10                  15


Pro Asn Arg Leu Trp Asp Met Val Ser Asn Gly Arg Ser Ala Leu Thr
            20                  25                  30


Glu Val Pro Lys Asp Arg Phe Asn Ile Asp Ala Phe Tyr His Pro His
        35                  40                  45


Ala Glu Arg Gln Gly Thr Met Asn Val Arg Arg Gly
    50                  55                  60


<210>  86
<211>  53
<212>  PRT
<213>  Penicillium coprobium PF1169

<400>  86

Ser Val Pro Ile Glu Glu His Ser Pro Val Val Thr Gln Leu Gly Thr
1               5                   10                  15

Thr Cys Val Gln Met Ala Leu Thr Lys Tyr Trp Thr Ser Leu Gly Val
            20                  25                  30

Thr Pro Ser Phe Val Met Gly His Ser Leu Gly Glu Phe Ala Ala Leu
        35                  40                  45

Asn Ala Ala Gly Val
    50

<210>  87
<211>  18
<212>  PRT
<213>  Penicillium coprobium PF1169

<400>  87

Ser Val Pro Ile Glu Glu His Ser Pro Val Val Thr Gln Leu Gly Thr
1               5                   10                  15

Thr Cys

<210>  88
<211>  62
<212>  PRT
<213>  Penicillium coprobium PF1169

<400>  88

Phe Leu Asp Asp Leu Ala Phe Thr Val Asn Glu Arg Arg Ser Ile Phe
1               5                   10                  15

Pro Trp Lys Ala Ala Val Val Gly Asp Thr Met Glu Gly Leu Ala Ala
            20                  25                  30

Ser Leu Ala Gln Asn Ile Lys Pro Arg Ser Val Leu Arg Met Pro Thr
        35                  40                  45

Leu Gly Phe Val Phe Thr Gly Gln Gly Ala Gln Trp Pro Gly
    50                  55                  60

<210>  89
<211>  51
<212>  PRT
<213>  Penicillium coprobium PF1169

<400>  89

Ser Ser Phe Leu Thr Ser Thr Val Gln Gln Ile Val Glu Glu Thr Ile
1               5                   10                  15

Gln Gly Gly Thr Gly Gln Val Val Met Glu Ser Asp Leu Met Gln Thr
            20                  25                  30

Glu Phe Leu Glu Ala Ala Asn Gly His Arg Met Asn Asp Cys Gly Val
        35                  40                  45

Val Thr Ser
    50

<210>  90
<211>  77
<212>  PRT
<213>  Penicillium coprobium PF1169

<400>  90

Glu Cys Gly Phe Val Glu Met His Gly Thr Gly Thr Lys Ala Gly Asp
1               5                   10                  15

Pro Val Glu Ala Ala Ala Val His Ala Ala Leu Gly Lys Asn Arg Thr
            20                  25                  30

Leu Arg Asn Pro Leu Tyr Ile Gly Ser Val Lys Ser Asn Ile Gly His
        35                  40                  45

Leu Glu Gly Ala Ser Gly Ile Val Ala Val Ile Lys Ala Ala Met Met
    50                  55                  60

Leu Asp Arg Asp Leu Met Leu Pro Asn Ala Glu Phe Lys
65                  70                  75

<210>  91
<211>  78
<212>  PRT
<213>  Penicillium coprobium PF1169

<220>
<221>  misc_feature
<222>  (4)..(4)
<223>  Xaa can be any naturally occurring amino acid

<400>  91

Phe Phe Lys Xaa Ser Gly Pro Ser Phe Ser Ile Asp Thr Ala Cys Ser
1               5                   10                  15

Ser Ser Leu Ala Thr Ile Gln Val Cys Thr His Leu Phe His Val His
            20                  25                  30

Leu Asn Arg Gln Leu Thr Ile Ala Ala Cys Thr Ser Leu Trp Asn Gly
        35                  40                  45

Glu Thr Asp Thr Val Val Ala Gly Gly Met Asn Ile Leu Thr Asn Ser
    50                  55                  60

Asp Ala Phe Ala Gly Leu Ser His Gly His Phe Leu Thr Lys
65                  70                  75

<210>  92
<211>  79
<212>  PRT
<213>  Penicillium coprobium PF1169

<400>  92

Ser Val Pro Ile Glu Glu His Ser Pro Val Val Thr Gln Leu Gly Thr
1               5                   10                  15

Thr Cys Val Gln Met Ala Leu Thr Lys Tyr Trp Thr Ser Leu Gly Val
            20                  25                  30

Thr Pro Ser Phe Val Met Gly His Ser Leu Gly Glu Phe Ala Ala Leu
        35                  40                  45

Asn Ala Ala Gly Val Leu Thr Ile Ser Asp Thr Ile Tyr Leu Ala Gly
    50                  55                  60

Arg Arg Ala Gln Leu Leu Thr Glu Gln Ile Glu Gly Gly Thr His
65                  70                  75

<210>  93
<211>  68
<212>  PRT
<213>  Penicillium coprobium PF1169

<400>  93

Leu Ser Ser Asp Gly Arg Cys His Thr Phe Asp Glu Lys Ala Asn Gly
1               5                   10                  15

Tyr Ala Arg Gly Glu Ala Val Gly Cys Leu Ile Leu Lys Pro Leu Ala
            20                  25                  30

Lys Ala Leu His Asp Gln Asn Lys Ile Arg Ala Val Ile Arg Gly Thr
        35                  40                  45

Gly Ser Asn Gln Asp Gly Arg Thr Ala Gly Ile Thr Val Pro Asn Gly
    50                  55                  60

Ala Ala Gln Glu
65

<210>  94
<211>  80
<212>  PRT
<213>  Penicillium coprobium PF1169

<400>  94

Ser Pro Leu Phe Gly Leu Ala Arg Ile Ile Ala Ser Glu His Pro Asp
1               5                   10                  15

Leu Gly Ser Leu Ile Asp Ile Glu Glu Pro Ile Ile Pro Leu Ser Thr
            20                  25                  30

Met Arg Tyr Ile Gln Gly Ala Asp Ile Val Arg Ile Ser Asp Gly Ile
        35                  40                  45

Ala Arg Thr Ser Arg Phe Arg Ser Leu Pro Arg Thr Lys Leu Arg Pro
    50                  55                  60

Val Ser Asp Gly Pro Arg Leu Leu Pro Arg Pro Glu Gly Thr Tyr Leu
65                  70                  75                  80

<210>  95
<211>  75
<212>  PRT
<213>  Penicillium coprobium PF1169

<400>  95

Asn Arg Ile Ser Tyr Tyr Phe Asp Trp Gln Gly Pro Ser Met Ala Val
1               5                   10                  15

Asp Thr Gly Cys Ser Ser Ser Leu Leu Ala Val His Leu Gly Val Glu
            20                  25                  30

Ala Leu Gln Asn Asp Asp Cys Ser Met Ala Val Ala Val Gly Ser Asn
        35                  40                  45

Leu Ile Leu Ser Pro Asn Ala Tyr Ile Ala Asp Ser Lys Thr Arg Met
    50                  55                  60

Leu Ser Pro Thr Gly Arg Ser Arg Met Trp Asp
65                  70                  75

<210>  96
<211>  81
<212>  PRT
<213>  Penicillium coprobium PF1169

<400>  96

Val Asp Val Asn Pro Ala Val Leu Lys Asp Ala Pro Leu Pro Trp Asp
1               5                   10                  15

Pro Ser Ser Trp Ala Pro Ile Leu Asp Ala Ala Thr Ser Val Gly Ser
            20                  25                  30

Thr Ile Phe Gln Thr Ala Ala Leu Arg Met Pro Ala Gln Ile Glu Arg
        35                  40                  45

Val Glu Ile Phe Thr Ser Glu Asn Pro Pro Lys Thr Ser Trp Leu Tyr
    50                  55                  60

Val Gln Glu Ala Ser Asp Ala Val Pro Thr Ser His Val Ser Val Val
65                  70                  75                  80

Ser

<210>  97
<211>  37
<212>  PRT
<213>  Penicillium coprobium PF1169

<400>  97

Pro Leu Phe Gly Leu Ala Arg Ile Ile Ala Ser Glu His Pro Asp Leu
1               5                   10                  15

Gly Ser Leu Ile Asp Ile Glu Glu Pro Ile Ile Pro Leu Ser Thr Met
            20                  25                  30

Arg Tyr Ile Arg Gly
        35

<210>  98
<211>  84
<212>  PRT
<213>  Penicillium coprobium PF1169

<400>  98

Ala Val Ile Arg Gly Thr Gly Ser Asn Gln Asp Gly Arg Thr Ala Gly
1               5                   10                  15

Ile Thr Val Pro Asn Gly Ala Ala Gln Glu Ser Leu Ile Arg Ser Val
            20                  25                  30

Tyr Ala Gln Ala Asp Leu Asp Pro Ser Glu Thr Asp Phe Val Glu Ala
        35                  40                  45

His Gly Thr Gly Thr Leu Ala Gly Asp Pro Val Glu Thr Gly Ala Ile
    50                  55                  60

Ala Arg Val Phe Gly Thr Asp Arg Pro Pro Gly Asp Pro Val Arg Ile
65                  70                  75                  80

Gly Ser Ile Lys

<210>  99

<211>  69
<212>  PRT
<213>  Penicillium coprobium PF1169

<400>  99

Leu Glu Val Val Trp Glu Cys Leu Glu Asn Ser Gly Glu Thr Gln Trp
1               5                   10                  15

Arg Gly Lys Glu Ile Gly Cys Phe Val Gly Val Phe Gly Glu Asp Trp
            20                  25                  30

Leu Glu Met Ser His Lys Asp Pro Gln His Leu Asn Gln Met Phe Pro
        35                  40                  45

Ile Ala Thr Gly Gly Phe Ala Leu Ala Asn Gln Val Ser Tyr Arg Phe
    50                  55                  60

Asp Leu Thr Gly Pro
65

<210>  100
<211>  79
<212>  PRT
<213>  Penicillium coprobium PF1169

<400>  100

Gly Gly Ala Thr Asp Thr Glu Lys Phe Trp Asp Leu Leu Ala Ser Gly
1               5                   10                  15

Val Asp Val His Arg Lys Ile Pro Ala Asp Arg Phe Asp Val Glu Thr
            20                  25                  30

His Tyr Asp Pro Asn Gly Lys Arg Met Asn Ala Ser His Thr Pro Tyr
        35                  40                  45

Gly Cys Phe Ile Asp Glu Pro Gly Leu Phe Asp Ala Ala Phe Phe Asn
    50                  55                  60

Met Ser Pro Arg Glu Ala Gln Gln Thr Asp Pro Met Gln Arg Leu
65                  70                  75

<210>  101
<211>  52
<212>  PRT
<213>  Penicillium coprobium PF1169

<400>  101

Glu Leu Arg His Gly Lys Asn Ile Asp Lys Pro Glu Tyr Ser Gln Pro
1               5                   10                  15

Leu Cys Thr Ala Ile Gln Ile Ala Leu Val Glu Leu Leu Glu Ser Phe

20                  25                  30

Gly Val Val Pro Lys Ala Val Val Gly His Ser Ser Gly Glu Ile Ala
        35                  40                  45

Ala Ala Tyr Val
    50

<210>  102
<211>  34
<212>  PRT
<213>  Penicillium coprobium PF1169

<400>  102

Val Gly Phe Val Phe Thr Gly Gln Gly Ala Gln Trp His Gly Met Gly
1               5                   10                  15

Lys Glu Leu Leu Ser Thr Tyr Pro Ile Phe Arg Gln Thr Met Gln Asp
            20                  25                  30

Val Asp

<210>  103
<211>  63
<212>  PRT
<213>  Penicillium coprobium PF1169

<400>  103

Phe Asp Ala Ala Phe Phe Asn Met Ser Pro Arg Glu Ala Gln Gln Thr
1               5                   10                  15

Asp Pro Met Gln Arg Leu Ala Ile Val Thr Ala Tyr Glu Ala Leu Glu
            20                  25                  30

Arg Ala Gly Tyr Val Ala Asn Arg Thr Ala Ala Thr Asn Leu His Arg
        35                  40                  45

Ile Gly Thr Phe Tyr Gly Gln Ala Ser Asp Asp Tyr Arg Glu Val
    50                  55                  60

<210>  104
<211>  43
<212>  PRT
<213>  Penicillium coprobium PF1169

<400>  104

Ala Val Val Ser Gly Val Ser Ile Leu Glu Asn Pro Val Glu Thr Ile
1               5                   10                  15

Gly Met Ser His His Gly Leu Leu Gly Pro Gln Gly Arg Ser Phe Ser

20                  25                  30

Phe Asp Ser Arg Ala Glu Gly Tyr Ala Arg Gly
        35                  40

<210>  105
<211>  71
<212>  PRT
<213>  Penicillium coprobium PF1169

<400>  105

Lys Ala Ser Leu Ser Leu Gln His Gly Met Ile Ala Pro Asn Leu Leu
1               5                   10                  15

Met Gln His Leu Asn Pro Lys Ile Lys Pro Phe Ala Ala Lys Leu Ser
            20                  25                  30

Val Pro Thr Glu Cys Ile Pro Trp Pro Ala Val Pro Asp Gly Cys Pro
        35                  40                  45

Arg Arg Ala Ser Val Asn Ser Phe Gly Phe Gly Gly Ala Asn Val His
    50                  55                  60

Val Val Leu Glu Ser Tyr Thr
65                  70

<210>  106
<211>  28
<212>  PRT
<213>  Penicillium coprobium PF1169

<400>  106

Pro Trp Pro Thr Thr Gly Leu Arg Arg Ala Ser Val Asn Ser Phe Gly
1               5                   10                  15

Tyr Gly Gly Thr Asn Ala His Cys Val Leu Asp Asp
            20                  25

<210>  107
<211>  71
<212>  PRT
<213>  Penicillium coprobium PF1169

<400>  107

Lys Ala Ser Leu Ser Leu Gln His Gly Met Ile Ala Pro Asn Leu Leu
1               5                   10                  15

Met Gln His Leu Asn Pro Lys Ile Lys Pro Phe Ala Ala Lys Leu Ser
            20                  25                  30

Val Pro Thr Glu Cys Ile Pro Trp Pro Ala Val Pro Asp Gly Cys Pro

        35                  40                  45

Arg Arg Ala Ser Val Asn Ser Phe Gly Phe Gly Gly Ala Asn Val His
    50                  55                  60

Val Val Leu Glu Ser Tyr Thr
65                  70

<210> 108
<211> 50
<212> PRT
<213> Penicillium coprobium PF1169

<400> 108

Asp Arg Leu Phe Leu Gln Met Ser His Glu Glu Trp Glu Ala Ala Leu
1               5                   10                  15

Ala Pro Lys Val Thr Gly Thr Trp Asn Leu His His Ala Thr Ala Gln
            20                  25                  30

His Ser Leu Asp Phe Phe Val Val Phe Gly Ser Ile Ala Gly Val Cys
        35                  40                  45

Gly Asn
    50

<210> 109
<211> 82
<212> PRT
<213> Penicillium coprobium PF1169

<400> 109

Thr Phe Leu Lys Gly Thr Gly Gly Gln Met Leu Gln Asn Val Val Leu
1               5                   10                  15

Arg Val Pro Val Ala Ile Asn Ala Pro Arg Ser Val Gln Val Val Val
            20                  25                  30

Gln Gln Asp Gln Val Lys Val Val Ser Arg Leu Ile Pro Ser Glu Ala
        35                  40                  45

Ser Val Leu Asp Asp Asp Ala Ser Trp Val Thr His Thr Thr Ala Tyr
    50                  55                  60

Trp Asp Arg Arg Val Leu Gly Ser Glu Asp Arg Ile Asp Leu Ala Ala
65                  70                  75                  80

Val Lys

<210>  110
<211>  30
<212>  PRT
<213>  Penicillium coprobium PF1169

<400>  110

Asp Thr Ala Cys Ser Ser Ser Leu Val Ala Leu His Tyr Ala Val Gln
1               5                   10                  15

Ser Leu Arg Asn Gly Glu Ser Thr Glu Ala Leu Ile Ala Gly
            20                  25                  30

<210>  111
<211>  38
<212>  PRT
<213>  Penicillium coprobium PF1169

<400>  111

Gly Thr Gly Asn Gly Ser Ala Met Ile Ser Asn Arg Ile Ser Trp Phe
1               5                   10                  15

Phe Asp Leu Lys Gly Pro Ser Leu Ser Leu Asp Thr Ala Cys Ser Ser
            20                  25                  30

Ser Leu Val Ala Leu His
        35

<210>  112
<211>  72
<212>  PRT
<213>  Penicillium coprobium PF1169

<400>  112

Thr Ser Thr Gln Leu Asn Asp Leu Asn Glu Thr Asn Ala Ile Lys Lys
1               5                   10                  15

Val Phe Gly Lys Gln Ala Tyr Asn Ile Pro Ile Ser Ser Thr Lys Ser
            20                  25                  30

Tyr Thr Gly His Leu Ile Gly Ala Ala Gly Thr Met Glu Thr Ile Phe
        35                  40                  45

Cys Ile Lys Thr Met Gln Glu Lys Ile Ala Pro Ala Thr Thr Asn Leu
    50                  55                  60

Lys Glu Arg Asp Ser Asn Cys Asp
65                  70

<210>  113
<211>  50
<212>  PRT
<213>  Penicillium coprobium PF1169

<400>  113

Val Ile Val Gly Ser Ala Ala Asn Gln Asn Leu Asn Leu Ser His Ile
1               5                   10                  15

Thr Val Pro His Ser Gly Ser Gln Val Lys Leu Tyr Gln Asn Val Met
            20                  25                  30

Ser Gln Ala Gly Val His Pro His Ser Val Thr Tyr Val Glu Ala His
        35                  40                  45

Gly Thr
    50

<210>  114
<211>  48
<212>  PRT
<213>  Penicillium coprobium PF1169

<400>  114

Leu Pro Thr Ala Ile Gln Pro Leu Phe Arg Ala Asn Val Ser Tyr Leu
1               5                   10                  15

Leu Val Gly Gly Leu Gly Gly Ile Gly Lys Glu Val Ala Leu Trp Met
            20                  25                  30

Val Gln Asn Gly Ala Lys Ser Leu Ile Phe Val Asn Arg Ser Gly Leu
        35                  40                  45

<210>  115
<211>  53
<212>  PRT
<213>  Penicillium coprobium PF1169

<400>  115

Val Ala Ile Val Gly Gly Val Asn Ala Leu Cys Gly Pro Gly Leu Thr
1               5                   10                  15

Arg Val Leu Asp Lys Ala Gly Ala Ile Ser Ser Asp Gly Ser Cys Lys
            20                  25                  30

Ser Phe Asp Asp Asp Ala His Gly Tyr Ala Arg Gly Glu Gly Ala Gly
        35                  40                  45

Ala Leu Val Leu Lys
    50

<210>  116
<211>  28
<212>  PRT
<213>  Penicillium coprobium PF1169

<400>  116

Pro Trp Glu Ser Pro Gly Ala Arg Arg Val Ser Val Asn Ser Phe Gly
1               5                   10                  15

Tyr Gly Gly Ser Asn Ala His Val Ile Ile Glu Asp
            20                  25

<210>  117
<211>  72
<212>  PRT
<213>  Penicillium coprobium PF1169

<400>  117

Lys Thr Leu Arg Glu Trp Met Thr Ala Glu Gly Lys Asp His Asn Leu
1               5                   10                  15

Ser Asp Ile Leu Thr Thr Leu Ala Thr Arg Arg Asp His His Asp Tyr
            20                  25                  30

Arg Ala Ala Leu Val Val Asp Asp Asn Arg Asp Ala Glu Leu Ala Leu
        35                  40                  45

Gln Ala Leu Glu His Gly Val Asp Gln Thr Phe Thr Thr Gln Ser Arg
    50                  55                  60

Val Phe Gly Ala Asp Ile Ser Lys
65                  70

<210>  118
<211>  80
<212>  PRT
<213>  Penicillium coprobium PF1169

<400>  118

Ser Asp Asp Tyr Arg Glu Val Asn Ser Gly Gln Asp Ile Asp Thr Tyr
1               5                   10                  15

Phe Ile Pro Gly Gly Asn Arg Ala Phe Thr Pro Gly Arg Ile Asn Tyr
            20                  25                  30

Tyr Phe Lys Phe Ser Gly Pro Ser Val Ser Val Asp Thr Ala Cys Ser
        35                  40                  45

Ser Ser Leu Ala Ala Ile His Val Ala Cys Asn Ser Leu Trp Arg Asn
    50                  55                  60

Glu Ser Asp Ser Ala Val Ala Gly Gly Val Asn Ile Leu Thr Asn Pro
65                  70                  75                  80

<210>  119
<211>  56
<212>  PRT
<213>  Penicillium coprobium PF1169

<400>  119

Leu Ser Ser Asp Gly Arg Cys His Thr Phe Asp Glu Lys Ala Asn Gly
1               5                   10                  15

Tyr Ala Arg Gly Glu Ala Val Gly Cys Leu Ile Leu Lys Pro Leu Ala
            20                  25                  30

Lys Ala Leu His Asp Gln Asn Lys Ile Arg Ala Val Ile Arg Gly Thr
        35                  40                  45

Gly Ser Asn Gln Gly Arg Ala Asn
    50                  55

<210>  120
<211>  63
<212>  PRT
<213>  Penicillium coprobium PF1169

<400>  120

Asp Thr Ala Cys Ser Ser Ser Leu Tyr Ala Leu His Ser Ala Cys Leu
1               5                   10                  15

Ala Leu Asp Ser Arg Asp Cys Asp Gly Ala Val Val Ala Ala Ala Asn
            20                  25                  30

Leu Ile Gln Ser Pro Glu Gln Gln Met Ile Ala Val Lys Ala Gly Ile
        35                  40                  45

Leu Ser Pro Asp Ser Met Cys His Thr Phe Asp Glu Ser Ala Asn
    50                  55                  60

<210>  121
<211>  28
<212>  PRT
<213>  Penicillium coprobium PF1169

<400>  121

Pro Trp Pro Thr Thr Gly Leu Arg Arg Ala Ser Val Asn Ser Phe Gly
1               5                   10                  15

Tyr Gly Gly Thr Asn Ala His Cys Val Leu Asp Asp
            20                  25

<210>  122
<211>  62
<212>  PRT
<213>  Penicillium coprobium PF1169

<400>  122

Ala Gly Ile Pro Leu Ala Asn Ile Met Gly Thr Lys Thr Ser Cys Phe
1               5                   10                  15

Val Gly Ser Phe Ser Ala Asp Tyr Thr Asp Leu Leu Leu Arg Asp Pro
            20                  25                  30

Glu Cys Val Pro Met Tyr Gln Cys Thr Asn Ala Gly Gln Ser Arg Ala
        35                  40                  45

Met Thr Ala Asn Arg Leu Ser Tyr Phe Leu Ile Lys Gly Pro
    50                  55                  60

<210>  123
<211>  80
<212>  PRT
<213>  Penicillium coprobium PF1169

<400>  123

Arg Trp Glu Pro Tyr Tyr Arg Arg Asp Pro Arg Asn Glu Lys Phe Leu
1               5                   10                  15

Lys Gln Thr Thr Ser Arg Gly Tyr Phe Leu Asp His Leu Glu Asp Phe
            20                  25                  30

Asp Cys Gln Phe Phe Gly Ile Ser Pro Lys Glu Ala Glu Gln Met Asp
        35                  40                  45

Pro Gln Gln Arg Val Ser Leu Glu Val Ala Ser Glu Ala Leu Glu Asp
    50                  55                  60

Ala Gly Ile Pro Ala Lys Ser Leu Ser Gly Ser Asp Thr Ala Val Phe
65                  70                  75                  80

<210>  124
<211>  28
<212>  PRT
<213>  Penicillium coprobium PF1169

<400>  124

Pro Gly Arg Ile Asn Tyr Phe Phe Lys Phe Ser Gly Pro Ser Phe Ser
1               5                   10                  15

Ile Asp Thr Ala Cys Ser Ser Ser Leu Ala Thr Ile
            20                  25

<210>  125
<211>  64
<212>  PRT
<213>  Penicillium coprobium PF1169

<400>  125

Ala Gly Ile Pro Leu Ala Asn Ile Met Gly Thr Lys Thr Ser Cys Phe
1               5                   10                  15

Val Gly Ser Phe Ser Ala Asp Tyr Thr Asp Leu Leu Leu Arg Asp Pro
            20                  25                  30

Glu Cys Val Pro Met Tyr Gln Cys Thr Asn Ala Gly Gln Ser Arg Ala
        35                  40                  45

Met Thr Ala Asn Arg Leu Ser Tyr Phe Phe Asp Leu Lys Gly Pro Ser
    50                  55                  60

<210>  126
<211>  52
<212>  PRT
<213>  Penicillium coprobium PF1169

<400>  126

Glu Leu Arg His Gly Lys Asn Ile Asp Lys Pro Glu Tyr Ser Gln Pro
1               5                   10                  15

Leu Cys Thr Ala Ile Gln Ile Ala Leu Val Glu Leu Leu Glu Ser Phe
            20                  25                  30

Gly Val Val Pro Lys Ala Val Val Gly His Ser Ser Gly Glu Ile Ala
        35                  40                  45

Ala Ala Tyr Val
    50

<210>  127
<211>  38
<212>  PRT
<213>  Penicillium coprobium PF1169

<400>  127

Gln Pro Leu Cys Thr Ala Ile Gln Ile Ala Leu Val Glu Leu Leu Glu
1               5                   10                  15

Ser Phe Gly Val Val Pro Lys Ala Val Val Gly His Ser Ser Gly Glu
            20                  25                  30

Ile Ala Ala Ala Tyr Val
        35

<210>  128
<211>  86
<212>  PRT
<213>  Penicillium coprobium PF1169

<400>  128

Arg Leu Pro Gly Asp Val Ser Thr Pro Glu Glu Phe Trp Asp Leu Cys
1               5                   10                  15

Ser Arg Gly Arg Gly Ala Trp Ser Pro Val Pro Lys Asp Arg Phe Asn
            20                  25                  30

Ala Gly Ser Phe Tyr His Pro Asn Ala Asp Arg Pro Gly Ser Phe Asn
        35                  40                  45

Ala Ala Gly Ala His Phe Leu Thr Glu Asp Ile Gly Leu Phe Asp Ala
    50                  55                  60

Pro Phe Phe Asn Ile Thr Leu Gln Glu Ala Gln Thr Met Asp Pro Gln
65                  70                  75                  80

Gln Arg Ile Phe Leu Glu
                85

<210>  129
<211>  69
<212>  PRT
<213>  Penicillium coprobium PF1169

<400>  129

Gln Phe Phe His Ala His Gly Thr Gly Thr Gln Ala Gly Asp Pro Gln
1               5                   10                  15

Glu Ala Glu Ala Val Ser Thr Ala Leu Phe Pro Asp Gly Ser Asn Ile
            20                  25                  30

Glu Thr Lys Leu Phe Val Gly Ser Ile Lys Thr Val Ile Gly His Thr
        35                  40                  45

Glu Gly Ser Ala Gly Leu Ala Ser Leu Ile Gly Ser Ser Leu Ala Met
    50                  55                  60

Lys His Gly Val Ile
65

<210>  130
<211>  64
<212>  PRT
<213>  Penicillium coprobium PF1169

<400>  130

Ala Gly Ile Pro Leu Ala Asn Ile Met Gly Thr Lys Thr Ser Cys Phe
1               5                   10                  15

Val Gly Ser Phe Ser Ala Asp Tyr Thr Asp Leu Leu Leu Arg Asp Pro
            20                  25                  30

Glu Cys Val Pro Met Tyr Gln Cys Thr Asn Ala Gly Gln Ser Arg Ala
        35                  40                  45

Met Thr Ala Asn Arg Leu Ser Tyr Phe Phe Asp Leu Lys Gly Pro Ser
    50                  55                  60

<210>  131
<211>  67
<212>  PRT
<213>  Penicillium coprobium PF1169

<400>  131

Leu Asp Asp Leu Ala Phe Thr Val Asn Glu Arg Arg Ser Ile Phe Pro
1               5                   10                  15

Trp Lys Ala Ala Val Val Gly Asp Thr Met Glu Gly Leu Ala Ala Ser
            20                  25                  30

Leu Ala Gln Asn Ile Lys Pro Arg Ser Val Leu Arg Met Pro Thr Leu
        35                  40                  45

Gly Phe Val Phe Thr Gly Gln Gly Ala Gln Trp Pro Gly Met Gly Lys
    50                  55                  60

Glu Leu Leu
65

<210>  132
<211>  21
<212>  PRT
<213>  Penicillium coprobium PF1169

<400>  132

Ala His Gly Thr Gly Thr Lys Val Gly Asp Pro Met Glu Val Glu Ala
1               5                   10                  15

Ile Ala Asp Val Phe
            20

<210>  133
<211>  71
<212>  PRT
<213>  Penicillium coprobium PF1169

<400>  133

Lys Gly Gly Met Leu Ala Val Gly Ala Ser Ala Ser Asp Ile Gln Gln
1               5                   10                  15

Ile Leu Asp Ala Met Arg Gly Asn Lys Ala Val Ile Ala Cys Val Asn
            20                  25                  30

Ser Glu Ser Ser Val Thr Leu Ser Gly Asp Leu Asp Val Ile Ala Asn
        35                  40                  45

Leu Gln Thr Ala Leu Asp Lys Glu Gly Ile Phe Thr Arg Lys Leu Lys
    50                  55                  60

Val Asp Val Ala Tyr His Ser
65                  70

<210>  134
<211>  75
<212>  PRT
<213>  Penicillium coprobium PF1169

<400>  134

Leu Glu Asn Leu Glu Thr Ala Leu Ala Arg Asn Ala Pro Ile Tyr Ala
1               5                   10                  15

Glu Val Thr Gly Tyr Ala Asn Tyr Ser Asp Ala Tyr Asp Ile Thr Ala
            20                  25                  30

Pro Ala Asp Asp Leu Met Gly Arg Tyr Met Ser Ile Thr Lys Ala Ile
        35                  40                  45

Glu Gln Ala Gln Leu Asn Ile Asn Glu Ile Asp Tyr Ile Asn Ala His
    50                  55                  60

Gly Thr Ser Thr Gln Leu Asn Asp Leu Asn Glu
65                  70                  75

<210>  135
<211>  53
<212>  PRT
<213>  Penicillium coprobium PF1169

<400>  135

Met Ala Met Lys Lys Ala Leu Lys Gln Ala Gln Leu Arg Pro Ser Ala
1               5                   10                  15

Val Asp Tyr Val Asn Ala His Ala Thr Ser Thr Ile Val Gly Asp Ala
            20                  25                  30

Ala Glu Asn Ala Ala Ile Lys Ala Leu Leu Leu Gly Ala Asp Gly Lys
        35                  40                  45

Asp Lys Ala Ala Asp
    50

<210>  136
<211>  38
<212>  PRT
<213>  Penicillium coprobium PF1169

<400>  136

Gly Thr Gly Asn Gly Ser Ala Met Ile Ser Asn Arg Ile Ser Trp Phe
1               5                   10                  15

Phe Asp Leu Lys Gly Pro Ser Leu Ser Leu Asp Thr Ala Cys Ser Ser
            20                  25                  30

Ser Leu Val Ala Leu His
        35

<210>  137
<211>  76
<212>  PRT
<213>  Penicillium coprobium PF1169

<400>  137

Gly Pro Ser Met Thr Ile Asp Thr Ala Cys Ser Ser Ser Leu Ile Ala
1               5                   10                  15

Leu His Gln Ala Val Gln Ser Leu Arg Ser Gly Glu Thr Asp Val Ala
            20                  25                  30

Val Ala Ala Gly Thr Asn Leu Leu Leu Gly Pro Glu Gln Tyr Ile Ala
        35                  40                  45

Glu Ser Lys Leu Lys Met Leu Ser Pro Asn Gly Arg Ser Arg Met Trp
    50                  55                  60

Asp Lys Asp Ala Asp Gly Tyr Ala Arg Gly Asp Gly
65                  70                  75

<210>  138
<211>  85
<212>  PRT
<213>  Penicillium coprobium PF1169

<400>  138

Ile Gly Ser Ile Lys Pro Asn Ile Gly His Leu Glu Ala Gly Ala Gly
1               5                   10                  15

Val Met Gly Phe Ile Lys Ala Ile Leu Ser Ile Gln Lys Gly Val Leu
            20                  25                  30

Ala Pro Gln Ala Asn Leu Thr Lys Leu Asn Ser Arg Ile Asp Trp Lys
        35                  40                  45

Thr Ala Gly Val Lys Val Val Gln Glu Ala Thr Pro Trp Pro Ser Ser
    50                  55                  60

Asp Ser Ile Arg Arg Ala Gly Val Cys Ser Tyr Gly Tyr Gly Gly Thr
65                  70                  75                  80

Val Ser His Ala Val
                85

<210>  139
<211>  57
<212>  PRT
<213>  Penicillium coprobium PF1169

<400>  139

Asn Ala Ala Gly Ala His Phe Leu Thr Glu Asp Ile Gly Leu Phe Asp
1               5                   10                  15

Ala Pro Phe Phe Asn Ile Thr Leu Gln Glu Ala Gln Thr Met Asp Pro
            20                  25                  30

Gln Gln Arg Ile Phe Leu Glu Cys Val Tyr Glu Ala Leu Glu Asn Gly
        35                  40                  45

Gly Ile Pro Thr His Glu Ile Thr Gly
    50                  55

<210>  140
<211>  68
<212>  PRT
<213>  Penicillium coprobium PF1169

<400>  140

Leu Ser Ser Asp Gly Arg Cys His Thr Phe Asp Glu Lys Ala Asn Gly
1               5                   10                  15

Tyr Ala Arg Gly Glu Ala Val Gly Cys Leu Ile Leu Lys Pro Leu Ala
            20                  25                  30

Lys Ala Leu His Asp Gln Asn Lys Ile Arg Ala Val Ile Arg Gly Thr
        35                  40                  45

Gly Ser Asn Gln Asp Gly Arg Thr Ala Gly Ile Thr Val Pro Asn Gly
    50                  55                  60

Ala Ala Gln Glu
65

<210>  141
<211>  37

<212>  PRT
<213>  Penicillium coprobium PF1169

<400>  141

Ser Phe Asp Ser Arg Ala Glu Gly Tyr Ala Arg Gly Glu Gly Val Gly
1               5                   10                  15

Thr Val Val Val Lys Pro Leu Ser Thr Ala Ile Arg Asp Gly Asp Thr
            20                  25                  30

Ile Arg Ala Val Ile
        35

<210>  142
<211>  72
<212>  PRT
<213>  Penicillium coprobium PF1169

<400>  142

Gly Ile Pro Ile Asp Thr Leu Pro Gly Ser Asn Thr Ala Val Tyr Ser
1               5                   10                  15

Gly Ser Met Thr Asn Asp Tyr Glu Leu Leu Ser Thr Arg Asp Ile Tyr
            20                  25                  30

Asp Met Pro His Asn Ser Ala Thr Gly Asn Gly Arg Thr Met Leu Ala
        35                  40                  45

Asn Arg Leu Ser Trp Phe Phe Asp Leu Gln Gly Pro Ser Ile Met Met
    50                  55                  60

Asp Thr Ala Cys Ser Ser Ser Leu
65                  70

<210>  143
<211>  83
<212>  PRT
<213>  Penicillium coprobium PF1169

<400>  143

Ala Gln Gln Ser Leu Ile Leu Ala Thr Tyr Ala Arg Ala Gly Leu Ser
1               5                   10                  15

Pro Gln Asn Asn Pro Glu Asp Arg Cys Gln Tyr Phe Glu Ala His Gly
            20                  25                  30

Thr Gly Thr Gln Ala Gly Asp Pro Gln Glu Ala Ala Ala Ile Asn Ser
        35                  40                  45

Ser Phe Phe Gly Pro Glu Ser Val Pro Asp Ser Thr Asp Arg Leu Tyr
    50                  55                  60

Val Gly Ser Ile Lys Thr Ile Ile Gly His Thr Glu Ala Thr Ala Gly
65                  70                  75                  80

Leu Ala Gly

<210>  144
<211>  69
<212>  PRT
<213>  Penicillium coprobium PF1169

<400>  144

Pro Leu Trp Arg Lys Ile Glu Thr Ala Pro Leu Asn Thr Gly Leu Thr
1               5                   10                  15

His Asp Val Glu Lys His Thr Leu Leu Gly Gln Arg Ile Pro Val Ala
            20                  25                  30

Gly Thr Asp Thr Phe Val Tyr Thr Thr Arg Leu Asp Asn Glu Thr Lys
        35                  40                  45

Pro Phe Pro Gly Ser His Pro Leu His Gly Thr Glu Ile Val Pro Ala
    50                  55                  60

Ala Gly Leu Ile Asn
65

<210>  145
<211>  64
<212>  PRT
<213>  Penicillium coprobium PF1169

<400>  145

Ala Gly Ile Pro Leu Ala Asn Ile Met Gly Thr Lys Thr Ser Cys Phe
1               5                   10                  15

Val Gly Ser Phe Ser Ala Asp Tyr Thr Asp Leu Leu Leu Arg Asp Pro
            20                  25                  30

Glu Cys Val Pro Met Tyr Gln Cys Thr Asn Ala Gly Gln Ser Arg Ala
        35                  40                  45

Met Thr Ala Asn Arg Leu Ser Tyr Phe Phe Asp Leu Lys Gly Pro Ser
    50                  55                  60

<210>  146
<211>  81
<212>  PRT
<213>  Penicillium coprobium PF1169

<400>  146

Gly Tyr Gly Arg Gly Glu Gly Val Ala Ser Val Val Leu Lys Arg Leu
1               5                   10                  15

Gln Asp Ala Ile Asn Asp Gly Asp Pro Ile Glu Cys Val Ile Arg Ala
            20                  25                  30

Ser Gly Ala Asn Ser Asp Gly Arg Thr Met Gly Ile Thr Met Pro Asn
        35                  40                  45

Pro Lys Ala Gln Gln Ser Leu Ile Leu Ala Thr Tyr Ala Arg Ala Gly
    50                  55                  60

Leu Ser Pro Gln Asn Asn Pro Glu Asp Arg Cys Gln Tyr Phe Glu Ala
65                  70                  75                  80

His

<210>  147
<211>  38
<212>  PRT
<213>  Penicillium coprobium PF1169

<400>  147

Gly Thr Gly Asn Gly Ser Ala Met Ile Ser Asn Arg Ile Ser Trp Phe
1               5                   10                  15

Phe Asp Leu Lys Gly Pro Ser Leu Ser Leu Asp Thr Ala Cys Ser Ser
            20                  25                  30

Ser Leu Val Ala Leu His
        35

<210>  148
<211>  53
<212>  PRT
<213>  Penicillium coprobium PF1169

<400>  148

Glu Ala Thr Ser Met Asp Ala Gln Gln Arg Lys Leu Leu Glu Val Thr
1               5                   10                  15

Tyr Glu Ala Leu Glu Asn Ala Gly Val Pro Leu Glu Thr Ile Gln Gly
            20                  25                  30

Ser Asn Thr Gly Val Tyr Val Gly Asn Phe Thr Asn Asp Phe Leu Asn
        35                  40                  45

Met Gln Tyr Lys Asp

    50

<210>  149
<211>  82
<212>  PRT
<213>  Penicillium coprobium PF1169

<400>  149

Gly Ser Leu Ile Asp Ile Glu Glu Pro Ile Ile Pro Leu Ser Thr Met
1               5                   10                  15

Arg Tyr Ile Gln Gly Ala Asp Ile Val Arg Ile Ser Asp Gly Ile Ala
            20                  25                  30

Arg Thr Ser Arg Phe Arg Ser Leu Pro Arg Thr Lys Leu Arg Pro Val
        35                  40                  45

Ser Asp Gly Pro Arg Leu Leu Pro Arg Pro Glu Gly Thr Tyr Leu Ile
    50                  55                  60

Thr Gly Gly Leu Gly Ile Leu Gly Leu Glu Val Ala Asp Phe Leu Val
65                  70                  75                  80

Glu Lys

<210>  150
<211>  65
<212>  PRT
<213>  Penicillium coprobium PF1169

<400>  150

Gln Leu Gly Thr Thr Cys Val Gln Met Ala Leu Thr Lys Tyr Trp Thr
1               5                   10                  15

Ser Leu Gly Val Thr Pro Ser Phe Val Met Gly His Ser Leu Gly Glu
            20                  25                  30

Phe Ala Ala Leu Asn Ala Ala Gly Val Leu Thr Ile Ser Asp Thr Ile
        35                  40                  45

Tyr Leu Ala Gly Arg Arg Ala Gln Leu Leu Thr Glu Gln Ile Lys Val
    50                  55                  60

Gly
65

<210>  151
<211>  78
<212>  PRT
<213>  Penicillium coprobium PF1169

<220>
<221>  misc_feature
<222>  (45)..(45)
<223>  Xaa can be any naturally occurring amino acid

<400>  151

Gly Pro Arg Leu Leu Pro Arg Pro Glu Gly Thr Tyr Leu Ile Thr Gly
1               5                   10                  15

Gly Leu Gly Ile Leu Gly Leu Glu Val Ala Asp Phe Leu Val Glu Lys
            20                  25                  30

Gly Ala Arg Arg Val Leu Leu Ile Ser Arg Arg Ala Xaa Pro Pro Arg
        35                  40                  45

Arg Thr Trp Asp Gln Val Ala Thr Glu Phe Gln Pro Ala Ile Thr Lys
    50                  55                  60

Ile Arg Leu Leu Glu Ser Arg Gly Ala Ser Val Tyr Val Leu
65                  70                  75

<210>  152
<211>  76
<212>  PRT
<213>  Penicillium coprobium PF1169

<400>  152

Gly Pro Ser Met Thr Ile Asp Thr Ala Cys Ser Ser Ser Leu Ile Ala
1               5                   10                  15

Leu His Gln Ala Val Gln Ser Leu Arg Ser Gly Glu Thr Asp Val Ala
            20                  25                  30

Val Ala Ala Gly Thr Asn Leu Leu Leu Gly Pro Glu Gln Tyr Ile Ala
        35                  40                  45

Glu Ser Lys Leu Lys Met Leu Ser Pro Asn Gly Arg Ser Arg Met Trp
    50                  55                  60

Asp Lys Asp Ala Asp Gly Tyr Ala Arg Gly Asp Gly
65                  70                  75

<210>  153
<211>  38
<212>  PRT
<213>  Penicillium coprobium PF1169

<400>  153

Asn Arg Ile Ser Tyr Phe Phe Asp Leu Arg Gly Pro Ser Ile Thr Ile
1               5                   10                  15

Asp Thr Ala Cys Ser Ser Ser Leu Val Ala Leu His Tyr Ala Val Gln
            20                  25                  30

Ser Leu Arg Asn Gly Glu
        35

<210>  154
<211>  74
<212>  PRT
<213>  Penicillium coprobium PF1169

<400>  154

Gly Ser Gly Leu Thr Val Leu Ala Asn Arg Ile Thr His Cys Phe Asp
1               5                   10                  15

Leu Arg Gly Pro Ser His Val Val Asp Thr Ala Cys Ser Ser Ser Leu
            20                  25                  30

Tyr Ala Leu His Ser Ala Cys Leu Ala Leu Asp Ser Arg Asp Cys Asp
        35                  40                  45

Gly Ala Val Val Ala Ala Ala Asn Leu Ile Gln Ser Pro Glu Gln Gln
    50                  55                  60

Met Ile Ala Val Lys Ala Gly Ile Leu Ser
65                  70

<210>  155
<211>  57
<212>  PRT
<213>  Penicillium coprobium PF1169

<400>  155

Gln Leu Gly Thr Thr Cys Val Gln Met Ala Leu Thr Lys Tyr Trp Thr
1               5                   10                  15

Ser Leu Gly Val Thr Pro Ser Phe Val Met Gly His Ser Leu Gly Glu
            20                  25                  30

Phe Ala Ala Leu Asn Ala Ala Gly Val Leu Thr Ile Ser Asp Thr Ile
        35                  40                  45

Tyr Leu Ala Gly Arg Arg Ala Gln Leu
    50                  55

<210>  156
<211>  72
<212>  PRT
<213>  Penicillium coprobium PF1169

<400>  156

His Leu Asn Leu Met Gly Pro Ser Thr Ala Val Asp Ala Ala Cys Ala
1               5                   10                  15

Ser Ser Leu Val Ala Ile His His Gly Val Gln Ala Ile Lys Leu Gly
            20                  25                  30

Glu Ser Arg Val Ala Ile Val Gly Gly Val Asn Ala Leu Cys Gly Pro
        35                  40                  45

Gly Leu Thr Arg Val Leu Asp Lys Ala Gly Ser Ile Ser Ser Asp Gly
    50                  55                  60

Ser Cys Lys Ser Phe Asp Asp Asp
65                  70

<210>  157
<211>  81
<212>  PRT
<213>  Penicillium coprobium PF1169

<400>  157

Leu Lys Gly Thr Gly Gly Gln Met Leu Gln Asn Val Val Leu Arg Val
1               5                   10                  15

Pro Val Ala Ile Asn Ala Pro Arg Ser Val Gln Val Val Val Gln Gln
            20                  25                  30

Asp Gln Val Lys Val Val Ser Arg Leu Ile Pro Ser Glu Ala Ser Val
        35                  40                  45

Leu Asp Asp Asp Ala Ser Trp Val Thr His Thr Thr Ala Tyr Trp Asp
    50                  55                  60

Arg Arg Val Leu Gly Ser Glu Asp Arg Ile Asp Leu Ala Ala Val Lys
65                  70                  75                  80

Ser

<210>  158
<211>  82
<212>  PRT
<213>  Penicillium coprobium PF1169

<400>  158

Ile Met Gly Thr Lys Thr Ser Cys Phe Val Gly Ser Phe Ser Ala Asp
1               5                   10                  15

Tyr Thr Asp Leu Leu Leu Arg Asp Pro Glu Cys Val Pro Met Tyr Gln

                    20                  25                  30

Cys Thr Asn Ala Gly Gln Ser Arg Ala Met Thr Ala Asn Arg Leu Ser
        35                  40                  45

Tyr Phe Phe Asp Leu Lys Gly Pro Ser Val Thr Val Asp Thr Ala Cys
    50                  55                  60

Ser Gly Ser Leu Val Ala Leu His Leu Ala Cys Gln Ser Leu Arg Thr
65                  70                  75                  80

Gly Asp

<210>  159
<211>  75
<212>  PRT
<213>  Penicillium coprobium PF1169

<400>  159

Gly Ser Gly Leu Thr Val Leu Ala Asn Arg Ile Thr His Cys Phe Asp
1               5                   10                  15

Leu Arg Gly Pro Ser His Val Val Asp Thr Ala Cys Ser Ser Ser Leu
            20                  25                  30

Tyr Ala Leu His Ser Ala Cys Phe Gly Pro Leu Asn Ser Arg Asp Cys
        35                  40                  45

Asp Gly Ala Val Val Ala Ala Ala Asn Leu Ile Gln Ser Pro Glu Gln
    50                  55                  60

Gln Met Ile Ala Val Lys Arg Asp Ser Ile Ala
65                  70                  75

<210>  160
<211>  28
<212>  PRT
<213>  Penicillium coprobium PF1169

<400>  160

Pro Trp Pro Thr Thr Gly Leu Arg Arg Ala Ser Val Asn Ser Phe Gly
1               5                   10                  15

Tyr Gly Gly Thr Asn Ala His Cys Val Leu Asp Asp
            20                  25

<210>  161
<211>  64
<212>  PRT
<213>  Penicillium coprobium PF1169

<400>  161

Gln Leu Gly Thr Thr Cys Val Gln Met Ala Leu Thr Lys Tyr Trp Thr
1               5                   10                  15

Ser Leu Gly Val Thr Pro Ser Phe Val Met Gly His Ser Leu Gly Glu
            20                  25                  30

Phe Ala Ala Leu Asn Ala Ala Gly Val Leu Thr Ile Ser Asp Thr Ile
        35                  40                  45

Tyr Leu Ala Gly Arg Arg Ala Gln Leu Leu Thr Glu Gln Ile Glu Gly
    50                  55                  60

<210>  162
<211>  60
<212>  PRT
<213>  Penicillium coprobium PF1169

<400>  162

Ile Ala Pro Asn Ile His Phe Lys Met Pro Asn Pro Gln Ile Pro Phe
1               5                   10                  15

Asn Glu Ala Asn Leu His Val Pro Leu Glu Pro Thr Pro Trp Pro Ala
            20                  25                  30

Gly Arg Pro Glu Arg Ile Ser Val Asn Ser Phe Gly Ile Gly Gly Ser
        35                  40                  45

Asn Ala His Ala Ile Leu Glu Ser Ala Ser Thr Val
    50                  55                  60

<210>  163
<211>  34
<212>  PRT
<213>  Penicillium coprobium PF1169

<400>  163

Gly Leu Val Asn Ile Leu Arg Ser Trp Gly Ile Glu Pro Ser Thr Val
1               5                   10                  15

Val Gly His Ser Ser Gly Glu Ile Val Ala Ala Tyr Thr Ala Arg Ala
            20                  25                  30

Ile Ser

<210>  164
<211>  51
<212>  PRT
<213>  Penicillium coprobium PF1169

<400>  164

Pro Trp Pro Ser Glu Gly Leu Arg Arg Ile Ser Val Asn Ser Phe Gly
1               5                   10                  15

Phe Gly Gly Ser Asn Thr His Val Ile Leu Asp Asp Ala Leu His Tyr
            20                  25                  30

Met Gln Gln Arg Gly Leu Thr Gly Asn His Cys Thr Ala Arg Leu Pro
        35                  40                  45

Gly Ile Leu
    50

<210>  165
<211>  71
<212>  PRT
<213>  Penicillium coprobium PF1169

<220>
<221>  misc_feature
<222>  (5)..(5)
<223>  Xaa can be any naturally occurring amino acid

<400>  165

Ile Gly His Thr Xaa Gly Ser Ala Gly Leu Ala Ser Leu Ile Gly Ser
1               5                   10                  15

Ser Leu Ala Met Lys His Gly Val Ile Pro Pro Asn Leu His Phe Gly
            20                  25                  30

Gln Leu Ser Glu Lys Val Ala Pro Phe Tyr Thr His Leu Asn Ile Pro
        35                  40                  45

Thr Glu Pro Val Pro Trp Pro Asn Ser Thr Ser Ser Gln Val Lys Arg
    50                  55                  60

Ala Ser Ile Asn Ser Phe Gly
65                  70

<210>  166
<211>  74
<212>  PRT
<213>  Penicillium coprobium PF1169

<400>  166

Gly Ser Asn Thr Ala Val Tyr Ser Gly Ser Met Thr Asn Asp Tyr Glu
1               5                   10                  15

Leu Leu Ser Thr Arg Asp Ile Tyr Asp Met Pro His Asn Ser Ala Thr
            20                  25                  30

Gly Asn Gly Arg Thr Met Leu Ala Asn Arg Leu Ser Trp Phe Phe Asp
        35                  40                  45

Leu Gln Gly Pro Ser Ile Met Met Asp Thr Ala Cys Ser Ser Ser Leu
    50                  55                  60

Thr Ala Val His Leu Ala Ala Gln Ser Leu
65                  70

<210>  167
<211>  85
<212>  PRT
<213>  Penicillium coprobium PF1169

<400>  167

Asp Ala Gln Phe Phe Gly Thr Lys Pro Val Glu Ala Asn Ser Ile Asp
1               5                   10                  15

Pro Gln Gln Arg Leu Leu Leu Glu Thr Val Tyr Glu Gly Leu Glu Thr
            20                  25                  30

Ser Gly Ile Pro Met Glu Arg Leu Gln Gly Ser Asn Thr Ala Val Tyr
        35                  40                  45

Val Gly Leu Met Thr Asn Asp Tyr Ala Asp Met Leu Gly Arg Asp Met
    50                  55                  60

Gln Asn Phe Pro Thr Tyr Phe Ala Ser Gly Thr Ala Arg Ser Ile Leu
65                  70                  75                  80

Ser Asn Arg Val Ser
                85

<210>  168
<211>  60
<212>  PRT
<213>  Penicillium coprobium PF1169

<400>  168

Val Val Ala Cys Val Asn Ser Pro Ala Ser Thr Thr Leu Ser Gly Asp
1               5                   10                  15

Val Asp Tyr Ile Asn Gln Leu Glu Ala Arg Leu Gln Gln Asp Gly His
            20                  25                  30

Phe Ala Arg Lys Leu Arg Ile Asp Thr Ala Tyr His Ser Pro His Met
        35                  40                  45

Glu Glu Leu Val Gly Val Val Gly Asp Ala Ile Ser

50                  55                  60

<210>  169
<211>  56
<212>  PRT
<213>  Penicillium coprobium PF1169

<400>  169

Phe Tyr Gly Met Thr Ser Asp Asp Tyr Arg Glu Val Asn Ser Gly Gln
1               5                   10                  15

Asp Ile Asp Thr Tyr Phe Ile Pro Gly Gly Asn Arg Ala Phe Thr Pro
            20                  25                  30

Gly Arg Ile Asn Tyr Tyr Phe Lys Phe Ser Gly Pro Ser Val Ser Val
        35                  40                  45

Asp Thr Ala Cys Ser Ser Ser Leu
    50                  55

<210>  170
<211>  53
<212>  PRT
<213>  Penicillium coprobium PF1169

<400>  170

Val Ala Ile Val Gly Gly Val Asn Ala Leu Cys Gly Pro Gly Leu Thr
1               5                   10                  15

Arg Val Leu Asp Lys Ala Gly Ala Ile Ser Ser Asp Gly Ser Cys Lys
            20                  25                  30

Ser Phe Asp Asp Asp Ala His Gly Tyr Ala Arg Gly Glu Gly Ala Gly
        35                  40                  45

Ala Leu Val Thr Lys
    50

<210>  171
<211>  40
<212>  PRT
<213>  Penicillium coprobium PF1169

<400>  171

Gln Leu Gly Thr Thr Cys Val Gln Met Ala Leu Thr Lys Tyr Trp Thr
1               5                   10                  15

Ser Leu Gly Val Thr Pro Ser Phe Val Met Gly His Ser Leu Gly Glu
            20                  25                  30

Phe Ala Ala Leu Asn Ala Ala Gly

35                  40

<210>  172
<211>  69
<212>  PRT
<213>  Penicillium coprobium PF1169

<400>  172

Arg Glu Trp Met Thr Ala Glu Gly Lys Asp His Asn Leu Ser Asp Ile
1               5                   10                  15

Leu Thr Thr Leu Ala Thr Arg Arg Asp His His Asp Tyr Arg Ala Ala
            20                  25                  30

Leu Val Val Asp Asp Asn Arg Asp Ala Glu Leu Ala Leu Gln Ala Leu
        35                  40                  45

Glu His Gly Val Asp Gln Thr Phe Thr Thr Gln Ser Arg Val Phe Gly
    50                  55                  60

Ala Asp Ile Ser Lys
65

<210>  173
<211>  51
<212>  PRT
<213>  Penicillium coprobium PF1169

<400>  173

Pro Trp Pro Ser Glu Gly Leu Arg Arg Ile Ser Val Asn Ser Phe Gly
1               5                   10                  15

Phe Gly Gly Ser Asn Thr His Val Ile Leu Asp Asp Ala Leu His Tyr
            20                  25                  30

Met Gln Gln Arg Gly Leu Thr Gly Asn His Cys Thr Ala Arg Leu Pro
        35                  40                  45

Gly Ile Leu
    50

<210>  174
<211>  71
<212>  PRT
<213>  Penicillium coprobium PF1169

<400>  174

Phe Val Glu Met His Gly Thr Gly Thr Lys Ala Gly Asp Pro Val Glu
1               5                   10                  15

Ala Ala Ala Val His Ala Ala Leu Gly Lys Asn Arg Thr Leu Arg Asn

20                  25                  30

Pro Leu Tyr Ile Gly Ser Val Lys Ser Asn Ile Gly His Leu Glu Gly
        35                  40                  45

Ala Ser Gly Ile Val Ala Val Ile Lys Ala Ala Met Met Leu Asp Arg
    50                  55                  60

Asp Leu Met Leu Pro Asn Ala
65                  70

<210>  175
<211>  41
<212>  PRT
<213>  Penicillium coprobium PF1169

<400>  175

Leu Ala Ile Val Gly Met Ala Cys Arg Leu Pro Gly Gln Ile Thr Thr
1               5                   10                  15

Pro Gln Glu Leu Trp Glu Leu Cys Ser Arg Gly Arg Ser Ala Trp Ser
            20                  25                  30

Glu Ile Pro Pro Glu Arg Phe Asn Pro
        35                  40

<210>  176
<211>  64
<212>  PRT
<213>  Penicillium coprobium PF1169

<400>  176

Gln Leu Gly Thr Thr Cys Val Gln Met Ala Leu Thr Lys Tyr Trp Thr
1               5                   10                  15

Ser Leu Gly Val Thr Pro Ser Phe Val Met Gly His Ser Leu Gly Glu
            20                  25                  30

Phe Ala Ala Leu Asn Ala Ala Gly Val Leu Thr Ile Ser Asp Thr Ile
        35                  40                  45

Tyr Leu Ala Gly Arg Arg Ala Gln Leu Leu Thr Glu Gln Ile Glu Gly
    50                  55                  60

<210>  177
<211>  74
<212>  PRT
<213>  Penicillium coprobium PF1169

<400>  177

Gly Ala Ser Val Tyr Val Leu Ala Leu Asp Ile Thr Lys Pro Asp Ala

1               5                   10                  15

Val Glu Gln Leu Ser Thr Ala Leu Asp Arg Leu Ala Leu Pro Ser Val
            20                  25                  30

Gln Gly Val Val His Ala Ala Gly Val Leu Asp Asn Glu Leu Val Met
        35                  40                  45

Gln Thr Thr Gln Glu Ala Phe Asn Arg Val Leu Ala Pro Lys Ile Ala
    50                  55                  60

Gly Ala Leu Ala Leu His Glu Pro Phe Pro
65                  70

<210>  178
<211>  72
<212>  PRT
<213>  Penicillium coprobium PF1169

<400>  178

Gly Leu Val Asn Ile Leu Arg Ser Trp Gly Ile Glu Pro Ser Thr Val
1               5                   10                  15

Val Gly His Ser Ser Gly Glu Ile Val Ala Ala Tyr Thr Ala Arg Ala
            20                  25                  30

Ile Ser Met Arg Thr Ala Ile Ile Leu Ala Tyr Tyr Arg Gly Lys Val
        35                  40                  45

Ala Gln Pro Leu Glu Gly Leu Gly Ala Met Val Ala Val Gly Leu Ser
    50                  55                  60

Pro Asp Glu Val Ala Gln Tyr Met
65                  70

<210>  179
<211>  70
<212>  PRT
<213>  Penicillium coprobium PF1169

<400>  179

Gly Arg Phe Leu Ser Ser Asp Gly Arg Cys His Thr Phe Asp Glu Lys
1               5                   10                  15

Ala Asn Gly Tyr Ala Arg Gly Glu Ala Val Gly Cys Leu Ile Leu Lys
            20                  25                  30

Pro Leu Ala Lys Ala Leu His Asp Gln Asn Lys Ile Arg Ala Val Ile
        35                  40                  45

Arg Gly Thr Gly Ser Asn Gln Asp Gly Arg Thr Ala Gly Ile Thr Val
    50                  55                  60

Pro Asn Gly Ala Ala Gln
65                  70

<210>  180
<211>  51
<212>  PRT
<213>  Penicillium coprobium PF1169

<400>  180

Ser Ser Phe Leu Thr Ser Thr Val Gln Gln Ile Val Glu Glu Thr Ile
1               5                   10                  15

Gln Gly Gly Thr Gly Gln Val Val Met Glu Ser Asp Leu Met Gln Thr
            20                  25                  30

Glu Phe Leu Glu Ala Ala Asn Gly His Arg Met Asn Asp Cys Gly Val
        35                  40                  45

Val Thr Ser
    50

<210>  181
<211>  64
<212>  PRT
<213>  Penicillium coprobium PF1169

<400>  181

Leu Leu Gly Leu Arg Leu Lys Trp Lys Glu Tyr His Gln Asp Phe Asn
1               5                   10                  15

Ala Ala His Arg Val Leu Pro Leu Pro Ser Tyr Lys Trp Asp Leu Lys
            20                  25                  30

Asn Tyr Trp Ile Pro Tyr Thr Asn Asn Phe Cys Leu Leu Lys Gly Ala
        35                  40                  45

Pro Ala Ala Pro Val Ala Glu Ala Thr Pro Ile Ser Val Phe Leu Ser
    50                  55                  60

<210>  182
<211>  26
<212>  PRT
<213>  Penicillium coprobium PF1169

<400>  182

Ser Phe Arg Arg Gln Glu Asp Thr Trp Lys Val Leu Ser Asn Ala Thr
1               5                   10                  15

Ser Thr Leu Tyr Leu Ala Gly Ile Glu Ile
            20                  25

<210>  183
<211>  65
<212>  PRT
<213>  Penicillium coprobium PF1169

<400>  183

Ala Gly Gly Asn Thr Thr Val Ala Leu Glu Asp Ala Pro Ile Arg Thr
1               5                   10                  15

Arg Ser Gly Ser Asp Pro Arg Ser Leu His Pro Ile Ala Ile Ser Ala
            20                  25                  30

Lys Ser Lys Val Ser Leu Arg Gly Asn Leu Glu Asn Leu Leu Ala Tyr
        35                  40                  45

Leu Asp Thr His Pro Asp Val Ser Leu Ser Asp Leu Ser Tyr Thr Thr
    50                  55                  60

Thr
65

<210>  184
<211>  96
<212>  PRT
<213>  Penicillium coprobium PF1169

<400>  184

Phe Asp Ala Ala Phe Phe Asn Met Ser Pro Arg Glu Ala Gln Gln Thr
1               5                   10                  15

Asp Pro Met Gln Arg Leu Ala Ile Val Thr Ala Tyr Glu Ala Leu Glu
            20                  25                  30

Arg Ala Gly Tyr Val Ala Asn Arg Thr Ala Ala Thr Asn Leu His Arg
        35                  40                  45

Ile Gly Thr Phe Tyr Gly Gln Ala Ser Asp Asp Tyr Arg Glu Val Asn
    50                  55                  60

Thr Ala Gln Glu Ile Ser Thr Tyr Phe Ile Pro Gly Gly Cys Arg Ala
65                  70                  75                  80

Phe Gly Pro Gly Arg Ile Asn Tyr Phe Phe Lys Phe Leu Gly Pro Ala
                85                  90                  95

<210>  185
<211>  58
<212>  PRT

<213>  Penicillium coprobium PF1169

<400>  185

Phe Leu Gln Ile Ser Gly Pro Ser Phe Ser Ile Asp Thr Ala Cys Ser
1               5                   10                  15

Ser Ser Leu Ala Thr Ile Gln Val Cys Thr His Leu Phe His Val His
            20                  25                  30

Leu Asn Arg Gln Leu Thr Ile Ala Ala Cys Thr Ser Leu Trp Asn Gly
        35                  40                  45

Glu Thr Asp Thr Val Val Ala Gly Gly Met
    50                  55

<210>  186
<211>  59
<212>  PRT
<213>  Penicillium coprobium PF1169

<400>  186

Val Tyr Ser Gly Ser Met Thr Asn Asp Tyr Glu Leu Leu Ser Thr Arg
1               5                   10                  15

Asp Ile Tyr Asp Met Pro His Asn Ser Ala Thr Gly Asn Gly Arg Thr
            20                  25                  30

Met Leu Ala Asn Arg Leu Ser Trp Phe Phe Asp Leu Gln Gly Pro Ser
        35                  40                  45

Ile Met Met Asp Thr Ala Cys Ser Ser Ser Leu
    50                  55

<210>  187
<211>  31
<212>  PRT
<213>  Penicillium coprobium PF1169

<400>  187

Leu Phe Leu Phe Pro Asp Gly Ser Gly Ser Ala Thr Ser Tyr Ala Thr
1               5                   10                  15

Ile Pro Gly Ile Ser Pro Asp Val Cys Val Tyr Gly Leu Asn Cys
            20                  25                  30

<210>  188
<211>  26
<212>  PRT
<213>  Penicillium coprobium PF1169

<400>  188

Ala Lys His Pro Pro Ala Thr Ser Ile Leu Leu Gln Gly Asn Pro Lys
1               5                   10                  15

Thr Ala Thr Gln Ser Phe Ile Phe Val Pro
            20                  25

<210>  189
<211>  38
<212>  PRT
<213>  Penicillium coprobium PF1169

<400>  189

Gly Asn Gly Ser Ala Met Ile Ser Asn Arg Ile Ser Trp Phe Phe Asp
1               5                   10                  15

Leu Lys Gly Pro Ser Leu Ser Leu Asp Thr Ala Cys Ser Ser Ser Leu
            20                  25                  30

Val Ala Leu His Leu Ala
        35

<210>  190
<211>  76
<212>  PRT
<213>  Penicillium coprobium PF1169

<400>  190

Ala Ile His His Gly Val Gln Ala Ile Lys Leu Gly Glu Ser Arg Val
1               5                   10                  15

Ala Ile Val Gly Gly Val Asn Ala Leu Cys Gly Pro Gly Leu Thr Arg
            20                  25                  30

Val Leu Asp Lys Ala Gly Ala Ile Ser Ser Asp Gly Ser Cys Lys Ser
        35                  40                  45

Phe Asp Asp Asp Ala His Gly Tyr Ala Arg Gly Glu Gly Ala Gly Ala
    50                  55                  60

Leu Val Leu Lys Ser Leu His Gln Ala Leu Leu Asp
65                  70                  75

<210>  191
<211>  65
<212>  PRT
<213>  Penicillium coprobium PF1169

<400>  191

Val Trp Ile Glu Ile Gly Pro His Pro Val Cys Leu Gly Phe Val Lys
1               5                   10                  15

Ala Thr Leu Glu Ser Val Ala Val Ala Val Pro Ser Leu Arg Arg Gly
            20                  25                  30

Glu Asn Ala Trp Cys Thr Leu Ala Gln Ser Leu Thr Thr Leu His Asn
        35                  40                  45

Ala Gly Val Pro Val Gly Trp Ser Glu Phe His Arg Pro Phe Glu Arg
    50                  55                  60

Ala
65

<210>  192
<211>  53
<212>  PRT
<213>  Penicillium coprobium PF1169

<400>  192

Thr Ser Asp Asp Tyr Arg Glu Val Asn Ser Gly Gln Asp Ile Asp Thr
1               5                   10                  15

Tyr Phe Ile Pro Gly Gly Asn Arg Ala Phe Thr Pro Gly Arg Ile Asn
            20                  25                  30

Tyr Tyr Phe Lys Phe Ser Gly Pro Ser Val Ser Val Asp Thr Ala Cys
        35                  40                  45

Ser Ser Ser Leu Ala
    50

<210>  193
<211>  40
<212>  PRT
<213>  Penicillium coprobium PF1169

<400>  193

Val Asp Thr Ala Cys Ser Ser Ser Leu Tyr Ala Leu His Ser Ala Cys
1               5                   10                  15

Phe Gly Pro Leu Asn Ser Arg Asp Cys Asp Gly Ala Val Val Ala Ala
            20                  25                  30

Ala Asn Leu Ile Gln Ser Pro Glu
        35                  40

<210>  194
<211>  68
<212>  PRT
<213>  Penicillium coprobium PF1169

<400>  194

Met Leu Ala Val Gly Ala Ser Ala Ser Asp Ile Gln Gln Ile Leu Asp
1               5                   10                  15

Ala Met Arg Gly Asn Lys Ala Val Ile Ala Cys Val Asn Ser Glu Ser
            20                  25                  30

Ser Val Thr Leu Ser Gly Asp Leu Asp Val Ile Ala Asn Leu Gln Thr
        35                  40                  45

Ala Leu Asp Lys Glu Gly Ile Phe Thr Arg Lys Leu Lys Val Asp Val
    50                  55                  60

Ala Tyr His Ser
65

<210>  195
<211>  62
<212>  PRT
<213>  Penicillium coprobium PF1169

<400>  195

Phe Leu Asp Asp Leu Ala Phe Thr Val Asn Glu Arg Arg Ser Ile Phe
1               5                   10                  15

Pro Trp Lys Ala Ala Val Val Gly Asp Thr Met Glu Gly Leu Ala Ala
            20                  25                  30

Ser Leu Ala Gln Asn Ile Lys Pro Arg Ser Val Leu Arg Met Pro Thr
        35                  40                  45

Leu Gly Phe Val Phe Thr Gly Gln Gly Ala Gln Trp Pro Gly
    50                  55                  60

<210>  196
<211>  76
<212>  PRT
<213>  Penicillium coprobium PF1169

<400>  196

Gly Pro Ser Met Thr Ile Asp Thr Ala Cys Ser Ser Ser Leu Ile Ala
1               5                   10                  15

Leu His Gln Ala Val Gln Ser Leu Arg Ser Gly Glu Thr Asp Val Ala
            20                  25                  30

Val Ala Ala Gly Thr Asn Leu Leu Leu Gly Pro Glu Gln Tyr Ile Ala
        35                  40                  45

Glu Ser Lys Leu Lys Met Leu Ser Pro Asn Gly Arg Ser Arg Met Trp
    50                  55                  60

Asp Lys Asp Ala Asp Gly Tyr Ala Arg Gly Asp Gly
65                  70                  75

<210>  197
<211>  79
<212>  PRT
<213>  Penicillium coprobium PF1169

<400>  197

Ser Val Pro Ile Glu Glu His Ser Pro Val Val Thr Gln Leu Gly Thr
1               5                   10                  15

Thr Cys Val Gln Met Ala Leu Thr Lys Tyr Trp Thr Ser Leu Gly Val
            20                  25                  30

Thr Pro Ser Phe Val Met Gly His Ser Leu Gly Glu Phe Ala Ala Leu
        35                  40                  45

Asn Ala Ala Gly Val Leu Thr Ile Ser Asp Thr Ile Tyr Leu Ala Gly
    50                  55                  60

Arg Arg Ala Gln Leu Leu Thr Glu Gln Ile Glu Gly Gly Thr His
65                  70                  75

<210>  198
<211>  40
<212>  PRT
<213>  Penicillium coprobium PF1169

<400>  198

Phe Asn Leu Lys Gly Ile Ser Gln Ser Ile Ala Ser Ala Cys Ala Thr
1               5                   10                  15

Ser Ala Asp Ala Ile Gly Tyr Ala Phe His Leu Ile Ala Ala Gly Lys
            20                  25                  30

Gln Asp Leu Met Leu Ala Gly Gly
        35                  40

<210>  199
<211>  70
<212>  PRT
<213>  Penicillium coprobium PF1169

<400>  199

Gly Arg Phe Leu Ser Ser Asp Gly Arg Cys His Thr Phe Asp Glu Lys
1               5                   10                  15

Ala Asn Gly Tyr Ala Arg Gly Glu Ala Val Gly Cys Leu Ile Leu Lys
            20                  25                  30

Pro Leu Ala Lys Ala Leu His Asp Gln Asn Lys Ile Arg Ala Val Ile
        35                  40                  45

Arg Gly Thr Gly Ser Asn Gln Asp Gly Arg Thr Ala Gly Ile Thr Val
    50                  55                  60

Pro Asn Gly Ala Ala Gln
65                  70

<210>  200
<211>  284
<212>  PRT
<213>  Penicillium coprobium PF1169

<400>  200

Leu Ser Val Lys Arg Val Gly Ile His Asp Asp Phe Phe Glu Leu Gly
1               5                   10                  15

Gly His Ser Leu Leu Ala Val Lys Leu Val Asn His Leu Lys Lys Val
            20                  25                  30

Phe Gly Thr Glu Leu Ser Val Ala Leu Leu Ala Gln Tyr Ser Thr Val
        35                  40                  45

Glu Ser Leu Gly Glu Ile Ile Arg Glu Asn Lys Glu Ile Lys Pro Ser
    50                  55                  60

Ile Val Ile Glu Leu Arg Ser Gly Thr Tyr Glu Gln Pro Leu Trp Leu
65                  70                  75                  80

Phe His Pro Ile Gly Gly Ser Thr Phe Cys Tyr Met Glu Leu Ser Arg
                85                  90                  95

His Leu Asn Pro Asn Arg Thr Leu Arg Ala Ile Gln Ser Pro Gly Leu
            100                 105                 110

Ile Glu Ala Asp Ala Ala Glu Val Ala Ile Glu Glu Met Ala Thr Leu
        115                 120                 125

Tyr Ile Ala Glu Met Gln Lys Met Gln Pro Gln Gly Pro Tyr Phe Leu
    130                 135                 140

Gly Gly Trp Cys Phe Gly Gly Ala Ile Ala Tyr Glu Ile Ser Arg Gln
145                 150                 155                 160

Leu Arg Gln Met Gly Gln Gln Val Thr Gly Ile Val Met Ile Asp Thr
                165                 170                 175

Arg Ala Pro Ile Pro Glu Asn Val Pro Glu Asp Ala Asp Asp Ala Met

180                 185                 190

Leu Leu Ser Trp Phe Ala Arg Asp Leu Ala Val Pro Tyr Gly Lys Lys
        195                 200                 205

Leu Thr Ile Ser Ala Gln Tyr Leu Arg Glu Leu Ser Pro Asp His Met
    210                 215                 220

Phe Asp His Val Leu Lys Glu Ala Lys Ala Ile Asn Val Ile Pro Leu
225                 230                 235                 240

Asp Ala Asn Pro Ser Asp Phe Arg Leu Tyr Phe Asp Thr Tyr Leu Ala
                245                 250                 255

Asn Gly Val Ala Leu Gln Thr Tyr Phe Pro Glu Pro Glu Asp Phe Pro
            260                 265                 270

Ile Leu Leu Val Lys Ala Lys Asp Glu Ser Glu Asp
        275                 280

<210>  201
<211>  73
<212>  PRT
<213>  Penicillium coprobium PF1169

<400>  201

Pro Met Asn Lys Asp Lys Val Tyr Trp Ser Ala Ile Ile Arg Thr Leu
1               5                   10                  15

Val Ala Lys Glu Met Arg Val Glu Pro Glu Thr Ile Asp Pro Glu Gln
            20                  25                  30

Lys Phe Thr Thr Tyr Gly Leu Asp Ser Ile Val Ala Leu Ser Val Ser
        35                  40                  45

Gly Asp Leu Glu Asp Leu Thr Lys Leu Glu Leu Glu Pro Thr Leu Leu
    50                  55                  60

Trp Asp Tyr Pro Thr Ile Asn Ala Leu
65                  70

<210>  202
<211>  63
<212>  PRT
<213>  Penicillium coprobium PF1169

<400>  202

Gly Ser Leu Ile Asp Ile Glu Glu Pro Ile Ile Pro Leu Ser Thr Met
1               5                   10                  15

Arg Tyr Ile Gln Gly Ala Asp Ile Val Arg Ile Ser Asp Gly Ile Ala
            20                  25                  30

Arg Thr Ser Arg Phe Arg Ser Leu Pro Arg Thr Lys Leu Arg Pro Val
        35                  40                  45

Ser Asp Gly Pro Arg Leu Leu Pro Arg Pro Glu Gly Thr Tyr Leu
    50                  55                  60

<210>  203
<211>  69
<212>  PRT
<213>  Penicillium coprobium PF1169

<400>  203

Leu Glu Val Val Trp Glu Cys Leu Glu Asn Ser Gly Glu Thr Gln Trp
1               5                   10                  15

Arg Gly Lys Glu Ile Gly Cys Phe Val Gly Val Phe Gly Glu Asp Trp
            20                  25                  30

Leu Glu Met Ser His Lys Asp Pro Gln His Leu Asn Gln Met Phe Pro
        35                  40                  45

Ile Ala Thr Gly Gly Phe Ala Leu Ala Asn Gln Val Ser Tyr Arg Phe
    50                  55                  60

Asp Leu Thr Gly Pro
65

<210>  204
<211>  96
<212>  PRT
<213>  Penicillium coprobium PF1169

<400>  204

Phe Asp Ala Ala Phe Phe Asn Met Ser Pro Arg Glu Ala Gln Gln Thr
1               5                   10                  15

Asp Pro Met Gln Arg Leu Ala Ile Val Thr Ala Tyr Glu Ala Leu Glu
            20                  25                  30

Arg Ala Gly Tyr Val Ala Asn Arg Thr Ala Ala Thr Asn Leu His Arg
        35                  40                  45

Ile Gly Thr Phe Tyr Gly Gln Ala Ser Asp Asp Tyr Arg Glu Val Asn
    50                  55                  60

Thr Ala Gln Glu Ile Ser Thr Tyr Phe Ile Pro Gly Gly Cys Arg Ala
65                  70                  75                  80

Phe Gly Pro Gly Arg Ile Asn Tyr Phe Phe Lys Phe Leu Gly Pro Ala
                85                  90                  95

<210>  205
<211>  58
<212>  PRT
<213>  Penicillium coprobium PF1169

<400>  205

Phe Leu Gln Ile Ser Gly Pro Ser Phe Ser Ile Asp Thr Ala Cys Ser
1               5                   10                  15

Ser Ser Leu Ala Thr Ile Gln Val Cys Thr His Leu Phe His Val His
            20                  25                  30

Leu Asn Arg Gln Leu Thr Ile Ala Ala Cys Thr Ser Leu Trp Asn Gly
        35                  40                  45

Glu Thr Asp Thr Val Val Ala Gly Gly Met
    50                  55

<210>  206
<211>  52
<212>  PRT
<213>  Penicillium coprobium PF1169

<400>  206

Glu Leu Arg His Gly Lys Asn Ile Asp Lys Pro Glu Tyr Ser Gln Pro
1               5                   10                  15

Leu Cys Thr Ala Ile Gln Ile Ala Leu Val Glu Leu Leu Glu Ser Phe
            20                  25                  30

Gly Val Val Pro Lys Ala Val Val Gly His Ser Ser Gly Glu Ile Ala
        35                  40                  45

Ala Ala Tyr Val
    50

<210>  207
<211>  59
<212>  PRT
<213>  Penicillium coprobium PF1169

<400>  207

Val Tyr Ser Gly Ser Met Thr Asn Asp Tyr Glu Leu Leu Ser Thr Arg
1               5                   10                  15

Asp Ile Tyr Asp Met Pro His Asn Ser Ala Thr Gly Asn Gly Arg Thr
            20                  25                  30

Met Leu Ala Asn Arg Leu Ser Trp Phe Phe Asp Leu Gln Gly Pro Ser
        35                  40                  45

Ile Met Met Asp Thr Ala Cys Ser Ser Ser Leu
    50                  55

<210>  208
<211>  28
<212>  PRT
<213>  Penicillium coprobium PF1169

<400>  208

Pro Trp Pro Thr Thr Gly Leu Arg Arg Ala Ser Val Asn Ser Phe Gly
1               5                   10                  15

Tyr Gly Gly Thr Asn Ala His Cys Val Leu Asp Asp
            20                  25

<210>  209
<211>  71
<212>  PRT
<213>  Penicillium coprobium PF1169

<400>  209

Lys Ala Ser Leu Ser Leu Gln His Gly Met Ile Ala Pro Asn Leu Leu
1               5                   10                  15

Met Gln His Leu Asn Pro Lys Ile Lys Pro Phe Ala Ala Lys Leu Ser
            20                  25                  30

Val Pro Thr Glu Cys Ile Pro Trp Pro Ala Val Pro Asp Gly Cys Pro
        35                  40                  45

Arg Arg Ala Ser Val Asn Ser Phe Gly Phe Gly Gly Ala Asn Val His
    50                  55                  60

Val Val Leu Glu Ser Tyr Thr
65                  70

<210>  210
<211>  80
<212>  PRT
<213>  Penicillium coprobium PF1169

<400>  210

Leu Lys Gly Thr Gly Gly Gln Met Leu Gln Asn Val Val Leu Arg Val
1               5                   10                  15

Pro Val Ala Ile Asn Ala Pro Arg Ser Val Gln Val Val Val Gln Gln
            20                  25                  30

Asp Gln Val Lys Val Val Ser Arg Leu Ile Pro Ser Glu Ala Ser Val
        35                  40                  45

Leu Asp Asp Asp Ala Ser Trp Val Thr His Thr Thr Ala Tyr Trp Asp
    50                  55                  60

Arg Arg Val Leu Gly Ser Glu Asp Arg Ile Asp Leu Ala Ala Val Lys
65                  70                  75                  80

<210>  211
<211>  38
<212>  PRT
<213>  Penicillium coprobium PF1169

<400>  211

Gly Asn Gly Ser Ala Met Ile Ser Asn Arg Ile Ser Trp Phe Phe Asp
1               5                   10                  15

Leu Lys Gly Pro Ser Leu Ser Leu Asp Thr Ala Cys Ser Ser Ser Leu
            20                  25                  30

Val Ala Leu His Leu Ala
        35

<210>  212
<211>  76
<212>  PRT
<213>  Penicillium coprobium PF1169

<400>  212

Ala Ile His His Gly Val Gln Ala Ile Lys Leu Gly Glu Ser Arg Val
1               5                   10                  15

Ala Ile Val Gly Gly Val Asn Ala Leu Cys Gly Pro Gly Leu Thr Arg
            20                  25                  30

Val Leu Asp Lys Ala Gly Ala Ile Ser Ser Asp Gly Ser Cys Lys Ser
        35                  40                  45

Phe Asp Asp Asp Ala His Gly Tyr Ala Arg Gly Glu Gly Ala Gly Ala
    50                  55                  60

Leu Val Leu Lys Ser Leu His Gln Ala Leu Leu Asp
65                  70                  75

<210>  213
<211>  69
<212>  PRT
<213>  Penicillium coprobium PF1169

<400>  213

Arg Glu Trp Met Thr Ala Glu Gly Lys Asp His Asn Leu Ser Asp Ile
1               5                   10                  15

Leu Thr Thr Leu Ala Thr Arg Arg Asp His His Asp Tyr Arg Ala Ala
            20                  25                  30

Leu Val Val Asp Asp Asn Arg Asp Ala Glu Leu Ala Leu Gln Ala Leu
        35                  40                  45

Glu His Gly Val Asp Gln Thr Phe Thr Thr Gln Ser Arg Val Phe Gly
    50                  55                  60

Ala Asp Ile Ser Lys
65

<210>  214
<211>  53
<212>  PRT
<213>  Penicillium coprobium PF1169

<400>  214

Thr Ser Asp Asp Tyr Arg Glu Val Asn Ser Gly Gln Asp Ile Asp Thr
1               5                   10                  15

Tyr Phe Ile Pro Gly Gly Asn Arg Ala Phe Thr Pro Gly Arg Ile Asn
            20                  25                  30

Tyr Tyr Phe Lys Phe Ser Gly Pro Ser Val Ser Val Asp Thr Ala Cys
        35                  40                  45

Ser Ser Ser Leu Ala
    50

<210>  215
<211>  63
<212>  PRT
<213>  Penicillium coprobium PF1169

<400>  215

Ala Gly Ile Pro Leu Ala Asn Ile Met Gly Thr Lys Thr Ser Cys Phe
1               5                   10                  15

Val Gly Ser Phe Ser Ala Asp Tyr Thr Asp Leu Leu Leu Arg Asp Pro
            20                  25                  30

Glu Cys Val Pro Met Tyr Gln Cys Thr Asn Ala Gly Gln Ser Arg Ala
        35                  40                  45

Met Thr Ala Asn Arg Leu Ser Tyr Phe Phe Asp Leu Lys Gly Pro
    50                  55                  60

<210>  216
<211>  68
<212>  PRT
<213>  Penicillium coprobium PF1169

<400>  216

Met Leu Ala Val Gly Ala Ser Ala Ser Asp Ile Gln Gln Ile Leu Asp
1               5                   10                  15

Ala Met Arg Gly Asn Lys Ala Val Ile Ala Cys Val Asn Ser Glu Ser
            20                  25                  30

Ser Val Thr Leu Ser Gly Asp Leu Asp Val Ile Ala Asn Leu Gln Thr
        35                  40                  45

Ala Leu Asp Lys Glu Gly Ile Phe Thr Arg Lys Leu Lys Val Asp Val
    50                  55                  60

Ala Tyr His Ser
65

<210>  217
<211>  39
<212>  PRT
<213>  Penicillium coprobium PF1169

<400>  217

Asn Ala Ala Gly Ala His Phe Leu Thr Glu Asp Ile Gly Leu Phe Asp
1               5                   10                  15

Ala Pro Phe Phe Asn Ile Thr Leu Gln Glu Ala Gln Thr Met Asp Pro
            20                  25                  30

Gln Gln Arg Ile Phe Leu Glu
        35

<210>  218
<211>  76
<212>  PRT
<213>  Penicillium coprobium PF1169

<400>  218

Gly Pro Ser Met Thr Ile Asp Thr Ala Cys Ser Ser Ser Leu Ile Ala
1               5                   10                  15

Leu His Gln Ala Val Gln Ser Leu Arg Ser Gly Glu Thr Asp Val Ala
            20                  25                  30

Val Ala Ala Gly Thr Asn Leu Leu Leu Gly Pro Glu Gln Tyr Ile Ala
        35                  40                  45

Glu Ser Lys Leu Lys Met Leu Ser Pro Asn Gly Arg Ser Arg Met Trp
    50                  55                  60

Asp Lys Asp Ala Asp Gly Tyr Ala Arg Gly Asp Gly
65                  70                  75

<210>  219
<211>  61
<212>  PRT
<213>  Penicillium coprobium PF1169

<400>  219

Gly Leu Val Asn Ile Leu Arg Ser Trp Gly Ile Glu Pro Ser Thr Val
1               5                   10                  15

Val Gly His Ser Ser Gly Glu Ile Val Ala Ala Tyr Thr Ala Arg Ala
            20                  25                  30

Ile Ser Met Arg Thr Ala Ile Ile Leu Ala Tyr Tyr Arg Gly Lys Val
        35                  40                  45

Ala Gln Pro Leu Glu Gly Leu Gly Ala Met Val Ala Val
    50                  55                  60

<210>  220
<211>  79
<212>  PRT
<213>  Penicillium coprobium PF1169

<400>  220

Ser Val Pro Ile Glu Glu His Ser Pro Val Val Thr Gln Leu Gly Thr
1               5                   10                  15

Thr Cys Val Gln Met Ala Leu Thr Lys Tyr Trp Thr Ser Leu Gly Val
            20                  25                  30

Thr Pro Ser Phe Val Met Gly His Ser Leu Gly Glu Phe Ala Ala Leu
        35                  40                  45

Asn Ala Ala Gly Val Leu Thr Ile Ser Asp Thr Ile Tyr Leu Ala Gly
    50                  55                  60

Arg Arg Ala Gln Leu Leu Thr Glu Gln Ile Glu Gly Gly Thr His
65                  70                  75

<210>  221
<211>  81
<212>  PRT
<213>  Penicillium coprobium PF1169

<400>  221

Val Tyr Thr Gly Arg Ile Ser Leu Lys Asp Leu Gly Met Arg Cys Leu
1               5                   10                  15

Pro Leu Cys Leu Phe Leu Phe Leu Trp Thr Ile Tyr Phe Asn Thr Ala
            20                  25                  30

Tyr Ser Tyr Gln Asp Ile Lys Asp Asp Cys Lys Leu Asn Val Asn Ser
        35                  40                  45

Ser Tyr Val Leu Ala Gly Ser His Val Arg Gly Met Leu Leu Leu Gln
    50                  55                  60

Ala Ile Ala Val Val Leu Val Ile Pro Trp Ile Leu Tyr Thr Ser Ala
65                  70                  75                  80

Ser

<210>  222
<211>  82
<212>  PRT
<213>  Penicillium coprobium PF1169

<400>  222

Arg His Phe Gly Leu Trp Asp Glu Pro Arg Glu Leu Glu Asp Val Glu
1               5                   10                  15

Phe Leu Leu Lys Ala Asp Val Arg Asn Asn Ser Ala Trp Asn His Arg
            20                  25                  30

Tyr Met Leu Arg Phe Gly Pro Arg Asp Thr Ser Leu Pro Asp Ala Gly
        35                  40                  45

Met Val Asn Ala Gly Asp Leu Ser Thr Ala Pro Ala Glu Lys Gly Arg
    50                  55                  60

Leu Ser Val Val Asp Glu Asp Met Val Asp Gly Glu Leu Lys Phe Ala
65                  70                  75                  80

Gln Glu

<210>  223
<211>  35
<212>  PRT
<213>  Penicillium coprobium PF1169

<400>  223

Ile Met Arg Gly Ala Gly Cys Ala Ile Asn Asp Leu Trp Asp Arg Asn

1                   5                   10                  15

Leu Asp Pro His Val Glu Arg Thr Lys Phe Arg Pro Ile Ala Arg Gly
            20                  25                  30

Ala Leu Ser
        35

<210>  224
<211>  86
<212>  PRT
<213>  Penicillium coprobium PF1169

<400>  224

Phe Pro Thr Phe Pro Pro Lys Glu Ala Asp Phe Leu Met Glu Met Phe
1               5                   10                  15

Ala Gln Asp Ser Lys Asn Tyr His Val Trp Thr Tyr Arg His Trp Leu
            20                  25                  30

Val Arg His Phe Gly Leu Trp Asp Glu Pro Arg Glu Leu Glu Asp Val
        35                  40                  45

Glu Phe Leu Leu Lys Ala Asp Val Arg Asn Asn Ser Ala Trp Asn His
    50                  55                  60

Arg Tyr Met Leu Arg Phe Gly Pro Arg Asp Thr Ser Leu Pro Asp Ala
65                  70                  75                  80

Gly Met Val Asn Ala Gly
                85

<210>  225
<211>  82
<212>  PRT
<213>  Penicillium coprobium PF1169

<400>  225

Asn His Arg Tyr Met Leu Arg Phe Gly Pro Arg Asp Thr Ser Leu Pro
1               5                   10                  15

Asp Ala Gly Met Val Asn Ala Gly Asp Leu Ser Thr Ala Pro Ala Glu
            20                  25                  30

Lys Gly Arg Leu Ser Val Val Asp Glu Asp Met Val Asp Gly Glu Leu
        35                  40                  45

Lys Phe Ala Gln Glu Ala Ile Leu Arg Ala Pro Glu Asn Arg Ser Pro
    50                  55                  60

Trp Trp Tyr Ala Arg Gly Val Leu Arg Ala Ala Gly Arg Gly Leu Gly
65                  70                  75                  80

Glu Trp

<210>  226
<211>  45
<212>  PRT
<213>  Penicillium coprobium PF1169

<400>  226

Arg Pro Thr Ser Arg Lys Leu Gly Val Tyr Pro Gln Tyr Ile Leu Gly
1               5                   10                  15

Ala Ser Ser Ala Leu Thr Ile Leu Pro Ala Trp Ala Ser Val Tyr Thr
            20                  25                  30

Gly Arg Ile Ser Leu Lys Asp Leu Gly Met Arg Cys Leu
        35                  40                  45

<210>  227
<211>  20
<212>  DNA
<213>  Artificial Sequence

<220>
<223>  a primer sequence for PCR

<400>  227
tacaggcggc ctaaattgtc                                                   20

<210>  228
<211>  20
<212>  DNA
<213>  Artificial Sequence

<220>
<223>  a primer sequence for PCR

<400>  228
gaacacagcg caagagatca                                                   20

<210>  229
<211>  20
<212>  DNA
<213>  Artificial Sequence

<220>
<223>  a primer sequence for PCR

<400>  229
cgcaagactt gaggaacaag                                                   20

<210>  230
<211>  20

<212>  DNA
<213>  Artificial Sequence

<220>
<223>  a primer sequence for PCR

<400>  230
tgaggtcaac agtggacagg                                                   20


<210>  231
<211>  20
<212>  DNA
<213>  Artificial Sequence

<220>
<223>  a primer sequence for PCR

<400>  231
cgcttttacg gcaatcatct                                                   20


<210>  232
<211>  20
<212>  DNA
<213>  Artificial Sequence

<220>
<223>   a primer sequence for PCR

<400>  232
tgttcgtcgt ccttgtatgc                                                   20


<210>  233
<211>  20
<212>  DNA
<213>  Artificial Sequence

<220>
<223>  a primer sequence for PCR

<400>  233
cagacgctgc ataggatcag                                                   20


<210>  234
<211>  20
<212>  DNA
<213>  Artificial Sequence

<220>
<223>  a primer sequence for PCR

<400>  234
ttactagcct ctggggtgga                                                   20


<210>  235
<211>  20
<212>  DNA
<213>  Artificial Sequence

<220>
<223>  a primer sequence for PCR

<400>  235
tctcttgcgc tgtgttcact                                                   20


<210>  236
<211>  19
<212>  DNA
<213>  Artificial Sequence

<220>
<223>  a primer sequence for PCR

<400>  236
atgcggcctt tttcaacat                                                    19


<210>  237
<211>  20
<212>  DNA
<213>  Artificial Sequence

<220>
<223>  a primer sequence for PCR

<400>  237
cgacgtaagg agctgtgagc                                                   20


<210>  238
<211>  18
<212>  DNA
<213>  Artificial Sequence

<220>
<223>  a primer sequence for PCR

<400>  238
acctcgatcc tgctgcaa                                                     18


<210>  239
<211>  21
<212>  DNA
<213>  Artificial Sequence

<220>
<223>  a primer sequence for PCR

<400>  239
ggttgtcagg atttgtcaga a                                                 21


<210>  240
<211>  20
<212>  DNA
<213>  Artificial Sequence

<220>
<223>  a primer sequence for PCR

<400>  240
ttacttcatc cccggtggta                                                   20

<210>  241
<211>  20
<212>  DNA
<213>  Artificial Sequence

<220>
<223>  a primer sequence for PCR

<400>  241
agagcatagc ccggttgtta                                                    20


<210>  242
<211>  20
<212>  DNA
<213>  Artificial Sequence

<220>
<223>  a primer sequence for PCR

<400>  242
cccaccttga tttgctcagt                                                    20


<210>  243
<211>  20
<212>  DNA
<213>  Artificial Sequence

<220>
<223>  a primer sequence for PCR

<400>  243
agagcatagc ccggttgtta                                                    20


<210>  244
<211>  20
<212>  DNA
<213>  Artificial Sequence

<220>
<223>  a primer sequence for PCR

<400>  244
gccaccttga tttgctcagt                                                    20


<210>  245
<211>  22
<212>  DNA
<213>  Artificial Sequence

<220>
<223>  a primer sequence for PCR

<400>  245
aagaacacag agattggtgt gg                                                 22


<210>  246
<211>  20
<212>  DNA
<213>  Artificial Sequence

<220>
<223>  a primer sequence for PCR

<400>  246
ccaggaagac acttggaagg                                                    20


<210>  247
<211>  20
<212>  DNA
<213>  Artificial Sequence

<220>
<223>  a primer sequence for PCR

<400>  247
agagcatagc ccggttgtta                                                    20


<210>  248
<211>  20
<212>  DNA
<213>  Artificial Sequence

<220>
<223>  a primer sequence for PCR

<400>  248
ccaccttcga tttgctcagt                                                    20


<210>  249
<211>  20
<212>  DNA
<213>  Artificial Sequence

<220>
<223>  a primer sequence for PCR

<400>  249
cgcaagactt gaggaacaag                                                    20


<210>  250
<211>  20
<212>  DNA
<213>  Artificial Sequence

<220>
<223>  a primer sequence for PCR

<400>  250
tccctctacg cagaagaacc                                                    20


<210>  251
<211>  20
<212>  DNA
<213>  Artificial Sequence

<220>
<223>  a primer sequence for PCR

<400>  251
agagcatagc ccggttgtta                                                    20

<210>  252
<211>  20
<212>  DNA
<213>  Artificial Sequence

<220>
<223>  a primer sequence for PCR

<400>  252
ccaccttcga tttgctcagt                                                    20


<210>  253
<211>  20
<212>  DNA
<213>  Artificial Sequence

<220>
<223>  a primer sequence for PCR

<400>  253
gaggcgctgg tgtagagaat                                                    20


<210>  254
<211>  20
<212>  DNA
<213>  Artificial Sequence

<220>
<223>  a primer sequence for PCR

<400>  254
tgtctcccgc tttgtctctt                                                    20


<210>  255
<211>  20
<212>  DNA
<213>  Artificial Sequence

<220>
<223>  a primer sequence for PCR

<400>  255
agacgtggag ttcctcctga                                                    20


<210>  256
<211>  20
<212>  DNA
<213>  Artificial Sequence

<220>
<223>  a primer sequence for PCR

<400>  256
caaacttcag ttcgccatca                                                    20


<210>  257
<211>  20
<212>  DNA

<213>  Artificial Sequence

<220>
<223>  a primer sequence for PCR

<400>  257
caactttccc acccaaagaa                                                    20


<210>  258
<211>  20
<212>  DNA
<213>  Artificial Sequence

<220>
<223>  a primer sequence for PCR

<400>  258
aagcatgtat cggtggttcc                                                    20


<210>  259
<211>  20
<212>  DNA
<213>  Artificial Sequence

<220>
<223>  a primer sequence for PCR

<400>  259
cgatacatgc ttcgttttgg                                                    20


<210>  260
<211>  18
<212>  DNA
<213>  Artificial Sequence

<220>
<223>  a primer sequence for PCR

<400>  260
cagcagccct cagcacac                                                      18


<210>  261
<211>  20
<212>  DNA
<213>  Artificial Sequence

<220>
<223>  a primer sequence for PCR

<400>  261
ggcgtctatc cgcaatacat                                                    20


<210>  262
<211>  20
<212>  DNA
<213>  Artificial Sequence

<220>
<223>  a primer sequence for PCR

<400>  262
gagacaccgc atacccagat                                                   20


<210>  263
<211>  406
<212>  DNA
<213>  Penicillium coprobium PF1169

<400>  263
cccagcccaa gacttgagta gactatattt attctctttg atatccatct cagcatcaag      60

tttttgacgt tgtattacta tcctcgtttg gaattctcct cccaggtctt gcttcattgc     120

ttatagcatt ctaccaaaaa cgtcactgtc atggacgggt ggtcagacat atcatcagcg     180

cctgccggat acaaggatgt tgtttggata gcagatcggg ctctgctagc ccaaggattg     240

ggatggtcaa tcaactacct ggccatgata taccaatcgc gcaaagaccg cacatacggc     300

atggccattt tgccactatg ttgcaacttt gcgtgggaat tcgtctacac tgtcatctat     360

ccttctcaaa atcccttcga gagagctgtc ctcacaacat ggatgg                    406


<210>  264
<211>  20
<212>  DNA
<213>  Artificial Sequence

<220>
<223>  a primer sequence for PCR

<400>  264
cccagcccaa gacttgagta                                                   20


<210>  265
<211>  20
<212>  DNA
<213>  Artificial Sequence

<220>
<223>  a primer sequence for PCR

<400>  265
ccatccatgt tgtgaggaca                                                   20


<210>  266
<211>  39008
<212>  DNA
<213>  Penicillium coprobium PF1169

<400>  266
gccagccaat gtctcgacga gactctcggt gtcaagctgc ttgaggaggc cattgtgaag      60

atcgaagagc gtatcaggtc gcacggcggt agctgcaccg tgaagatggc acccaaggcc     120

gtcaccgagc aggacgatgc gatcctgcag gagcttatgg agaagcgcga acgtgagaac     180

acccaggtca gcggagatga ggactctgaa agtgatgagg gtgttcccga gtaagcgacg     240

ggctacaaat tcgagtcgag gggcatacag cggtcaccag cgctaaaatt caaagctggt     300

atcaccgcta gaggggagtt ggtgaaagat ggatagaaaa aacttgcaca tatcggaaaa 360

aaggctcgat gggccagtgt gctgatgggc aggattacag tcagaactcg cccaggtaag 420

tcgcctggac ttcggggtct ggatatgaca tattcacacc tgtgtatgcg gtattcccat 480

tgcggtcgaa atcctcgttc ccggcatcaa atacactggg tccgcacagg gtgcaagttc 540

tgatgcacat aatgtttgat gcaaccgata cgttcaatgc cagtcatgct tttagatgca 600

attatccctg tagaggccat gtagcaatgt atgtagcaat gtatgtagca atgtatatag 660

caatgtatgt agcaatgtat gtaagatatc ataacaatcg agctcatgaa atggcgggga 720

gagctgaagc ttatctaccg ccgccgatca ttggtgccct caaagccatc gagaacttcc 780

ctttcggcac ttctcttttt ccaccaactt tcattctacg cgatatggga cattgggcaa 840

agatctttac cgccgatcgt ggcaggaccc ggcatcgggt cgaggtggaa cgtcgtcggc 900

gtacgtcatt ttccaaacat gcggaacact actgacaagc cgcagtgcta ccggcctatg 960

cggccgatga gtctgacgcc tcggatgctt caaaggaaat tgcaaaggtt gctcttcggt 1020

tgaaatatca aattgagcag gttgtctcct gtgaagtgga ggagaacgtc ttgaccgacc 1080

caaacagccg tatcatcacg gatgatgtgg ttgcgactgc taagcaggcc ggtggagatg 1140

aatacaaagc atgcattgtt tattgtctcc tggtttgtct gcgatggttc aaaatccaat 1200

catccgtcga gctttgggat tccgatctcc atgagattcg agctgtggct tgcgaggtca 1260

tcgccaagcg catgtaatgc ccctttttca ttccatgttc tcggccattt cctgacccaa 1320

acagtatcga atccgagcag aaccaagaat acgtgctaaa agacatttta ctcaagcgat 1380

actcaatctt cagtgaaggt gtggagactg atcccgccaa tgtcattgaa cgatcggtag 1440

atctccatgc tttaaggatc atcagctgtg ctgcgtacca gaagtgtatc cagtatctct 1500

ggagaggttg gatctgccag gaagaaggca acccaactaa ctttgtcgaa tacagtgaga 1560

agtcaaaccc caattattgg gttcatttcc atcctgatcg gatgcggact cctctgtatc 1620

agaatgtctg ccaaattttg ttttccttga tttaccttgc gacttatacc gcagttatca 1680

ataccgtgaa tcccaccggt gacctggatg tagctgaagc catactgtat gttatgactc 1740

tcgcgttcat ctgcgacgag gcggtcaaat tctggaaggt tggatggaat tatctcgaat 1800

tctggaatgc gttcaactca acgctctact ctatcctggc agtgtctctt gtcttgcgct 1860

ttattgcctt ggcacactca tcatctacgc acgatgaaac aaggcaggca tacaatgaac 1920

tcagctacaa cttcctcgcc tttgcgggcc ctatgttctg gatgcggatg atgctatatc 1980

ttgactcgtt ccgcttcttc ggtgccatgt tcgtggtcct tcgagtgatg atgaaagaaa 2040

gcttgatatt ctttgctctt ctattcgtgg ttatggctgg tttcttccag ggcttcgtcg 2100

gcatggccca agtggatgct gatatcccca tccaccgaaa tattctccag ggaatgatca 2160

atagtatcat gcaaagccct gagtttgaca cttttcagga atttgcattt ccctttggta 2220

tcatcctcta ttatgtgttc aacttcattg ttatgactgg taagtctgta ttacatttgt 2280

ttggggtgtc gctaaacatt tttagttctg ttgaatattc tcattgcctt gtacaacagc 2340
gcatatgaag atatctctgg caatgccacg gacgagttca tggccatctt cgcgcagaaa 2400
accatgcagt tcgtccgcgc cccagatgaa aatgtcttca tcccacgtac gtgtttactc 2460
aattctgata tagcatacgt atgactaact ttggtctggg taatagcctt caatctcatc 2520
gagattctct gtttgatagc tccattcgaa tggtggcttt cgcgggagac ttacgccaag 2580
gtgaatgaca ttgtaatggc cgtgatatat tctccgctgc ttgtcgttgc agcctgggtt 2640
gagacccgtc aggcgcataa gattcgatgg aatcgccgtc atggcgaaga agacgatgac 2700
tgcgctcagg aatgggagca tgtggccaag gaggtcaatt ttgatcttga cgatacctgg 2760
aaacagcacg taattgagtc cacgccggat atcaaggttg atagttgtac atatgaactc 2820
cgagagctga gggagcaggt taaaatgttg acggggatgg tgaaggaatt gactcaggag 2880
atggaaaaga aggcggatgg agcaagctag gaagtcctgt tgaattgtac agcaagaata 2940
ctacactgag catgggacat cgcaaaggtg atttgctact gcagtttcac caatattaca 3000
ttgcgaaaac tgtatattct cttaatgtct aatagcagca atcagcccag tggcacggag 3060
gaaagtcacc gtcctgtaag gcaaatactt gtgcttcaaa tgaattttga ctatttttca 3120
tgcgataact ggcaaagggc agggggagaa aaaatgatca ttattcaacc caagcaaact 3180
gtccagaaag tgacatgccc actttgcaag taaagaagat atgtgacaat ctaacagtct 3240
caggtagaca ttcgctcttc attaaaatcc atgcgttgct cgccgtagcc caattcgaag 3300
cactgggcaa cccacatcga gaccttaaaa tcgggtgatc atcacacagc aacaggctca 3360
gcaagaatgg aggcaatcgt ctccctttga tgatccagct gtgagagctt cgctcgatgg 3420
tgcttgccaa tacctatccg aggaatgtca tccacaaata caacacctcc atctagggct 3480
ttatagctgg ccagttggct ttgaatcaga cctgccactt gatcggccgt cgtctccggg 3540
gacgtatcat tgcggacgac ataagctcga ggaacctcgc tgctgccatc tgggagcatg 3600
actccgatca cggctgcgtc cttgatactc gggtccttgc gtaggatccc ttcaatctct 3660
gcgggagcga cggagtatct aaatcaagca cgatatgtta gtctatcatc tgctgcatcg 3720
gatgtcatat ggagaggaaa gaaagcgaag gatgtgaagg atgaagccta gaggactggg 3780
taacttgccc tcgaactttg atgagatctt tggtccgtcc gatgacatgg tagtttccgt 3840
cttccacatg gaacatgtct ccagtccgga accatccttg ctcatctttg gcatcagttc 3900
gtcctttgta tgctagaagg agtcccggtc cacggacata caactctcca ggggagtctg 3960
gtgtcccggc gacatcttcg cccgtgtcgg gattgacaaa gcgcagctca tatctgggca 4020
aaagagtccc tacactgcca aattgtggtt gtatcccgta gcgattctgg aaaaccactc 4080
caacctcaga catgccccac agatttcccg ctatagcgtc cggtgatagc aggctctgga 4140
attgctgcat agagtacccg tctatgggag cacccgaaat accgatatag cgcagagaag 4200

acaagctctc ggctacattc aaggaggacc tattgagaat gtggatcatg gcaggaacca 4260
tgtacgtttc cgtgatgtgg tgctggcgga tgccgtcgag caaagcggtg atttcgaagc 4320
gcgggataat gtacagaggc tggccgtacc gaatggggaa gatgttgccc cagaagtcgc 4380
caaaagaatg gtacagtggc agtgccatca aacgaacgac ggggtatggc acttcatagt 4440
agacgctcag atggtgggaa atgatcgtgt ggtgggttcg aattgcggct ttggggagac 4500
cgctggtgcc actggttagg aacatagccg ccggcgtgat cttgctctcc tcgctatctt 4560
cgaaacgaag ccaatccaac tcgccatact ggagcagact ctccaggcgg ataggttggt 4620
ccactgtctg ggtgtcgaga tcctccgtct gctccgcctg gccatgtgca aattggacta 4680
cactttcgat agacttctca tccatcagaa ggacttggtt tgaggacatt ccttgattat 4740
tgcaaacttc caggactctg gtcagcgcac tcggagcagt aataatcaac cgaggctcgg 4800
cgacacgaag cagatgagcc acttcatggg ggcgactagc gacatcaaac cccatataca 4860
ctccgcccgc accaacgatg gcaaagaaaa gagcagagtg tagaacctag gctgatgtta 4920
gcagcatatc attgttatgg ggtagtgtga ttacactgtt ctccagttgc acgagtacac 4980
aatcgcctcg ttccacaccc cgggctttga ggcccgcaat gagtgatcgc accagccgtc 5040
ggaattggat ggcattgaaa gcacgcgaag ggttgcgggc atcaatatag atgggcttag 5100
attggtcaaa ggcaggacca ctaaaagcaa agctgactag gtctgtctcg tgctccatat 5160
cgatgcttat attgtacagt tctcgtgtgc tattgacatg cagaacttga tgcaggattt 5220
gtgctcactt taagtagtag tacatggaat gctcagacct cccatatcac tttgatcgac 5280
actgcacggg acaagtatca tgcagaagac tattgagaag aatgccacgc caccaattcg 5340
tattatacta atctagccta agccaataca tgtaaagagt actatttagg acccacactg 5400
tcattgcaga gctttgaagc agctgcatgc gctaattcac ccacagatac gccactaaga 5460
atcaaaatta ccccgatgtc gacgctcagc tctttcgtaa accattgact cagcccaatg 5520
gcgataagcg agtcaatccc gagctcagga atcagcgtgt cagcagagag cggtgcatcc 5580
tcggccaagt tcaagctggc ccgaattttc tccattagtg gtctcacgac tgcttcggct 5640
ttttcttcca agcttgtcgc tgcagtgaca agatctttgg tcgaccgggt ctcaatcaat 5700
gcaagtattt gatcttgaga cgccgtggcc gtataggagt agaatggcca taacttcggt 5760
atcgggcact cgccatagcc acacttcaaa ctctggtgtc ggagtccccc gattaactca 5820
gcgttggaat tggaatctga gcgcccgcag aggattgcct cggcgagtat ctcgtcgaca 5880
tcccgctggg atacagctac cgggccacac caaagaggct gactgggaga aggactagaa 5940
atgccgtgga tctcgcccag atgtactaga cttgctggtc tgccctgggc tcgacggtgg 6000
cggaccagca gggccatttt ctcggacatc gctgcagtca ttgcctggtc cgcatggcct 6060
aacactcctg caatagatcc gatgagcacc caaaagtcca gagttggggt cttgtagagc 6120
tcatctagct gctgcagccc cttcaagacc ggatgcagat ggttccggag ggaatctatc 6180

gtgagctggg acaaggaaca gtcaggtaga ggcggaggct gaattagaac tcctcccact 6240

accgggggga acgcataggg aatagtttga tgcaaactgg tggcagaaat gccatcgatc 6300

aggtttctgg cattgattag cttgtgcatt tgaattcatg ggtacgtaag taacttacat 6360

tttcgagacc gctatgcgcg ttccccgtcg agaaacttct tccaaccacc acgcatctga 6420

gtcaagtcta gagccagcaa ggaggatcca ttttgccccg tgtgttgcta gccaaagaca 6480

gatagcatgg gctagttcac tgcctagacc tacaagtatg taggtttttt tctctgacag 6540

ctgcacctgg gaaccagcag tcggtatctg ggcgagcacc ggagttgtag agtcccaatc 6600

taccacggca tcctgggagt caaggattgg gtactcggaa atcctgctga ttggtaacga 6660

gtccacagaa tttggtggga gcccctcgcg gccggtgtac gccactaagc aggcggttag 6720

gaaagccttg gctatgagtg acgaatcatc cgcgttgatc ggccctgtcg atgcagaggt 6780

aaggtagaaa tcctgcaaat ggattcgtgt cgcgttgtca ggcaagagcg atagcatgcg 6840

atcatagaca ccctgtccac gacgatgtag aatcgctatg gccgacacat ctgagggaag 6900

tacctgagac aattgccgcg cagtgctatg ctcgtgcaaa agcaacatcg gtctttcttt 6960

gtctgggttg cttttgctag tgctgaagat aaccttacca tcccgccggg tcagcatttg 7020

gtggaaaaca gactgaagga ccccatcagc ctcgtgcact acaagcgtgc ctgattgtgg 7080

gacttgttcg accaggtatc ctgccaacag agcagccgct gtggcgcgga gataagactg 7140

ctcgtgggct tccaacaccg tgtctggcac tgaccacgcc caggagtctg gaacgataac 7200

atgagatgca atgtgggacg acagagcgat cattctcttg ttgctcttaa cgtccagccc 7260

tatgaccagc cgcagaaata tggcccctgc aacccgcacg gctgctatac tggaatgtcg 7320

aactcgcaga tgaagggttg ggccataact tgcggttatc ggcggatcag ccatcgaaag 7380

aaggcggaac ccttcacaag tcttgtcggt cgtagcagga aggatttgta caactccctt 7440

gtcgaggtcc acgcagtctg tcactttttg ccgcctggct agatggcgca gcccagtagc 7500

atggtcgtga tattgccgag gaacacggaa catagagccg tcgtattgaa tttcgggctc 7560

gatgttggtg agtccgcacg aatgaggatt ttcttgagct gcactagctt gaacaaagtg 7620

tccaagagct gtcgccaata tttccgttgt aactccaacc ggatctgtga tatgtagcag 7680

ttgtagcaga gaagacgatc gttcagaagc caggaaggtg gaaaggagac ctttcaccag 7740

tccggcatca gggtggtcga ccttctcaca ggtcaccact aacattcgct tactgactgt 7800

catcagtctg cacaactcgc tcaaggtagc gtttgtcaga tctcgatcat cgacgaggta 7860

cagaacagtc aatttagata aatcgcgatc ttcaatgaga tctaggtctg gcgcatgagc 7920

aaccttgacg aagtcatcct gtaccaattc aaaaagctct gaggtaagac aatctgcctc 7980

ctcagcgtca ccgccaagca acagcaggtc tccgcggctc tgtggttccg caggactgtc 8040

tggagtgcga cagagaagga gtgaaaagtc cccaaggctt tcactctctt gggacgcatc 8100

gaacgaatcc aagccataga atccgccgtt agacagtagc tcaacccagt ccctcctggt 8160
agtaattggc tcaccagagc agtagccttt cccggtctct gtgcatctca ttggaggacc 8220
gaatagaaga ttcaaatatg tagtgctggg attcgttcgt actagtagga ccaagaatcc 8280
accaggcttg agcaagcgac gaacatgagc caccgcgacc tcttgcagaa ataccgcggc 8340
tgtgatcagc accatatcgt agaattgctc gcggcagccc tgctcgacag gatcctcatt 8400
gatgtccaac gttttgtgcg acacctcgcc aggttgctca aggtcttcct caatcgcttg 8460
taggccagaa acggagagtc cagcataagt aaatgaccga taagtccgac ccatcttctt 8520
cagtccagag tgaacatggc ctccaaattg gccgatctga aggatattca tttgtggaaa 8580
gcggaaacac gcctggctga cgacagatac gagctcgtct tctagatcca agacttgcaa 8640
gtcctccttc agatattgac tttcctcatc gatggccggc caggcctcta tttgaagacc 8700
agaatcacgc agaacgcgag gtagccgctg gcctaccgca gcaatggccg tgagaccagg 8760
gtcattcaag agtgacgggc tcactccagc tgtgaagtcc tcaatcttct ggtccaagca 8820
ctcggattcc ccgacagggt ctggctcttg gctagcattt gcaatgcatt ggttcatcca 8880
tgcaagcaga cgagcaccat cgaaatccaa tccgcttcgc tccaagtccg tcagtccatt 8940
gcgagcctgc ttgagataca gtagcgcaag ctcctctcgg agagagtgta gctgtagcat 9000
ggtagctggc aacttccgtg atcccttctt cagagtgggc tcaagcggtc cccacgcggt 9060
ctgggaaaga acctgcaagt tgtttggtgc agttccagat ggctggcata tgagagaaac 9120
accttcgagc tggacagcct tttccccatt catggtgaag atgtcaatat caccgcgaat 9180
tcgatctcca ttaacgcagg tcagatagct tgctaccgtc aattccttgc cttgccaatc 9240
tgaagcacat aacaccggat ttatccaggt actgtcaaca tttctcgata agaatggtcc 9300
cgtcagcagc gtctcttcaa gcccaccaat tgcagcaatc attgtttgaa caccaaggtc 9360
caaaatagcc gggtgaagag ccatgggctc atccgaatca tttgagggaa cgggcacact 9420
cccagtggct agatcacgcc ttttgcggag tcccgtcaag gtagagaatg ggccagtaca 9480
gtggtagtca gcgcggcgca ggctgtcata gaattcagtg ctgtccacgg gctccaaggc 9540
ctgaggtagc tgtccctgtg ggggtaggag agcacggtca gaatcccctg gatgcatgat 9600
catcttggct gttgcacact gaacgagctc tccggataca acagcttcgc agcagaacca 9660
agcagtaatg gctccatcat gcgagtgaat actacccacg gtgacaagca cttcagtgcc 9720
gatgggatca ttctgaatcg ggagctgagt gtggatggtc aagtccttga cattcaacaa 9780
gcgtaggcct tgtgtctgtg ccattatcat acctgcctcc agtgccatcg atatgtatcc 9840
tgtctcaggg aagacagatc ccgaatcggc acgacggtcg gccaaccagg gcagctcctc 9900
tggtcgtaga tagtttcgcc aacggaactt ttctgctccg gtctctggac tgagagaacc 9960
gagaagtgcg ttaggagatg tagcacgatg gttatggttc gaagacattc gcgactgtgt 10020
ccagtatgtc tgagtatggt cgaaggggta gaatggtagc gattctacca acacaggcca 10080

atgatttgga tcaaagagtg agacatagtc tgtgaggcgg acgacatttg ggccgaggtg 10140

tgcccaggaa gatcctaggg ccgttgccca tgtatcgagg ccgggctttc ctcgctcagc 10200

aagagcaagg taaggaattg ccgagtgggc cgagtgcatt ttggagaggg tctgtaggac 10260

aggccctctc agtgtcggat ggggcccgat ctcaatgatg agatctggtg gcccagcgtc 10320

tcgtgccgcg gcctctaggg cttgggaaaa ctgaacagga cgcagcatat tctcaaccca 10380

gtactcccct gtcaattcct gctggtcata cccagtcatg acctcccctg ggtagacact 10440

cgagtaccag cgcgaggcag aagctgacag ggcgacagga tacgctttca ttgcgtcacg 10500

atatggatct gcacaaggct tcatatgcgg agagtgatat gcggtgtcca ctcgaagcat 10560

acgcggagtg aggcccaggc tcttcagcag ccactccagc tcccgcaggc actctgcgtc 10620

gccggataac gtgacgctgg atggcgagtt ggcggcagca acgcttatac gtccggagta 10680

ggcttctaaa gcacagatat tctgcgcctg ctgccatgtc aaattcacgg ccatcatccg 10740

acctgtcgga tcgcgtgact tatcaatggt catcccccta aggtacgcga tgcggattgc 10800

atccgaggcc gtcagcacac ccgcagcata ggctgctaca atctcgccgg aagagtgacc 10860

gaccacaatg gtaagctcaa tccctaccgc acggagcatg ttgacttgca tgatttgcaa 10920

cgctgtccgt agggggagag aaaggaggcc ctcgtttacg cgcgaggacg atgccggctg 10980

tgacaactcg tcgagaagag aaaactgtgg acgaaggtct agtggaagct catccaaagc 11040

ttcctccaga ttcataatcc attttcgaat tgagggactt gcctcaatca gatcaagtcc 11100

catttgtggc cattggactc cttggcccgt gaagatgccc atgacgcgtc tgggccgagt 11160

gttggatctg gagacgacag aggctggttt acccgtgacc cttcgactta tttctgtatt 11220

gatctggtct ttcaactctt gtattgagtg tgccattagc gtcaaccggt ggcgatgagt 11280

ggaacgtcga tcccacaaag agagcgccag accaacgaga ctgactgttg cgtgttcctg 11340

gaggaatgtt gcgtatgatt ccatcacaca agtgagggtc cgctcagacg cagcagaaaa 11400

gacaaagggc agactggagg gtatgttgtt ggacggactg agctccgagc gagtgtagct 11460

ttctaggacg acatgcacat tggcaccccc gaatccaaag gagttcaccg aggctcgacg 11520

aggacagcca tctgggactg caggccacgg gatgcattct gtggggacag aaagcttggc 11580

ggcaaacggt ttaatttttg gattgagatg ctgcatcagg agattaggag caatcatccc 11640

gtgttgcagc gagagggatg ccttgatcaa tcccgctagt ccagcagtgg cctctgtatg 11700

tccaattatt gtcttaattg acccaacata cagccgatcg gtcgaatctg gaacggattc 11760

gggcccaaag aagcttgagt tgattgcggc tgcttcctgc ggatctccgg cctgggttcc 11820

cgtgccatga gcctcgaagt actggcaccg atcttccggg ttgttttgag gagagagccc 11880

cgcgcgtgca taggttgcga ggatcaatga ttgttgtgcc tttggattag gcatcgtgat 11940

ccccatagtt cgcccatccg agtttgcccc tgaggcacgg atcacgcatt cgataggatc 12000

tccatcatta atcgcgtcct gcagacgttt tagtacaacc gaagccacac cctcgccacg 12060
tccgtagccg tcggccttgc tgtcccacat tctactccgg ccggtagggg acagcatccg 12120
tgttttagaa tccgcaatat aggcattggg agacaggatc aggttgcttc ctactgccac 12180
tgccatggaa cagtcatcgt tctgcagagc ctcgactccc agatgaacag ccaagagact 12240
cgaagaacat ccggtgtcaa cggccataga aggaccttgc cagtcaaagt agtaagagat 12300
acgattggcc atgattgacg gtgagtttcc cgtaaccaca tacgcgggga acgcctgagg 12360
atccatggcc tggatttgat tgtaatcgtt gcgaagtgta ccgcagaaca ccccggtctt 12420
tgagcgctgc agcgcatcca tccgtaaccc ggccgcatcg agcgattcgt acacggtctc 12480
taggagcaat cgctgttgtg gatccattgc taccgcttca gttggcgaga tattgaagaa 12540
ggccgcatca aaggctttga tgtcctcgtc caagaagtat gactctttga cgtttgttgt 12600
gccatggtgg tctccatctg gatgataaaa ggcatctata ttgaatctgt cggccggaac 12660
tttgcgcgcg atatcccgag ggctttgaag aagctcccac agtttcgaag gagaggaagc 12720
gccaccggga aagcggcatc ctgtaccaat aatagcaaca ggctctgttg ctttcattgt 12780
gagattataa gagaggtgta aaacctgaga tcaaaataat ttgcagttgg gtggctgtag 12840
ctctactgag agtacgttca tagatataag caatgcagtg ttgccttact tacttccacg 12900
atcttgtcag catatctatc gaacgaatag caaaactgga cctatagagc aatttccggc 12960
catcgataga tcattggata gctgtcctat ttgggaagta tgatctacaa tttatgcagc 13020
cacaaactat acaaagtggt ccatcgccag atttggcgat gagcagcggt gtggaatagt 13080
gactttgatg aacatgtcag gtcctgcatc tacatgtgca ggtgtccaag gatgctcctt 13140
gcgcgaagaa gtggagtagg gacattcagc tacctcctta tcttttccct tcttttaatg 13200
ctcactctgt gcataataat agtggcgaat atcgaagcat cgaaatccaa cgacattgag 13260
acaacatgga taacatggac aacatgaaca acacaccttt aggtttcaac tgggcctggg 13320
cagtcatcat ctctttcctg ggtctgctga ctttttcctt tgtctcgcca cacctctttc 13380
cttcaagatt gacggtgatt aatggtggaa gagcctggga tatctttcgt accaaggcca 13440
aaaagcgatt tcgctcggac gcagcacgtc ttataaagaa cggcttcgag gaggtgagta 13500
tggaaaaact gcatcattta ggataaagtg ctaaacgttc cttcttactc cagtctcctg 13560
atgcctttcg cattatcacg gataacggtc ctttgctggt cttgtcacct caatacgctc 13620
gtgaggttcg cagcgatgat agactcagcc ttgaccattt cattgcctcg gtttgtcttg 13680
cttcatgtcc aacgtttttc tagttggcgt cgctaagctt ctactgttta ggaatttcac 13740
cccaacatcc caggtttcga gccgttcaaa ttgatcttgg atccaaagaa cccgttgaac 13800
acgatcctca agtccaatct cacacaagca ctgggtactg acatcgtcct ctccgctctt 13860
atgcagccca ttacatagct aacattgttt acctggatag cttatctgac agaggacttg 13920
tctgcggagg taacagaggc actatctgca acctgtaccg atgaccctgg taagctataa 13980

aacatggttt tccaaaggtt ctggtatcaa tactaacttt ctttcttctc ttaatcaaag 14040

agtggcacga ggtcagcgtt agtcaaacgg ctctcaaaat tatcgcacaa atggcgtcca 14100

aagccttcat tggacaagaa agatgccggg atgccaagtg gcataacatt atcatcacgt 14160

acacgcacaa cgtctatgga gcagcacagg cactccactt ttggcccagt ttcctacgac 14220

ccatagtggc acagtttttg ccagcatgcc gaactttgca ggctcagatt gctgaagcgc 14280

gagagatctt ggagccattg gtagcccaga gacgagccga gagagccacc cgagccgctc 14340

aggagaagcc tcatccgtct ggtggggata tcattgactg gctggaacag ttttatgggg 14400

accaaccgta tgatcccgtg gccgcacagc tactgctctc atttgctgct atccatggaa 14460

cttccaatct cctggcgcaa gcgctcatag atctctgtgg ccaaccggag ctagtacagg 14520

atctccggga agaagctgtg tccgtgctgg gtaaagaggg atggaccagg gccgccttgt 14580

accaactcaa actaatggac agcgccctga aagaaagcca gcggttggcg ccaaacagat 14640

tgtgtgagtg ggcccttcct cttgcccccc aatttgacca ttcaactggc cattagagac 14700

taattcaggt gtgcttttac agtatcgatg ggacgcattg cgcaaggcga tatggacctg 14760

tctgatggtc tccgtatcca ccggggcacg accctcatgg tgtctgccca caacatgtgg 14820

gatcctgaaa tctaccctga tccccgaaaa tacgatggct accgattcca taagttgcga 14880

caaacatcag ggcaagaggg ccagcaccaa ctcgtatcct cgacgccgga tcacatggga 14940

ttcggatacg gaaagcatgc ttgcccggga cggtttttcg ccgcagccca gatcaaagtt 15000

gcattgtgca atatcctcct caagtatgat attgaataca ggggtggcaa gtccccaggt 15060

gtgtggggtc agggcataca tctgtttccc gatccgacgt ctaggatcca cgtccgtcgt 15120

cggaaagagg agattaactt gtgatactat tgtctaacta tgcggatgtg gttgaatgca 15180

aggactctct ctctctctct gtctgattga tatttgagtt ttctatggtg atcgagcaag 15240

atttttgcaa tgtggagccc atgcatgctc atgaggccta ttgggccgat ctcttcgaga 15300

tcgtgatcga gagcaaattt gagaacctca gaccttgttt atttgaaagt agcagatgaa 15360

caatagaatt gtttttactt ttggaatggt tccacaataa tcctagtcta gatttaagat 15420

accaatattg aagtgttatg tttgcatgta tcttcagctg ctccacccgc gtggagtgat 15480

tattagctta ttagcgcctt ctcattaata cgccctccag ttccagcctc tcaaaagtaa 15540

tatgctggaa tgatagaggt aattggctaa tggcctcaag gcaaccctgc agatagtgaa 15600

gcaaaagcaa taaatattca atattcacac ataatttgac atacggagta ctccgtactc 15660

cgtttaagat cgggcatagt attggatgat gttagaatat atcttggcaa ggtgacatat 15720

acaatgtact ccgtatgttg tacagtgtca atggctttgt ggagctgaag atgcggtgat 15780

ttcttttcct gatgcatcat caagtccgga aaattgatga aaatctacga gtacctcgag 15840

ggatgaactt ccctgcacag atcatgacat acatataaac tattgatcca cttgcattag 15900

cgggagtcta gcaagagcaa gtctatgtat tccctacatg gtcgaggagg taagttcggg 15960

ctgaaaaata cgatgcagca tacactaccc ttacaactag ctgtttaatc agaaaaagca 16020

aatagaaatt agggcacaat ttactcttta ctgccaaccc cccgtcgtaa cccttgctgc 16080

tagcattgat tggctgtcag tcgtacaacg aagaaacgac actgtctgtg attatattct 16140

attccatcac aaacgtagcc cggagtgccc ttcccagagt ccttgtcttg tacaccgtgc 16200

ttgtcttagc attttcatta tgatcgagct caaagatgct tcgatggggg ctgtattgct 16260

gacatgcgtc cttgtgcttg caggcctata tctcattcga ttgacgttat caagcgacca 16320

attggacaag tttcctagca tcaatcctcg gaagccctgg gaaatcgtca atgtcttcgc 16380

ccaaagaaga tttcaacagg atggccctag gtatctggaa gctgggtatg caaaggtgtg 16440

ttccataagc aactgctcca aaaggcgaat aaggctgaaa gttactacag tcccccatct 16500

ttagcgtggt caccgacctg gggccaaaat tagtggtttc gggtgcattc atcgaggaat 16560

tcaaggatga aaagctgttg gaccattatc ggtcaatgat cgaggtttgt acgacgttag 16620

tgattatgaa agagcaagcg cttacttgtg caaggacttc atggcagagg tacctggttt 16680

tgagtcgatg ttcctgggga atctacacaa tacggtactt cgcgatgtga tttctgtcat 16740

cactcgcgaa ctaggtaaat attctttcct tttgactgtc cggttatccg ctgagttcta 16800

attttataga acaactgcta gcacctctct cggatgaagt atcagcggct ctggtagata 16860

cttggacgga ctcaccaggt gggtcaaagc acacttccca atagaaatca ggaggaaata 16920

aaaactaata tcaatataga ctggcatgag gtagcactgc ttccaagcat gctgggcttg 16980

atcgcaaagg tttcatctct cgtcttcgtg ggtgaaccgt tgtgccgcca cccagtctgg 17040

ttggagacag tgatcaactt caccctcatt cgacacaacg caatcttagc cctccaccag 17100

tgccctgctg tacttcggcc cgtccttcac tgggttcttc caccatgcca gaaactccga 17160

cgagagatca gaactgcacg gacactgatc gactctgctc tggaaaaatc aagaaagaat 17220

ccgcagaccg agaaattttc cagcgttgcc tgggttgatg cttttgccaa aggcaacaag 17280

tataatgcag ccatggtgca gttaagactg gcaaatgcgt ccatccactc cagcgccgat 17340

ctcctggtca agattcttat caatctatgc gagcagccag aattgattcg ggacctccgg 17400

gacgagatta tctctgttct tggggagaat ggatggcgat cctcgacact gaaccaatta 17460

aagctccttg atagtgttct gaaggagagc cagcggttgc atccagtcac aaccggtatg 17520

catcgtcggc tgttcaaact gcgtgcccag tgcatatgct gaccatttac tttaggagca 17580

ttttcgcgct ttactcggca agatatcaag ttgaccaatg gcactgagat tccttcagga 17640

acacccatta tggtcactaa tgatgtcgcc ggggatgcca gtatctatga tgatcccgat 17700

gtcttcgatg ggtatcggta cttcagaatg cgtgaaggag ccgataaggc ccgggcacca 17760

ttcacaacga cgggccaaaa tcaccttggg tttgggtacg ggaagtatgc ttgtcctggt 17820

cgattctttg ctgctaccga gattaagata gcgctctgcc atatgttgtt gaagtatgaa 17880

tggaggctag taaaggacag gccgcatggg atagttacaa gcgggttcgc agcattccgt 17940

gacccacgag caagcataga agtccgcaga cgcgcggtgg cgggagaaga gctcgaggta 18000

ttgactggaa agaagtgatc tagggaaaat tacgaactca tagtatgagc aaccataccc 18060

aaaacaaaga gacttaccaa ccccatcatc aaggtagact ggggattttg actatgtcga 18120

tgtaaatcgg tcaacagcct tattaggata tataaattat acgcttctca ggctttaaag 18180

catcacccag cacgataatt tctctggatt attgcaaaac caagaaattc tctgatccac 18240

agctgtatac tccgtactcc gttcatcatc ttacagtcat gcagagggtg aaaggggtca 18300

gtgtgtgacg gtatttcggt atctcgcctc gtaatttgac agatccagcg ttaaacccag 18360

cccaagactt gagtagacta tatttattct ctttgatatc catctcagca tcaagttttt 18420

gacgttgtat tactatcctc gtttggaatt ctcctcccag gtcttgcttc attgcttata 18480

gcattctacc aaaaacgtca ctgtcatgga cgggtggtca gacatatcat cagcgcctgc 18540

cggatacaag gatgttgttt ggatagcaga tcgggctctg ctagcccaag gattgggatg 18600

gtcaatcaac tacctggcca tgatatacca atcgcgcaaa gaccgcacat acggcatggc 18660

cattttgcca ctatgttgca actttgcgtg ggaattcgtc tacactgtca tctatccttc 18720

tcaaaatccc ttcgagagag ctgtcctcac aacatggatg gtcctgaacc tctacctcat 18780

gtacactacc atcaaattcg ctcccaacga atggcagcac gccccgctcg tccagcgaat 18840

tcttccagtg atattccctg tggcaatcgc ggcatttacg gcggggcatc tcgccttggc 18900

tgcgacagtg ggagtggcca aggcagtcaa ctggagcgcc tttctgtgct ttgagctatt 18960

gactgccggt gccgtgtgcc agctcatgag tcggggatct agcagagggg cgtcgtatac 19020

aatctggtat gttctttttg ccttgtggat cttgcttggg tttattggct aatgtgaatt 19080

gtggttggca gggtctcaag atttctgggc tcgtatatcg gtagtatctt tatgcatgtt 19140

cgagagaccc actggccgca ggagtttgac tggatcagct accctttcgt ggcgtggcat 19200

ggcatcatgt gcttctcgct ggatatttct tatgtgggct tactgtggta cattcgtcgg 19260

caggagcgcc agggccaatt gaagaaagct atgtgatcga caggaccatg catgatggag 19320

gtccgcacta acctcaactg tactttgtac aggtctgagt gctatatgac gatagtcaca 19380

aaacagagtt ggaggttatt tgcgcacatt gactaaaaat gggagagctg atggatatat 19440

gcaaggggga tcaggtctcg atctgatcgt gccgatcgac aagaacaatg ctttgtctgg 19500

gcgggtccaa ttgtctagcc tagaagtcta aatttcaatt ttcttcggac tttttacata 19560

gtaactactg cctaggactc gggatatgaa gtataatggc gagaaatggc tggctgcagg 19620

ggacatacag gtgataattt gccctcgatc tggcagctag ttacgtcaat atcttgttag 19680

taaacaccag ttgtagatct ttgcgtatat atgaaactca aaagcatttg tgtctactcc 19740

gtaattacct tcccaacccc tccagtgcca ttgaaaccat gaaggtcatc attgtcggag 19800

ggtccatcgc gggtctcgcc ctcgcccatt gcttggacaa ggccaacatt gactatgtca 19860

ttctagaaaa gaagaaagaa attgcccccc aggaaggtgc ttccattggt atcatgccta 19920

atggtggtcg gatcctggaa cagcttgggt tatacgacca gatcgaggag ctgatcgagc 19980

ctttggtgag ggcgcatgta acttaccccg acggcttcaa ctatacaagt cgataccctg 20040

cactcataca gcagcggtgc gtcaatataa gctttctact ttctgatttg aaactaatgc 20100

gagaggtctt aggtttggct atccacttgc attcttggat cgacagaagt tactgcaaat 20160

tctggcaact cagccggtcc aatccagccg agtgaaacta gaccacaagg ttgagagcat 20220

tgaggtctcc ccatgtggcg tcacggtgat aacaagcaac ggacacacct atcagggcga 20280

tcttgtcgtc ggggctgatg gagtgcatag tcgggtacga gcggagatgt ggcgactggc 20340

agatgcctcg caggggaacg tatgtggaaa tggagacaaa ggtaacatta ttcctactgt 20400

tttgtcctat cctcgctttt ttttttcttg gccaagtgtt ttgactttga gctggaaagc 20460

taatatattg atttatagca tttacgatca actatgcctg catctttgga atttcgtcac 20520

acgtcgatca attggaccct ggcgagcaaa taacctgtta caatgatggg tggagtatcc 20580

ttagtgtgat cggacagaat ggcaggatct actggttcct ctttatcaag ctggaaaaag 20640

aattcgttta tgatggatca cacaaaaccc agctccactt tagccgtgaa gacgcccgag 20700

ctcattgcga gaggctggcg caggagcctc tctggaaaga tgtgacattt ggtcaggtct 20760

gggctcgatg tgaggtcttt caaatgacac ccttggaaga aggggtgctt ggcaaatggc 20820

actggagaaa cattatctgc atcggagaca gcatgcataa ggtcagcagc tcattatcac 20880

tcctggctta ctgacttttg taattaattg acattctcat gcagttcgca ccgcatattg 20940

gacagggtgc taattgcgct atcgaggatg cagctcagct cagcaatagt ttgcacactt 21000

ggctgagcgg atctggaaag gagcatcaac taaaaaccga tgatttgaca gagattctgg 21060

ctcaatttgc acaaactcgc ctccagaggc taggtccgac ggccatggcc gctcgatctg 21120

ctatgcgtct gcatgcgcgg gaagggctca aaaactggat actgggacgc tacttcttgc 21180

cctacgctgg tgacaagccg gccgactggg cctcccgagg aatcgcaggt gggaatactt 21240

tggacttcgt agagcctccc acgcgggctg gtcctggctg gattcagttc agccagtcgg 21300

gtaaaaggac ttcgtttccc atggcagtgg caggtctgtg cctagtgagc attgtggccc 21360

gaatcatgta tttgaaatta gttgcataga gaggcccacc atatctggag tacttcatac 21420

agagtgtttt atgggacaat ataaacttta gggcaattta gcgctttgat atagatcatc 21480

tgcatactag taaggcaacc ctgaaggtga tcgacacgat ctgcaaaaat caatatcgtg 21540

cttcgttacg gagtattgtt ttctacatgt catagtgcgc gctgccccag tggggctatg 21600

cagaaagtga tttcgatgta ttgctactta cagtgatgtg gtccagcatg tcagccattg 21660

ctctagtgcg tgcgtgtact gaccacatcg cggccattgc catttatcta gggtcctgtc 21720

gcctcaaaag cttgtggtca caaatcgttt gatccttcga gatcatactg aatttttgtt 21780

caatctgtca tcatggctgg ctctcagtct acggcgcagt tggctcgcct tctcattgat 21840

atctcccgat ttgacaaata caactgttta tttgctatat tccctggagg tacggagtag 21900

tgcagaccac ttcaacatta taccaccgcg ctcacaattt catatagtct ggtctatctt 21960

ccttgcagca gcctcacgac acgctgatgg cgaccccgtc cctctggact ttgtattggg 22020

ccgcgcagga ctggccttca tgtacacgta tatgctgagc ggcgcaggaa tggtatggaa 22080

cgactggatc gaccgcgata tcgatgccca ggtggcccgt accaagaatc ggcccctcgc 22140

ctccggtcgg ctttccacca gagctgccct catttggatg cttgtccagt acgcagcctc 22200

ggtctggctg atggaccgca tggtgagcgg gcaggatgtg tacgtctttt ttcctcctcg 22260

taccccaaac aattattctg ttgattgaaa actgacccta atcattctcc agatggacat 22320

acatgcttcc tctcacaacc gggattatct tgtatccctt cggcaagcga ccgacaagtc 22380

gcaagctggg cgtctatccg caatacatcc tcggtgcaag cagcgccctt actatcctcc 22440

cagcctgggc ctccgtctac acaggccgta tatctttgaa ggatctgggt atgcggtgtc 22500

tcccgctttg tctcttcctg tttctgtgga ccatctactt caacaccgcc tacagctatc 22560

aggatattaa ggatgactgt aagctgaatg tgaattcgtc gtacgtcctc gcggggagcc 22620

atgtgcgtgg aatgcttctg ttacaggcta ttgctgtggt gctggtgatc ccctggattc 22680

tctacaccag cgcctccact tggctctggg tctcatggct gggggtatgg acggcatctc 22740

tcggcgagca gctttatctc tttgatgtga aggatccgag tagcggtgga aaggttcatc 22800

ggcggaattt cgcactgggg atttggaatg tgctggcctg ctttgttgag ctgctatatg 22860

cttcaggctc tctgtgaatg atgttaatac gatgtggtcc ggatgagact tggggagtag 22920

agtctgagag gcttaaaatg ggtaaatggt gcgatgttgg cacagtgtga actattcata 22980

aatctttgct acgaagttgg gcttcacctt tcaattgaga agttgttact ggaatttttc 23040

gacactcaaa attcgaagag acttgtatta ttagagggat atagcctatg tcttccaatt 23100

ggtgtagaat cccaactacg agaccgcttc agaacgttgg agcacaagga tagaaagttc 23160

acctattcga aattctctac tgtcgtacat atgctatgta catgttactc ctttgcttgc 23220

gcacctatag cccagcaaaa caagggatcc tttgctaaca ggagctgatc atcacggttc 23280

agagtcagat gcaaatccca cggctccgta ctcgccacat catcctgacc ctttggaagg 23340

ataaagcaca tcccccctaa gacaggcaaa tgtagttgga accctcgagg ttgcgctcca 23400

aggctctccc caaagtccag tccgaagatt tcaaaattcc taaagctgct cagagggata 23460

ggaactcccc gaaatccgat atccgcccag tcaggctgcg agtggagatg ggatagagcg 23520

tcttgaatat attctgcgtc aaccgccaaa agactctggc gtatacgagc tgcaatttgt 23580

gtgagatcct ccagacactc ctggcgcaat tccaccgaag gatctgtgcc atcaactagg 23640

gcctcgtttc tcccagcttg aattggcgta tatgtcaata gcaccatgtt tcccagatag 23700

tcatcaaagg ctggagtttt gaaattccca cgcatatcca ccgcgattga cagttcggta 23760
gatttaccag ccaattgtcc cgcttgccga agtatcatgg ccaaaagggc gctcacgatg 23820
tcgttactgg acaggaaccc tggactcggc ctaccatcag cctggaaaga cgtttgccct 23880
ttgatcaacg tattgcaagc ctccttcaaa tactcgatct taggaccggg gattttcagt 23940
cgccaggtga caagctcggt ggctctcgcc cggacgaagc ccgaccaatt ctttgcaagt 24000
agtgctgccc agtctccgag gccacagtag tgcttgctaa aatccatcct ggaaagaccg 24060
gagctgcttt ctgggacaag acgctcaatc tccgatcgta actgccgatc tggcgacaca 24120
cttgcagaag acatcgccgt cgggtctctg cagcaatcgg ctagaaggcc caagactcgc 24180
gcagcgcctg caccatccat tgcggaatga tgaaacgtca tggcgagaat gatcccatcg 24240
cgcatgacat ttgcttgaaa tcgtaggatc ggccttcgtg gcaacgaaat atccatgtcg 24300
ataggcaatg gcgccagccg acttatgatt tcctgctcct cagtgcccgt taggaggcat 24360
tttgattgga tttccttgaa tgactcggcc tggtagtgcc gtatccggag tatagggaac 24420
tggacaagcg actctgaggc ttctggttcg atttgccagg tgtacttcgt ttggctggac 24480
tctgtccgcc gagtcacgtc ccctgcgagg aaggggtgta ccttcaatag cagctcgatg 24540
ccattctcga gaacaccaat gctcttctca ggttgcgtgg tctggaaaaa cagcagaaag 24600
gtgacgttca ttccgagggg attgtggtcg agagaagata aagggtaagc agagcggtcg 24660
ccagttcttc gggcatcgca cattccatct tcacatagac cgtggagtct cacaggtccc 24720
tctttgacct gatctctttg actgactggg agacatactt cctgggtgct catgatttct 24780
gggtgttatc ctattgagtt gagttgtgtc ttgatctttt tttttatttt ttttggattt 24840
ctgaccttgt ttcgcttata ttggactttg cttttctttg tatattgtat tgcattaccg 24900
tacaacaaag catgggattc tctgtgttct gcatgattgt ggagcgtatt ttcctcgatt 24960
tggtatacaa tcaggtcgat ccctggcgga ttccggatct gatgcatgta tacaggtcat 25020
atatctgctt tcctcggtat ttttgagctg aatatcacta tatatgcttt ggagacgatc 25080
aatcgcaaga gagggttagt gattaaatca gttagtctca tccatagtgg gcattagagc 25140
caataaaaga tggtttccac cttgagatgt gatcgccaca agaagatttt gtaaatagta 25200
tgtattttcc aggccctgat ttctatctgc atatttgtca gcttgatcta cggagtacat 25260
cttactgctt ttagatactg acagcagcaa aactccgcgt tgaaggacga gctttgacac 25320
aaggtcaggc acttctctag tacacaaatc ctaatcatcc gacgacatac tactccgtat 25380
gctgtacata gagatccatg tccaattctt gagtctgccc ctctttgatc cacagtccag 25440
ctcagccagg cgcaatctgc atgcattggc atggaagcta ggagctgaca ttggctggaa 25500
ctacgccatc tggggcacaa tgcaagctag gcaactgacc atgtactggg tcagttttga 25560
ttgagtatgc tatacggaag aaagcgacta gtactccgta ggtttgtgta ctacctgcaa 25620
gtggaaagag atacctagat aggtgacatt agtgtccgaa ccaatgacca atggccctta 25680

tgcacccata tcccttacat ctttcagaaa gagaaaagcc acaagtatat catgtactcc 25740
gtactccgta caacggaatt acttgatctc tatattacct tcttcctgaa gaccgtttct 25800
cgctattgtc agttacacac acaatggatt ccctattgac gagcccgtta tggctcaaaa 25860
ttgcacatga gctagcactt tacctctctt ttattgtgcc aaccgccttt ctcatcataa 25920
caactcaaaa atcatccatt attcgatggg cctggacacc atgtctgctt tatatcctgt 25980
accaattctc tcttcgggta ccctctctgt cgacaagtca attcttgaag ggcgttgcag 26040
cgggtcaagc aaccgtggct gctttgcaat gccttaatct tcttctgatc acgaagctgg 26100
accaaacgga tctgctacgg gcaaatctat acagtccgtc tgcaggactg ctttctcgcc 26160
ttgctcaatc ctgcgcattg ctggtcaact tccgcggaat cggcacaatc tgggaggtta 26220
gaaacattcc ccagcacgca gcgtttgtcc aaccaaaagg caaggatcaa tcaatgagcc 26280
ggaagcggtt tgtcttgcgg gaaattgcaa tcattgtatg gcagtacctg ctccttgatt 26340
tcatttacga gtcaaccaag ggcacgtcag ccgaggattt gatgcgtctc tttggccctg 26400
gtatggaaat caagtatctc gatgcaacgt tcgaacaatg gatggggcgc ctctccgtgg 26460
gaatattctc ttggcttgta ccttcccgag tctgtcttaa tatcacttcc cgcctgtact 26520
ttctcatctt ggtagtattg ggcatttctt cgcccgagtc ttgtcgaccg ggcttcggca 26580
gagtgcggga tgtatgcacc atccgtggag tctgggggta agtgaactat tccgactgct 26640
ttcattcatt cactaacgcc accacagcaa gttctggcat caatcctttc gttggccact 26700
cacctctgtc ggaaactata tcgcaagaga cgtcctcgga cttgctcatc cctctctttt 26760
ggaacgctac accaatatct tctttacctt tttcacatcc ggcgtattgc accttgtctg 26820
tgatgctatt ctcggcgtcc cgccatctgc gtccggcgcc atgcagttct tctgctcgtt 26880
tccgcttgct attatgattg aggatggggt tcaagaaatc tggcggagag cgacggccca 26940
aaccaaggac agtgatcgtg cagtaccgtt ctggcagagg ctcgtgggat atctttgggt 27000
ggctgtctgg atgtgtgtca catctccgtt ctacttgtac ccagctgcgc ggcaacatgc 27060
ggagaagaac tggatagtgc cattcagtat agtggaagaa attggccttg gaactgcgca 27120
aaagattttg ctgggttatg gcttgtttgt gtactgggcg gttggtgggg agatttaaat 27180
tcatgtgtcg ggattgttca tcgtggtcaa cactgtttag attgtgatat atattttcac 27240
cgaacacccc agaaacaaaa gatttaagcc ccaattaact accttgaagg gctcatgaga 27300
tttgatcaat gtagcaaccg tcagtatcct aggtcgtgat tcccccagcc agagcgagat 27360
aattttccag acatcatctt atctacatgc aaccaaaaac tccctggcat atattaacag 27420
agcaaaacta gaggagcaaa aaagaaatct caggtttggt ttttaggaat agccgaacgc 27480
gggggtcgaa cccgcagcct taagattaag agtcttacgc tctaccgatt gagctagccc 27540
ggccgggctg ttgaagagag ttgccatata gcgctacata atcctaaagc ggtcagggcc 27600

tggggggcga acacgctgac ataatgctag cgcgtcgagc ggcgaatcct ctggaaccaa 27660
aattgttagg tggaaggtgg cttcatctac gaatctgggt gtttcctcga ttggatctta 27720
tcattgcttc cctgattcgt atgagtcttt aattttctgg ttgcttgact ctgaccgcgg 27780
tcactagatt gcccaccatg tgcgttacta gaacctttcc ccgattcttt gctgcagcta 27840
acactataca gggcaaagct cgtggacgac catcagatcc atactgcctc gttgcataac 27900
ccgattcctt ggcaattgca tacatacgtc tggcctttcc tgatcatctg gcccgtgttc 27960
tttgcctttt acctctctcc cgagcgctat gatacctaca ttcagggaca ggagtggacc 28020
tttgtgtttg cggggtctat catcacagtc cagtcgctct tctggctgat gaccaagtgg 28080
aacatcgata ttaacaccct attcacaact actcgatcca aatccatcga cactgcccgg 28140
cttatcaaag tggttccgat caccaatgcc ggctctgccg agatctgtaa cctgattaga 28200
gagcacattg gcccgaagaa gaccctttcg ttcctcttcc agaagcgccg cttcctcttt 28260
taccccgaga ctcgctcctt cgcacccctt tcttacgccc tcgacgccga gccgaagccg 28320
gccctcaaga ctttccagca gagcgagggc ttcacgtcga aggccgagat tgagcgcgtc 28380
caaaaccact atggtgacaa taccttcgat attcccgttc ccggtttcat tgagctcttc 28440
caggagcatg ccgtcgcgcc gttcttcgtc ttccagatct tctgtgttgg attgtggatg 28500
ttggatgaat actggtacta ctcgctcttc accctcttca tgctcgtgat gtttgagagt 28560
accgttgtgt ggcagcgcca gaggacattg agcgagttcc gtgggatgag catcaagcct 28620
tacgatgtct gggtataccg tgaacggaaa tggcaggaga tcaccagtga taagcttctt 28680
cccggtgatc tcatgtcggt gaaccgcacc aaggaggaca gcggtgttgc ttgtgatatt 28740
cttctggttg aaggcagtgt cattgtcaac gaggctatgc tttctggcga gagcacccct 28800
cttctgaaag actctatcca gctccgtcct ggcgatgact tgattgagcc agatggattg 28860
gataagctct cgtttgtgca tggaggtacc aaagtcctcc aggttactca ccctaatctg 28920
actggcgacg cgggcttgaa gaacttggcc agcaacgtta ccatgcctcc agacaatggt 28980
gccttgggtg tggttgtgaa gaccggtttc gaaaccagcc agggtagcct cgtccgtact 29040
atgatctact cgactgaacg tgtctctgcc aacaatgttg aagctctgct gttcattctc 29100
ttccttttga ttttcgccat tgccgcttcg tggtacgtgt ggcaagaagg tgtgattcgg 29160
gatcgcaaac gctccaagct tctgctcgac tgcgtcctta ttatcaccag tgttgttcct 29220
cccgaattgc ctatggaact cagcttggcc gtcaacacta gtcttgctgc tctgagcaag 29280
tatgccattt tctgcactga gccattccgt atcccctttg ctggtcgtgt tgatatcgct 29340
tgcttcgata agactggtac cctgaccgga gaggatcttg tcgttgatgg tattgctgga 29400
ctcactttgg gtgaggctgg ttcaaaggtc gaagctgatg gtgctcacac cgagttggcc 29460
aattcttctg ctgctggacc cgacaccact ctcgttctcg ccagtgctca tgccttggtg 29520
aaattggatg agggtgaagt cgtcggtgac cccatggaga aggctacttt ggaatggctt 29580

ggctggactc tgggcaagaa cgacactttg tcttccaagg gcaacgctcc cgttgtttct 29640

ggtcgcagcg ttgagtctgt tcaaatcaag agaagattcc agttctcctc ggccctgaag 29700

cgtcagagca ctatcgcgac cattacgacc aatgaccgca atgcttccaa gaagaccaag 29760

tctacttttg tgggtgtcaa gggtgccccc gagaccatca acactatgct ggtcaacaca 29820

cctcccaact acgaggagac ctacaagcac ttcacccgta acggtgctcg tgtgcttgct 29880

cttgcttaca agtacctttc ttcggagacc gagctttccc agagccgtgt gaacaattat 29940

gtccgcgaag agatcgaatc cgaactgatt tttgccggtt tccttgtcct gcagtgcccg 30000

ctgaaggacg atgccatcaa gtctgtccaa atgttaaatg aaagcagtca ccgtgttgtc 30060

atgatcaccg gtgataaccc attgactgct gtccacgtcg cacgcaaggt tgaaattgtt 30120

gaccgtgagg ttctcattct tgatgccccc gaacatgaca actctggaac caagattgtc 30180

tggcgtacca ttgacgataa gctcaacctt gaagtcgacc ccactaagcc tcttgatcct 30240

gaaatcttga agactaagga tatttgtatc actggatatg ccttggcaaa gttcaagggc 30300

cagaaggctc tccctgatct gctccgtcac acctgggttt acgctcgtgt ctctcccaag 30360

cagaaggaag agattctcct tggtcttaaa gatgctggat acaccactct gatgtgcggt 30420

gatggaacca acgatgttgg tgctctgaag caggcccacg tcggtgtcgc gcttctgaac 30480

ggctcgcaag aggatctcac caagatcgct gaacactacc ggaacactaa gatgaaggag 30540

ctgtacgaga agcaggtcag catgatgcaa agatttaacc agcccgcccc tccagtacct 30600

gttctgatcg ctcacctgta tccccccggc cctaccaacc cacactacga gaaagcgatg 30660

gagagagagt cgcagcgcaa gggtgctgcg atcaccgctc ccggcagcac tcccgaagct 30720

attccgacta tcacatcccc tggcgcacag gccctgcagc aatcgaactt gaacccccag 30780

cagcagaaaa agcagcaggc ccaggcagct gcagctggcc ttgcagacaa gctcacatcg 30840

tctatgatgg aacaggagct ggatgacagc gagcccccca ctatcaagct gggtgatgca 30900

tccgtcgctg ctcccttcac tagcaagttg gccaacgtta ttgctatccc gaatattatc 30960

cgtcaaggtc gttgcaccct ggtcgcgact attcagatgt ataaaatcct cgctttgaac 31020

tgcttgatca gtgcctacag tcttagtgtc atctacctgg atggtatcaa gtttggtgat 31080

ggacaggtca ctatcagcgg tatgctgatg agtgtctgct tcctttcaat ttcccgcgcc 31140

aaggtatgtc gtatttccca tgtcgaccaa atgatttgct aatatgttac tgtgtgaagt 31200

ctgtcgaggg tctgtccaag gaacgcccgc aacccaatat tttcaacgtc tacatcattg 31260

gatctgttct tggacagttt gccatccaca ttgcgactct gatctacctt tccaactatg 31320

tctataagca cgagccgtac gtgatgaaaa cttccccttt catttgtcct acttcatagc 31380

taacataatc aacaggagag attctgatat tgatctcgag ggcgagtttg agccttccct 31440

tctgaacagt gccatctacc tcctccagct gattcagcaa atctccacct tctcgattaa 31500

ctaccaaggc cgtcccttcc gtgagtcaat ccgcgagaac aagggcatgt actggggcct 31560
cattgccgcg tccggtgtcg cattctcctg cgccactgaa ttcattcccg agctgaatga 31620
gaagttgcgc ctcgtcccct tcaccaacga atttaaggtg acattgactg tgctgatgat 31680
cttcgactac ggtggctgtt ggttgattga gaacgtcctc aagcacctgt tcagtgactt 31740
ccgtcccaag gacattgcca ttcgtcgccc tgaccagctc aagcgggagg cggaacggaa 31800
gttgcaagag caagtcgacg ctgaggccca gaaggagctg caaaggaagg tctagaggtt 31860
ggtggtttga agatttgtat ctgtaaacat agagaggagg ttgttgaatt ttagaaatgt 31920
tcaagtggtg tgtgacattt aatacattta tttttggctt ttattgaagc attcttggaa 31980
actatatgta gaaacaaatt cgtatagttg aatggctcct actctgtact gtccaatcgt 32040
cgtgaggcca ggtattgcct tggtagagaa cagtgtagac tcaaatgtgg cgatcgtccg 32100
atcagcttgt tacgaggtta gggctcgaaa tgatcggccc accataactt cttgtagctc 32160
cttgtttgag aggatgcagt ctacccgtta tgtagaccta attatccagg atggtcgaga 32220
atacttctca atacacaggg ttagacccca gatatatgat atgtcacctc agagaggggc 32280
aaagactggg taattccaaa aaatgtgatt ttgcagaggg tcaaagctat atcggatact 32340
gcttcttttc tctgcctcat agtgaaggaa acactatatt cttcatggta ggcaaagagg 32400
taaaagtgta cgtgccccaa ttcggtagaa ggataggccc tgtttgaaaa ttccacattt 32460
tgaccgatat atctatagaa acatatgaag tagccgcttg gcccttctcc atttgaagct 32520
tcgagctgac gtggacttca aatgcaggat gctttgttcc tttgtactgc catgcaatat 32580
aatgttgcct tgaactcgag attataatgc gaaaacctcg tagagccgat cgcagcccga 32640
gccaacattt ttctataata cataggtaaa cgatctgtga attcagaaag ctcccacatt 32700
gtattataag catgaatcat tcaacgcgag acttcaagct tcatgaaatc cttcaggaac 32760
ccaacagttg aaagaccacc aattccctag atcccactga tttcgattac gacattccgg 32820
attgtagtag ggcatatggc gatgccgggt ttgattgcaa agaatatatt cccatacatt 32880
gcagtaccca cctttgacaa tccaggattc aggtgcgtcg acgccgccgt gattacaact 32940
agtgctggag gcgccgttgt catagaacca tttgcaggag ttaccgtcaa ttgattcggt 33000
gggtagactt cggtctggtc gcaggcattt gaggtccttc gtccagccca cattatgtcc 33060
tttcatttgg atgtagttgc ccttgcagtt cttcttggtg tacatttcca ctgtccatgg 33120
atggacatat ggcgcatcgc tggttgtggt gcttggtttc gtgttggatg ttttggttga 33180
cttgaccgat gtagttttgc tagtggtggt ggccttggtg gttgtggtgc ttgacgcagc 33240
actggccgat atcgttttat tggatgggga caaggcaagt ctctgaatag tcataaattt 33300
tgcaaaagca ttaaccgcat tagtaggcat ggcagtcttt tttgaggtgg tcgctccaat 33360
ggtgcggttg gtagaagtac agaattccga agtgcttcca caataaccat acgaagagca 33420
ctatgaattt attagatgtt gaagagcagg atttagagcc tcttgactca cacattcatt 33480

agctgcacat ggattcaaag aacccagatc agaccagttg ctagggcgct ttgtcccagg 33540
cacccggggg ccacaaacag cattggaaag cgtagctggc atcatgggtt ctccagaact 33600
gaggcatata acagcaccct gcactaaatg gtcacatcca agccattccc aggtttgagc 33660
attgtaggtc tcgatatcag ccaaggcgag agagtgttct tgagcaatcg tggtgcaggt 33720
ttcgcccgcc tggacaacat atttgtgaca aagaccatca ctacctgctt gaggtgcacc 33780
tgatattgct gtcggactgg ccaaaaggac gcctatgaga gtcacgaagt tgcttggtcg 33840
gaagcaccac atcatattga ctgatggaga gtagttgctt tgcttgtctt tattttgcaa 33900
ggcaggtttc atctttatct gttcaaagag gaaaacatgt gccaaactgc caaggataga 33960
tgcacgcatg aatatgacat tgccggggag gggcaaatgt ttgtgaaaga actaggatac 34020
tgtgccaggg ccattagcat agtattgaag caaattatag aatggcactg catcaaaatg 34080
tggaatcctc gaattttttc tttgtcttct aacgcctagt gcatgtcttt ccaggttgtc 34140
cttgaaggct ttgtctggtc tcccagaaat ggaaggactc aagggtatgt atacagcttc 34200
taaaacgtaa atgattcacc cgagaaagga attcataatc cgaggaaggt cagacacata 34260
aggctgtctc gaaacccctt gaatgcccaa ggaagaaagg aaattcctac ggctgggtca 34320
gactagcaag aaaacgtcac ttgacttctg agatccactc agatagcaga agaacgtgtt 34380
tggtgatttt cgttctttgt aaatgcatag gaccagatga ttcgaggaat cttcttgtta 34440
gcacccttaa tccaaatctt ctgtagacca agcactcggc tattgatact gtttcgagag 34500
tctgtaagat atgacattac tctgatacag atacgtggaa tggaaacatt gcgggctttc 34560
gaatgacatt gggttgacta acgaaggccc cttcacgcag tgacgaggcc ccaaagttca 34620
aggccaacgc gcaaagcggg accaacatcg aactccccat tctcggggag ctgagggccc 34680
gccttgattt tgacatcttc ccatttgtca aagtcattat tgaacgcctg cgtcatttcc 34740
gaagcattca gaatgcgggc caaccggaag tgacaatcta ggagatcagc acttggcaga 34800
cttcgtacat tgtctgcgtc cttgttgctg aaaaccacct ctatcggaaa aaagaccttg 34860
taatttgaag aaattccttc gaacgtgtga actttgtatt tattgtccac atcctttgaa 34920
atttcggtta gcaaggtaat aaagtaaaat cattccacaa atgggagcgc actcactgtg 34980
ggttcaaagg caagacggaa cgcgccgaag tgttcatgta cccagctagt aagggtgaga 35040
ccgttggata agctgttgat ctcttggtgc ctgaaattca tttcccggat cctgggaaaa 35100
cagcgccaga ggacctccca agctcgtgac gcatttggta tgagatcgtt cttgagcccg 35160
tgagtcattg atcttaatga ggatccgaga acttactcgc cggggatccc agactgcata 35220
tgacaaggga attatgtgtg cgctttctgt attcccgagt ttttcaatat cctctgattt 35280
gcctagactg tcccaccgat cgagatccat gtcaccagtg acaacacagc agtaaccgtc 35340
gcgttttagt aaaccttcct tgaactcccc tgtccgggac ataggctggt ccaaagtgga 35400

atttttcttc gcatgggaat cctgccgttc ggttgacgag atagttactg atgggggctt 35460
agaccgagaa cgcactattt cgagtgaacg tataagtcag ggctttgtac ttttttgtta 35520
acttactacg ggttcttaga ccagtgtaca ggttgcgaaa gtattgataa attccatcat 35580
cgtcttccag gctgatgata tccttcgcaa tggagctttt gccgcgatct gggaggaaat 35640
tcaaaaaagc ttgtaatgat ttttgcagga tgtcttcgcg ttctccacgg ctcttgggtt 35700
tgtaactttc tattcttaag cgtgcggttt caatatcctg cttttgttcg gtttccgatg 35760
cttcagtaga ttgtgaaaac agcgattgtg cgcggggata ggcacgtgct ggaccagaaa 35820
cttcgctctc ttgggtttgc tgtttacgtt tttgagaccg ggtggtgggt cgatctgatt 35880
caggcaattc agaacggcgc cttttaagat tagttttgga tggtggcgac gaggaagttg 35940
gagggatgtt ttggttcggg gcatcggctt gtagtgaagg gggcggtcgc tttgctggtt 36000
tctttttctt ccccatgatg tctgctagta gtagtatatt tcttgctttc cttttccaat 36060
actgagatgg tagtttcagt ggatgaaaat gagaacaatg ggataattca gtggatggaa 36120
atgagaacaa tgtgatgatg ggggagaaaa gtgatgtggg ggtgtcgggg gatagctccg 36180
agatattcct ccggcagaat cgctccaccg aaaaacagtc cgccggacgg gtcatccccc 36240
ttttggagaa aatgtatttg tagttacaga aaggcattag cccacagaac aagaattcat 36300
ccatatttca ttgttttcca tcaagcaatt actcgtccaa tcgtctctcg gagggtgcag 36360
agaataggct ctctctggaa ggccgctgga aaaagtggga aaaggataca ttctgtggcc 36420
acaggcgtgg gacagggttt ccccctgaca ctgggggaga aatgtggaaa tgtgggggaa 36480
ctctgcggag acggaagaac aaaaggcggt caactgctgc ctccacgtga tgtcacgtgg 36540
agcttagccg tccagcttgg aagataaccc tagaggaata tgagcatatt ctacggagaa 36600
ctactccgta caacatacgg agtactcata caactctgta gcaacccctg atgtgatctg 36660
tatttgaagt gtggacctga taccgactgc tcctcaaacc ccttaaaccc gtatcgagta 36720
ctccgtaata tgtacaccgt tcactgactc acattgatta atcacattag atctctcgtt 36780
ttcatgtacg tggatcatta tgagttcgag cattgaatat aagctaaaac cataccccct 36840
gaccctaagg ggccttctgg aaagaaaaat cttgtctttt gcaaatcaaa atatatatag 36900
agttgtttac ccgaactgtc gggttatgca tcttcaggcc tgtggagctg tgtcatcatt 36960
ttgttactcc cccttatcta ccgcaggatc gccaaaatgc ctagcgagac tgctacaggt 37020
gactttggtc cagcgccgcc tgggatagac ttgacagaga accaaactgg cgacttgcta 37080
ggagcagtga ttcctgtagc ggtggtcgcg acgactgcgg tgatattgcg gacgattgcg 37140
ccgacgagga tcaaagagat ccgacaaaca gctattgatg actatctcat tgttgcggcg 37200
cttttattct cttggggaac ggcaatatca tgcttcatca gtgagttgac catgaggcca 37260
aagccgatgg gcccagtact cacaacagac tctttaggca ttccatatgg caacggttat 37320
catttgcaat ctgtgacaaa agcagagttt aacactgttt ggaaagtaag gaatccaata 37380

ttaaatgaga tgcctgggat agacgttgac cagacattca gatccttttc gcctatgtca 37440

tgatttacgc tacagccgtt acctgcacca aagcctcgat cgtcttattt tacggccgca 37500

tcttccactt tcgctggtca ctggccatct gcctgtttct ggtcgttgga tattgggttg 37560

ccattattgt cacggttggg atggcctgtc gaccactgcc acatttctgg ttggtctaca 37620

cagatccatc agcccttggt gtctgcattg atattcccac gttctttttc gcaaatggca 37680

ttgctgccat ggcgattgat gtgatcatac tgtgcatgcc gatgccagca atataccagt 37740

ctcagatgca gttgtcgcaa aaggtagcgg tcgtgggtat cctactcttg ggaagtttgt 37800

atgtacctct gcccgggccc tcctacgaga aggactgtag ctaattattc tcagtgtttg 37860

cgtggcaagt atctgccgga tcatcgcact tcagaatatc accgacggga cagatacgac 37920

gtgggctatc gccccagtct ttatttggtc gtccgtggaa ccatttgttg ggattatttg 37980

cgcatgcctc ccaacatttg ggcctttctt tcggcaatgg cggtccatcg ctcggacgcg 38040

ctcatcaact gatggcagta ccgatccaag ctctgagcta ccatctgaga caacgacctg 38100

gctccgaaga tcccgaacca aaaaacctgc caaggactca atattcagta tcaatgattt 38160

ttgctgtgtc gatgaggtcc aactaatgaa cgatatcaat gccactcggt cgctggggga 38220

cgaggctgcg agtgaccatc aggacgtgga gggaggctgt atcacagtcc aaaaagatgt 38280

ggaagtgaca tgggccaagt acaagtcagg aaaaaaaaat gatctggcct tcaagtatca 38340

taaaggggct tgatcagctt tgcaaatatt tcgacttgac acggactata tttgcgtttt 38400

gtgtatattt aataaaaata gacgccactg gcaatttgta attgataaag gtaagtctta 38460

ttccgtaatc cataccccgt actctataca aagtactctg tgctccgtac ggagtacacg 38520

gaaacaaacg gggatatagt cgtggcacct ttcccgtgtt ggcggacttg cccgtaacgt 38580

aaacactccg cagatccctt ccaacacagt acataatcct gcagcgaaga gcgatctgat 38640

agacgctatg tgccgtcgtg acttgttatg ccaattaacg gtggcagaat tgtggagcaa 38700

tctagcagag gaaagtttcg atgtgcatgc cgagccctaa aaagtcccag tgcggagaat 38760

gtagtaatcg actggacatt ccatgtactt tgcacgctat aacatatttc tatgccatat 38820

acccctctgg taatcatgta gatcctcttg cttactgcgt tggctccttt gtatcgtact 38880

ttccgcgtcg cagcattata agaggataga gagaccgcat gagagaatac acaagagaaa 38940

tcactaattc actacctgat cccccaattc actcaacatg tctcacattc acacttccag 39000

attgcaaa 39008

<210> 267
<211> 556
<212> PRT
<213> Penicillium coprobium PF1169

<400> 267

Met Glu His Glu Thr Asp Leu Val Ser Phe Ala Phe Ser Gly Pro Ala
1               5                   10                  15

Phe Asp Gln Ser Lys Pro Ile Tyr Ile Asp Ala Arg Asn Pro Ser Arg
            20                  25                  30

Ala Phe Asn Ala Ile Gln Phe Arg Arg Leu Val Arg Ser Leu Ile Ala
        35                  40                  45

Gly Leu Lys Ala Arg Gly Val Glu Arg Gly Asp Cys Val Leu Val Gln
    50                  55                  60

Leu Glu Asn Ser Val Leu His Ser Ala Leu Phe Phe Ala Ile Val Gly
65                  70                  75                  80

Ala Gly Gly Val Tyr Met Gly Phe Asp Val Ala Ser Arg Pro His Glu
                85                  90                  95

Val Ala His Leu Leu Arg Val Ala Glu Pro Arg Leu Ile Ile Thr Ala
            100                 105                 110

Pro Ser Ala Leu Thr Arg Val Leu Glu Val Cys Asn Asn Gln Gly Met
        115                 120                 125

Ser Ser Asn Gln Val Leu Leu Met Asp Glu Lys Ser Ile Glu Ser Val
    130                 135                 140

Val Gln Phe Ala His Gly Gln Ala Glu Gln Thr Glu Asp Leu Asp Thr
145                 150                 155                 160

Gln Thr Val Asp Gln Pro Ile Arg Leu Glu Ser Leu Leu Gln Tyr Gly
                165                 170                 175

Glu Leu Asp Trp Leu Arg Phe Glu Asp Ser Glu Glu Ser Lys Ile Thr
            180                 185                 190

Pro Ala Ala Met Phe Leu Thr Ser Gly Thr Ser Gly Leu Pro Lys Ala
        195                 200                 205

Ala Ile Arg Thr His His Thr Ile Ile Ser His His Leu Ser Val Tyr
    210                 215                 220

Tyr Glu Val Pro Tyr Pro Val Val Arg Leu Met Ala Leu Pro Leu Tyr
225                 230                 235                 240

His Ser Phe Gly Asp Phe Trp Gly Asn Ile Phe Pro Ile Arg Tyr Gly
                245                 250                 255

Gln Pro Leu Tyr Ile Ile Pro Arg Phe Glu Ile Thr Ala Leu Leu Asp

260                 265                 270

Gly Ile Arg Gln His His Ile Thr Glu Thr Tyr Met Val Pro Ala Met
        275                 280                 285

Ile His Ile Leu Asn Arg Ser Ser Leu Asn Val Ala Glu Ser Leu Ser
    290                 295                 300

Ser Leu Arg Tyr Ile Gly Ile Ser Gly Ala Pro Ile Asp Gly Tyr Ser
305                 310                 315                 320

Met Gln Gln Phe Gln Ser Leu Leu Ser Pro Asp Ala Ile Ala Gly Asn
                325                 330                 335

Leu Trp Gly Met Ser Glu Val Gly Val Val Phe Gln Asn Arg Tyr Gly
            340                 345                 350

Ile Gln Pro Gln Phe Gly Ser Val Gly Thr Leu Leu Pro Arg Tyr Glu
        355                 360                 365

Leu Arg Phe Val Asn Pro Asp Thr Gly Glu Asp Val Ala Gly Thr Pro
    370                 375                 380

Asp Ser Pro Gly Glu Leu Tyr Val Arg Gly Pro Gly Leu Leu Leu Ala
385                 390                 395                 400

Tyr Lys Gly Arg Thr Asp Ala Lys Asp Glu Gln Gly Trp Phe Arg Thr
                405                 410                 415

Gly Asp Met Phe His Val Glu Asp Gly Asn Tyr His Val Ile Gly Arg
            420                 425                 430

Thr Lys Asp Leu Ile Lys Val Arg Gly Gln Val Thr Gln Tyr Ser Val
        435                 440                 445

Ala Pro Ala Glu Ile Glu Gly Ile Leu Arg Lys Asp Pro Ser Ile Lys
    450                 455                 460

Asp Ala Ala Val Ile Gly Val Met Leu Pro Asp Gly Ser Ser Glu Val
465                 470                 475                 480

Pro Arg Ala Tyr Val Val Arg Asn Asp Thr Ser Pro Glu Thr Thr Ala
                485                 490                 495

Asp Gln Val Ala Gly Leu Ile Gln Ser Gln Leu Ala Ser Tyr Lys Ala
            500                 505                 510

Leu Asp Gly Gly Val Val Phe Val Asp Asp Ile Pro Arg Ile Gly Ile
        515                 520                 525

Gly Lys His His Arg Ala Lys Leu Ser Gln Leu Asp His Gln Arg Glu
    530                 535                 540

Thr Ile Ala Ser Ile Leu Ala Glu Pro Val Ala Val
545                 550                 555

<210>  268
<211>  2447
<212>  PRT
<213>  Penicillium coprobium PF1169

<400>  268

Met Lys Ala Thr Glu Pro Val Ala Ile Ile Gly Thr Gly Cys Arg Phe
1               5                   10                  15

Pro Gly Gly Ala Ser Ser Pro Ser Lys Leu Trp Glu Leu Leu Gln Ser
            20                  25                  30

Pro Arg Asp Ile Ala Arg Lys Val Pro Ala Asp Arg Phe Asn Ile Asp
        35                  40                  45

Ala Phe Tyr His Pro Asp Gly Asp His His Gly Thr Thr Asn Val Lys
    50                  55                  60

Glu Ser Tyr Phe Leu Asp Glu Asp Ile Lys Ala Phe Asp Ala Ala Phe
65                  70                  75                  80

Phe Asn Ile Ser Pro Thr Glu Ala Val Ala Met Asp Pro Gln Gln Arg
                85                  90                  95

Leu Leu Leu Glu Thr Val Tyr Glu Ser Leu Asp Ala Ala Gly Leu Arg
            100                 105                 110

Met Asp Ala Leu Gln Arg Ser Lys Thr Gly Val Phe Cys Gly Thr Leu
        115                 120                 125

Arg Asn Asp Tyr Asn Gln Ile Gln Ala Met Asp Pro Gln Ala Phe Pro
    130                 135                 140

Ala Tyr Val Val Thr Gly Asn Ser Pro Ser Ile Met Ala Asn Arg Ile
145                 150                 155                 160

Ser Tyr Tyr Phe Asp Trp Gln Gly Pro Ser Met Ala Val Asp Thr Gly
                165                 170                 175

Cys Ser Ser Ser Leu Leu Ala Val His Leu Gly Val Glu Ala Leu Gln
            180                 185                 190

Asn Asp Asp Cys Ser Met Ala Val Ala Val Gly Ser Asn Leu Ile Leu
        195                 200                 205

Ser Pro Asn Ala Tyr Ile Ala Asp Ser Lys Thr Arg Met Leu Ser Pro
    210                 215                 220

Thr Gly Arg Ser Arg Met Trp Asp Ser Lys Ala Asp Gly Tyr Gly Arg
225                 230                 235                 240

Gly Glu Gly Val Ala Ser Val Val Leu Lys Arg Leu Gln Asp Ala Ile
                245                 250                 255

Asn Asp Gly Asp Pro Ile Glu Cys Val Ile Arg Ala Ser Gly Ala Asn
            260                 265                 270

Ser Asp Gly Arg Thr Met Gly Ile Thr Met Pro Asn Pro Lys Ala Gln
        275                 280                 285

Gln Ser Leu Ile Leu Ala Thr Tyr Ala Arg Ala Gly Leu Ser Pro Gln
    290                 295                 300

Asn Asn Pro Glu Asp Arg Cys Gln Tyr Phe Glu Ala His Gly Thr Gly
305                 310                 315                 320

Thr Gln Ala Gly Asp Pro Gln Glu Ala Ala Ala Ile Asn Ser Ser Phe
                325                 330                 335

Phe Gly Pro Glu Ser Val Pro Asp Ser Thr Asp Arg Leu Tyr Val Gly
            340                 345                 350

Ser Ile Lys Thr Ile Ile Gly His Thr Glu Ala Thr Ala Gly Leu Ala
        355                 360                 365

Gly Leu Ile Lys Ala Ser Leu Ser Leu Gln His Gly Met Ile Ala Pro
    370                 375                 380

Asn Leu Leu Met Gln His Leu Asn Pro Lys Ile Lys Pro Phe Ala Ala
385                 390                 395                 400

Lys Leu Ser Val Pro Thr Glu Cys Ile Pro Trp Pro Ala Val Pro Asp
                405                 410                 415

Gly Cys Pro Arg Arg Ala Ser Val Asn Ser Phe Gly Phe Gly Gly Ala
            420                 425                 430

Asn Val His Val Val Leu Glu Ser Tyr Thr Arg Ser Glu Leu Ser Pro
        435                 440                 445

Ser Asn Asn Ile Pro Ser Ser Leu Pro Phe Val Phe Ser Ala Ala Ser

450                 455                 460

Glu Arg Thr Leu Thr Cys Val Met Glu Ser Tyr Ala Thr Phe Leu Gln
465                 470                 475                 480

Glu His Ala Thr Val Ser Leu Val Gly Leu Ala Leu Ser Leu Trp Asp
                485                 490                 495

Arg Arg Ser Thr His Arg His Arg Leu Thr Leu Met Ala His Ser Ile
            500                 505                 510

Gln Glu Leu Lys Asp Gln Ile Asn Thr Glu Ile Ser Arg Arg Val Thr
        515                 520                 525

Gly Lys Pro Ala Ser Val Val Ser Arg Ser Asn Thr Arg Pro Arg Arg
    530                 535                 540

Val Met Gly Ile Phe Thr Gly Gln Gly Val Gln Trp Pro Gln Met Gly
545                 550                 555                 560

Leu Asp Leu Ile Glu Ala Ser Pro Ser Ile Arg Lys Trp Ile Met Asn
                565                 570                 575

Leu Glu Glu Ala Leu Asp Glu Leu Pro Leu Asp Leu Arg Pro Gln Phe
            580                 585                 590

Ser Leu Leu Asp Glu Leu Ser Gln Pro Ala Ser Ser Ser Arg Val Asn
        595                 600                 605

Glu Gly Leu Leu Ser Leu Pro Leu Arg Thr Ala Leu Gln Ile Met Gln
    610                 615                 620

Val Asn Met Leu Arg Ala Val Gly Ile Glu Leu Thr Ile Val Val Gly
625                 630                 635                 640

His Ser Ser Gly Glu Ile Val Ala Ala Tyr Ala Ala Gly Val Leu Thr
                645                 650                 655

Ala Ser Asp Ala Ile Arg Ile Ala Tyr Leu Arg Gly Met Thr Ile Asp
            660                 665                 670

Lys Ser Arg Asp Pro Thr Gly Arg Met Met Ala Val Asn Leu Thr Trp
        675                 680                 685

Gln Gln Ala Gln Asn Ile Cys Ala Leu Glu Ala Tyr Ser Gly Arg Ile
    690                 695                 700

Ser Val Ala Ala Ala Asn Ser Pro Ser Ser Val Thr Leu Ser Gly Asp
705                 710                 715                 720

Ala Glu Cys Leu Arg Glu Leu Glu Trp Leu Leu Lys Ser Leu Gly Leu
                725                 730                 735

Thr Pro Arg Met Leu Arg Val Asp Thr Ala Tyr His Ser Pro His Met
            740                 745                 750

Lys Pro Cys Ala Asp Pro Tyr Arg Asp Ala Met Lys Ala Tyr Pro Val
        755                 760                 765

Ala Leu Ser Ala Ser Ala Ser Arg Trp Tyr Ser Ser Val Tyr Pro Gly
    770                 775                 780

Glu Val Met Thr Gly Tyr Asp Gln Gln Glu Leu Thr Gly Glu Tyr Trp
785                 790                 795                 800

Val Glu Asn Met Leu Arg Pro Val Gln Phe Ser Gln Ala Leu Glu Ala
                805                 810                 815

Ala Ala Arg Asp Ala Gly Pro Pro Asp Leu Ile Ile Glu Ile Gly Pro
            820                 825                 830

His Pro Thr Leu Arg Gly Pro Val Leu Gln Thr Leu Ser Lys Met His
        835                 840                 845

Ser Ala His Ser Ala Ile Pro Tyr Leu Ala Leu Ala Glu Arg Gly Lys
    850                 855                 860

Pro Gly Leu Asp Thr Trp Ala Thr Ala Leu Gly Ser Ser Trp Ala His
865                 870                 875                 880

Leu Gly Pro Asn Val Val Arg Leu Thr Asp Tyr Val Ser Leu Phe Asp
                885                 890                 895

Pro Asn His Trp Pro Val Leu Val Glu Ser Leu Pro Phe Tyr Pro Phe
            900                 905                 910

Asp His Thr Gln Thr Tyr Trp Thr Gln Ser Arg Met Ser Ser Asn His
        915                 920                 925

Asn His Arg Ala Thr Ser Pro Asn Ala Leu Leu Gly Ser Leu Ser Pro
    930                 935                 940

Glu Thr Gly Ala Glu Lys Phe Arg Trp Arg Asn Tyr Leu Arg Pro Glu
945                 950                 955                 960

Glu Leu Pro Trp Leu Ala Asp Arg Arg Ala Asp Ser Gly Ser Val Phe
                965                 970                 975

Pro Glu Thr Gly Tyr Ile Ser Met Ala Leu Glu Ala Gly Met Ile Met
            980                 985                 990

Ala Gln Thr Gln Gly Leu Arg Leu  Leu Asn Val Lys Asp  Leu Thr Ile
        995                 1000                 1005

His Thr  Gln Leu Pro Ile Gln  Asn Asp Pro Ile Gly  Thr Glu Val
    1010                 1015                 1020

Leu Val  Thr Val Gly Ser Ile  His Ser His Asp Gly  Ala Ile Thr
    1025                 1030                 1035

Ala Trp  Phe Cys Cys Glu Ala  Val Val Ser Gly Glu  Leu Val Gln
    1040                 1045                 1050

Cys Ala  Thr Ala Lys Met Ile  Met His Pro Gly Asp  Ser Asp Arg
    1055                 1060                 1065

Ala Leu  Leu Pro Pro Gln Gly  Gln Leu Pro Gln Ala  Leu Glu Pro
    1070                 1075                 1080

Val Asp  Ser Thr Glu Phe Tyr  Asp Ser Leu Arg Arg  Ala Asp Tyr
    1085                 1090                 1095

His Cys  Thr Gly Pro Phe Ser  Thr Leu Thr Gly Leu  Arg Lys Arg
    1100                 1105                 1110

Arg Asp  Leu Ala Thr Gly Ser  Val Pro Val Pro Ser  Asn Asp Ser
    1115                 1120                 1125

Asp Glu  Pro Met Ala Leu His  Pro Ala Ile Leu Asp  Leu Gly Val
    1130                 1135                 1140

Gln Thr  Met Ile Ala Ala Ile  Gly Gly Leu Glu Glu  Thr Leu Leu
    1145                 1150                 1155

Thr Gly  Pro Phe Leu Ser Arg  Asn Val Asp Ser Thr  Trp Ile Asn
    1160                 1165                 1170

Pro Val  Leu Cys Ala Ser Asp  Trp Gln Gly Lys Glu  Leu Thr Val
    1175                 1180                 1185

Ala Ser  Tyr Leu Thr Cys Val  Asn Gly Asp Arg Ile  Arg Gly Asp
    1190                 1195                 1200

Ile Asp  Ile Phe Thr Met Asn  Gly Glu Lys Ala Val  Gln Leu Glu
    1205                 1210                 1215

Gly Val Ser Leu Ile Cys Gln Pro Ser Gly Thr Ala Pro Asn Asn
    1220                1225                1230

Leu Gln Val Leu Ser Gln Thr Ala Trp Gly Pro Leu Glu Pro Thr
    1235                1240                1245

Leu Lys Lys Gly Ser Arg Lys Leu Pro Ala Thr Met Leu Gln Leu
    1250                1255                1260

His Ser Leu Arg Glu Glu Leu Ala Leu Leu Tyr Leu Lys Gln Ala
    1265                1270                1275

Arg Asn Gly Leu Thr Asp Leu Glu Arg Ser Gly Leu Asp Phe Asp
    1280                1285                1290

Gly Ala Arg Leu Leu Ala Trp Met Asn Gln Cys Ile Ala Asn Ala
    1295                1300                1305

Ser Gln Glu Pro Asp Pro Val Gly Glu Ser Glu Cys Leu Asp Gln
    1310                1315                1320

Lys Ile Glu Asp Phe Thr Ala Gly Val Ser Pro Ser Leu Leu Asn
    1325                1330                1335

Asp Pro Gly Leu Thr Ala Ile Ala Ala Val Gly Gln Arg Leu Pro
    1340                1345                1350

Arg Val Leu Arg Asp Ser Gly Leu Gln Ile Glu Ala Trp Pro Ala
    1355                1360                1365

Ile Asp Glu Glu Ser Gln Tyr Leu Lys Glu Asp Leu Gln Val Leu
    1370                1375                1380

Asp Leu Glu Asp Glu Leu Val Ser Val Val Ser Gln Ala Cys Phe
    1385                1390                1395

Arg Phe Pro Gln Met Asn Ile Leu Gln Ile Gly Gln Phe Gly Gly
    1400                1405                1410

His Val His Ser Gly Leu Lys Lys Met Gly Arg Thr Tyr Arg Ser
    1415                1420                1425

Phe Thr Tyr Ala Gly Leu Ser Val Ser Gly Leu Gln Ala Ile Glu
    1430                1435                1440

Glu Asp Leu Glu Gln Pro Gly Glu Val Ser His Lys Thr Leu Asp
    1445                1450                1455

Ile Asn Glu Asp Pro Val Glu Gln Gly Cys Arg Glu Gln Phe Tyr

1460                1465                1470

Asp Met Val Leu Ile Thr Ala Ala Val Phe Leu Gln Glu Val Ala
    1475                1480                1485

Val Ala His Val Arg Arg Leu Leu Lys Pro Gly Gly Phe Leu Val
    1490                1495                1500

Leu Leu Val Arg Thr Asn Pro Ser Thr Thr Tyr Leu Asn Leu Leu
    1505                1510                1515

Phe Gly Pro Pro Met Arg Cys Thr Glu Thr Gly Lys Gly Tyr Cys
    1520                1525                1530

Ser Gly Glu Pro Ile Thr Thr Arg Arg Asp Trp Val Glu Leu Leu
    1535                1540                1545

Ser Asn Gly Gly Phe Tyr Gly Leu Asp Ser Phe Asp Ala Ser Gln
    1550                1555                1560

Glu Ser Glu Ser Leu Gly Asp Phe Ser Leu Leu Leu Cys Arg Thr
    1565                1570                1575

Pro Asp Ser Pro Ala Glu Pro Gln Ser Arg Gly Asp Leu Leu Leu
    1580                1585                1590

Leu Gly Gly Asp Ala Glu Glu Ala Asp Cys Leu Thr Ser Glu Leu
    1595                1600                1605

Phe Glu Leu Val Gln Asp Asp Phe Val Lys Val Ala His Ala Pro
    1610                1615                1620

Asp Leu Asp Leu Ile Glu Asp Arg Asp Leu Ser Lys Leu Thr Val
    1625                1630                1635

Leu Tyr Leu Val Asp Asp Arg Asp Leu Thr Asn Ala Thr Leu Ser
    1640                1645                1650

Glu Leu Cys Arg Leu Met Thr Val Ser Lys Arg Met Leu Val Val
    1655                1660                1665

Thr Cys Glu Lys Val Asp His Pro Asp Ala Gly Leu Val Lys Gly
    1670                1675                1680

Leu Leu Ser Thr Phe Leu Ala Ser Glu Arg Ser Ser Ser Leu Leu
    1685                1690                1695

Gln Leu Leu His Ile Thr Asp Pro Val Gly Val Thr Thr Glu Ile
    1700                1705                1710

Leu Ala Thr Ala Leu Gly His Phe Val Gln Ala Ser Ala Ala Gln
    1715                1720                1725

Glu Asn Pro His Ser Cys Gly Leu Thr Asn Ile Glu Pro Glu Ile
    1730                1735                1740

Gln Tyr Asp Gly Ser Met Phe Arg Val Pro Arg Gln Tyr His Asp
    1745                1750                1755

His Ala Thr Gly Leu Arg His Leu Ala Arg Arg Gln Lys Val Thr
    1760                1765                1770

Asp Cys Val Asp Leu Asp Lys Gly Val Val Gln Ile Leu Pro Ala
    1775                1780                1785

Thr Thr Asp Lys Thr Cys Glu Gly Phe Arg Leu Leu Ser Met Ala
    1790                1795                1800

Asp Pro Pro Ile Thr Ala Ser Tyr Gly Pro Thr Leu His Leu Arg
    1805                1810                1815

Val Arg His Ser Ser Ile Ala Ala Val Arg Val Ala Gly Ala Ile
    1820                1825                1830

Phe Leu Arg Leu Val Ile Gly Leu Asp Val Lys Ser Asn Lys Arg
    1835                1840                1845

Met Ile Ala Leu Ser Ser His Ile Ala Ser His Val Ile Val Pro
    1850                1855                1860

Asp Ser Trp Ala Trp Ser Val Pro Asp Thr Val Leu Glu Ala His
    1865                1870                1875

Glu Gln Ser Tyr Leu Arg Ala Thr Ala Ala Ala Leu Leu Ala Gly
    1880                1885                1890

Tyr Leu Val Glu Gln Val Pro Gln Ser Gly Thr Leu Val Val His
    1895                1900                1905

Glu Ala Asp Gly Val Leu Gln Ser Val Phe His Gln Met Leu Thr
    1910                1915                1920

Arg Arg Asp Gly Lys Val Ile Phe Ser Thr Ser Lys Ser Asn Pro
    1925                1930                1935

Asp Lys Glu Arg Pro Met Leu Leu Leu His Glu His Ser Thr Ala
    1940                1945                1950

Arg Gln Leu Ser Gln Val Leu Pro Ser Asp Val Ser Ala Ile Ala
    1955                1960                1965

Ile Leu His Arg Arg Gly Gln Gly Val Tyr Asp Arg Met Leu Ser
    1970                1975                1980

Leu Leu Pro Asp Asn Ala Thr Arg Ile His Leu Gln Asp Phe Tyr
    1985                1990                1995

Leu Thr Ser Ala Ser Thr Gly Pro Ile Asn Ala Asp Asp Ser Ser
    2000                2005                2010

Leu Ile Ala Lys Ala Phe Leu Thr Ala Cys Leu Val Ala Tyr Thr
    2015                2020                2025

Gly Arg Glu Gly Leu Pro Pro Asn Ser Val Asp Ser Leu Pro Ile
    2030                2035                2040

Ser Arg Ile Ser Glu Tyr Pro Ile Leu Asp Ser Gln Asp Ala Val
    2045                2050                2055

Val Asp Trp Asp Ser Thr Thr Pro Val Leu Ala Gln Ile Pro Thr
    2060                2065                2070

Ala Gly Ser Gln Val Gln Leu Ser Glu Lys Lys Thr Tyr Ile Leu
    2075                2080                2085

Val Gly Leu Gly Ser Glu Leu Ala His Ala Ile Cys Leu Trp Leu
    2090                2095                2100

Ala Thr His Gly Ala Lys Trp Ile Leu Leu Ala Gly Ser Arg Leu
    2105                2110                2115

Asp Ser Asp Ala Trp Trp Leu Glu Glu Val Ser Arg Arg Gly Thr
    2120                2125                2130

Arg Ile Ala Val Ser Lys Ile Asn Leu Ile Asp Gly Ile Ser Ala
    2135                2140                2145

Thr Ser Leu His Gln Thr Ile Pro Tyr Ala Phe Pro Pro Val Val
    2150                2155                2160

Gly Gly Val Leu Ile Gln Pro Pro Pro Leu Pro Asp Cys Ser Leu
    2165                2170                2175

Ser Gln Leu Thr Ile Asp Ser Leu Arg Asn His Leu His Pro Val
    2180                2185                2190

Leu Lys Gly Leu Gln Gln Leu Asp Glu Leu Tyr Lys Thr Pro Thr
    2195                2200                2205

Leu Asp Phe Trp Val Leu Ile Gly Ser Ile Ala Gly Val Leu Gly
    2210                2215                2220

His Ala Asp Gln Ala Met Thr Ala Ala Met Ser Glu Lys Met Ala
    2225                2230                2235

Leu Leu Val Arg His Arg Arg Ala Gln Gly Arg Pro Ala Ser Leu
    2240                2245                2250

Val His Leu Gly Glu Ile His Gly Ile Ser Ser Pro Ser Pro Ser
    2255                2260                2265

Gln Pro Leu Trp Cys Gly Pro Val Ala Val Ser Gln Arg Asp Val
    2270                2275                2280

Asp Glu Ile Leu Ala Glu Ala Ile Leu Cys Gly Arg Ser Asp Ser
    2285                2290                2295

Asn Ser Asn Ala Glu Leu Ile Gly Gly Leu Arg His Gln Ser Leu
    2300                2305                2310

Lys Cys Gly Tyr Gly Glu Cys Pro Ile Pro Lys Leu Trp Pro Phe
    2315                2320                2325

Tyr Ser Tyr Thr Ala Thr Ala Ser Gln Asp Gln Ile Leu Ala Leu
    2330                2335                2340

Ile Glu Thr Arg Ser Thr Lys Asp Leu Val Thr Ala Ala Thr Ser
    2345                2350                2355

Leu Glu Glu Lys Ala Glu Ala Val Val Arg Pro Leu Met Glu Lys
    2360                2365                2370

Ile Arg Ala Ser Leu Asn Leu Ala Glu Asp Ala Pro Leu Ser Ala
    2375                2380                2385

Asp Thr Leu Ile Pro Glu Leu Gly Ile Asp Ser Leu Ile Ala Ile
    2390                2395                2400

Gly Leu Ser Gln Trp Phe Thr Lys Glu Leu Ser Val Asp Ile Gly
    2405                2410                2415

Val Ile Leu Ile Leu Ser Gly Val Ser Val Gly Glu Leu Ala His
    2420                2425                2430

Ala Ala Ala Ser Lys Leu Cys Asn Val Ser Val Gly Lys Pro

2435                2440                2445

<210>  269
<211>  509
<212>  PRT
<213>  Penicillium coprobium PF1169

<400>  269

Met Asp Asn Met Asp Asn Met Asn Asn Thr Pro Leu Gly Phe Asn Trp
1               5                   10                  15

Ala Trp Ala Val Ile Ile Ser Phe Leu Gly Leu Leu Thr Phe Ser Phe
            20                  25                  30

Val Ser Pro His Leu Phe Pro Ser Arg Leu Thr Val Ile Asn Gly Gly
        35                  40                  45

Arg Ala Trp Asp Ile Phe Arg Thr Lys Ala Lys Lys Arg Phe Arg Ser
    50                  55                  60

Asp Ala Ala Arg Leu Ile Lys Asn Gly Phe Glu Glu Ser Pro Asp Ala
65                  70                  75                  80

Phe Arg Ile Ile Thr Asp Asn Gly Pro Leu Leu Val Leu Ser Pro Gln
                85                  90                  95

Tyr Ala Arg Glu Val Arg Ser Asp Asp Arg Leu Ser Leu Asp His Phe
            100                 105                 110

Ile Ala Ser Glu Phe His Pro Asn Ile Pro Gly Phe Glu Pro Phe Lys
        115                 120                 125

Leu Ile Leu Asp Pro Lys Asn Pro Leu Asn Thr Ile Leu Lys Ser Asn
    130                 135                 140

Leu Thr Gln Ala Leu Glu Asp Leu Ser Ala Glu Val Thr Glu Ala Leu
145                 150                 155                 160

Ser Ala Thr Cys Thr Asp Asp Pro Glu Trp His Glu Val Ser Val Ser
                165                 170                 175

Gln Thr Ala Leu Lys Ile Ile Ala Gln Met Ala Ser Lys Ala Phe Ile
            180                 185                 190

Gly Gln Glu Arg Cys Arg Asp Ala Lys Trp His Asn Ile Ile Ile Thr
        195                 200                 205

Tyr Thr His Asn Val Tyr Gly Ala Ala Gln Ala Leu His Phe Trp Pro
    210                 215                 220

Ser Phe Leu Arg Pro Ile Val Ala Gln Phe Leu Pro Ala Cys Arg Thr
225                 230                 235                 240

Leu Gln Ala Gln Ile Ala Glu Ala Arg Glu Ile Leu Glu Pro Leu Val
                245                 250                 255

Ala Gln Arg Arg Ala Glu Arg Ala Thr Arg Ala Ala Gln Glu Lys Pro
            260                 265                 270

His Pro Ser Gly Gly Asp Ile Ile Asp Trp Leu Glu Gln Phe Tyr Gly
        275                 280                 285

Asp Gln Pro Tyr Asp Pro Val Ala Ala Gln Leu Leu Leu Ser Phe Ala
    290                 295                 300

Ala Ile His Gly Thr Ser Asn Leu Leu Ala Gln Ala Leu Ile Asp Leu
305                 310                 315                 320

Cys Gly Gln Pro Glu Leu Val Gln Asp Leu Arg Glu Glu Ala Val Ser
                325                 330                 335

Val Leu Gly Lys Glu Gly Trp Thr Arg Ala Ala Leu Tyr Gln Leu Lys
            340                 345                 350

Leu Met Asp Ser Ala Leu Lys Glu Ser Gln Arg Leu Ala Pro Asn Arg
        355                 360                 365

Leu Leu Ser Met Gly Arg Ile Ala Gln Gly Asp Met Asp Leu Ser Asp
    370                 375                 380

Gly Leu Arg Ile His Arg Gly Thr Thr Leu Met Val Ser Ala His Asn
385                 390                 395                 400

Met Trp Asp Pro Glu Ile Tyr Pro Asp Pro Arg Lys Tyr Asp Gly Tyr
                405                 410                 415

Arg Phe His Lys Leu Arg Gln Thr Ser Gly Gln Glu Gly Gln His Gln
            420                 425                 430

Leu Val Ser Ser Thr Pro Asp His Met Gly Phe Gly Tyr Gly Lys His
        435                 440                 445

Ala Cys Pro Gly Arg Phe Phe Ala Ala Ala Gln Ile Lys Val Ala Leu
    450                 455                 460

Cys Asn Ile Leu Leu Lys Tyr Asp Ile Glu Tyr Arg Gly Gly Lys Ser
465                 470                 475                 480

Pro Gly Val Trp Gly Gln Gly Ile His Leu Phe Pro Asp Pro Thr Ser
                485                 490                 495

Arg Ile His Val Arg Arg Arg Lys Glu Glu Ile Asn Leu
            500                 505

<210>  270
<211>  505
<212>  PRT
<213>  Penicillium coprobium PF1169

<400>  270

Met Ile Glu Leu Lys Asp Ala Ser Met Gly Ala Val Leu Leu Thr Cys
1               5                   10                  15

Val Leu Val Leu Ala Gly Leu Tyr Leu Ile Arg Leu Thr Leu Ser Ser
            20                  25                  30

Asp Gln Leu Asp Lys Phe Pro Ser Ile Asn Pro Arg Lys Pro Trp Glu
        35                  40                  45

Ile Val Asn Val Phe Ala Gln Arg Arg Phe Gln Gln Asp Gly Pro Arg
    50                  55                  60

Tyr Leu Glu Ala Gly Tyr Ala Lys Ser Pro Ile Phe Ser Val Val Thr
65                  70                  75                  80

Asp Leu Gly Pro Lys Leu Val Val Ser Gly Ala Phe Ile Glu Glu Phe
                85                  90                  95

Lys Asp Glu Lys Leu Leu Asp His Tyr Arg Ser Met Ile Glu Asp Phe
            100                 105                 110

Met Ala Glu Val Pro Gly Phe Glu Ser Met Phe Leu Gly Asn Leu His
        115                 120                 125

Asn Thr Val Leu Arg Asp Val Ile Ser Val Ile Thr Arg Glu Leu Glu
    130                 135                 140

Gln Leu Leu Ala Pro Leu Ser Asp Glu Val Ser Ala Ala Leu Val Asp
145                 150                 155                 160

Thr Trp Thr Asp Ser Pro Asp Trp His Glu Val Ala Leu Leu Pro Ser
                165                 170                 175

Met Leu Gly Leu Ile Ala Lys Val Ser Ser Leu Val Phe Val Gly Glu
            180                 185                 190

Pro Leu Cys Arg His Pro Val Trp Leu Glu Thr Val Ile Asn Phe Thr
        195                 200                 205

Leu Ile Arg His Asn Ala Ile Leu Ala Leu His Gln Cys Pro Ala Val
    210                 215                 220

Leu Arg Pro Val Leu His Trp Val Leu Pro Pro Cys Gln Lys Leu Arg
225                 230                 235                 240

Arg Glu Ile Arg Thr Ala Arg Thr Leu Ile Asp Ser Ala Leu Glu Lys
                245                 250                 255

Ser Arg Lys Asn Pro Gln Thr Glu Lys Phe Ser Ser Val Ala Trp Val
            260                 265                 270

Asp Ala Phe Ala Lys Gly Asn Lys Tyr Asn Ala Ala Met Val Gln Leu
        275                 280                 285

Arg Leu Ala Asn Ala Ser Ile His Ser Ser Ala Asp Leu Leu Val Lys
    290                 295                 300

Ile Leu Ile Asn Leu Cys Glu Gln Pro Glu Leu Ile Arg Asp Leu Arg
305                 310                 315                 320

Asp Glu Ile Ile Ser Val Leu Gly Glu Asn Gly Trp Arg Ser Ser Thr
                325                 330                 335

Leu Asn Gln Leu Lys Leu Leu Asp Ser Val Leu Lys Glu Ser Gln Arg
            340                 345                 350

Leu His Pro Val Thr Thr Gly Ala Phe Ser Arg Phe Thr Arg Gln Asp
        355                 360                 365

Ile Lys Leu Thr Asn Gly Thr Glu Ile Pro Ser Gly Thr Pro Ile Met
    370                 375                 380

Val Thr Asn Asp Val Ala Gly Asp Ala Ser Ile Tyr Asp Asp Pro Asp
385                 390                 395                 400

Val Phe Asp Gly Tyr Arg Tyr Phe Arg Met Arg Glu Gly Ala Asp Lys
                405                 410                 415

Ala Arg Ala Pro Phe Thr Thr Thr Gly Gln Asn His Leu Gly Phe Gly
            420                 425                 430

Tyr Gly Lys Tyr Ala Cys Pro Gly Arg Phe Phe Ala Ala Thr Glu Ile
        435                 440                 445

Lys Ile Ala Leu Cys His Met Leu Leu Lys Tyr Glu Trp Arg Leu Val
    450                 455                 460

Lys Asp Arg Pro His Gly Ile Val Thr Ser Gly Phe Ala Ala Phe Arg
465                 470                 475                 480

Asp Pro Arg Ala Ser Ile Glu Val Arg Arg Arg Ala Val Ala Gly Glu
                485                 490                 495

Glu Leu Glu Val Leu Thr Gly Lys Lys
            500                 505

<210>  271
<211>  241
<212>  PRT
<213>  Penicillium coprobium PF1169

<400>  271

Met Asp Gly Trp Ser Asp Ile Ser Ser Ala Pro Ala Gly Tyr Lys Asp
1               5                   10                  15

Val Val Trp Ile Ala Asp Arg Ala Leu Leu Ala Gln Gly Leu Gly Trp
            20                  25                  30

Ser Ile Asn Tyr Leu Ala Met Ile Tyr Gln Ser Arg Lys Asp Arg Thr
        35                  40                  45

Tyr Gly Met Ala Ile Leu Pro Leu Cys Cys Asn Phe Ala Trp Glu Phe
    50                  55                  60

Val Tyr Thr Val Ile Tyr Pro Ser Gln Asn Pro Phe Glu Arg Ala Val
65                  70                  75                  80

Leu Thr Thr Trp Met Val Leu Asn Leu Tyr Leu Met Tyr Thr Thr Ile
                85                  90                  95

Lys Phe Ala Pro Asn Glu Trp Gln His Ala Pro Leu Val Gln Arg Ile
            100                 105                 110

Leu Pro Val Ile Phe Pro Val Ala Ile Ala Ala Phe Thr Ala Gly His
        115                 120                 125

Leu Ala Leu Ala Ala Thr Val Gly Val Ala Lys Ala Val Asn Trp Ser
    130                 135                 140

Ala Phe Leu Cys Phe Glu Leu Leu Thr Ala Gly Ala Val Cys Gln Leu
145                 150                 155                 160

Met Ser Arg Gly Ser Ser Arg Gly Ala Ser Tyr Thr Ile Trp Val Ser
                165                 170                 175

Arg Phe Leu Gly Ser Tyr Ile Gly Ser Ile Phe Met His Val Arg Glu

180                 185                 190

Thr His Trp Pro Gln Glu Phe Asp Trp Ile Ser Tyr Pro Phe Val Ala
        195                 200                 205

Trp His Gly Ile Met Cys Phe Ser Leu Asp Ile Ser Tyr Val Gly Leu
    210                 215                 220

Leu Trp Tyr Ile Arg Arg Gln Glu Arg Gln Gly Gln Leu Lys Lys Ala
225                 230                 235                 240

Met

<210>  272
<211>  464
<212>  PRT
<213>  Penicillium coprobium PF1169

<400>  272

Met Lys Val Ile Ile Val Gly Gly Ser Ile Ala Gly Leu Ala Leu Ala
1               5                   10                  15

His Cys Leu Asp Lys Ala Asn Ile Asp Tyr Val Ile Leu Glu Lys Lys
            20                  25                  30

Lys Glu Ile Ala Pro Gln Glu Gly Ala Ser Ile Gly Ile Met Pro Asn
        35                  40                  45

Gly Gly Arg Ile Leu Glu Gln Leu Gly Leu Tyr Asp Gln Ile Glu Glu
    50                  55                  60

Leu Ile Glu Pro Leu Val Arg Ala His Val Thr Tyr Pro Asp Gly Phe
65                  70                  75                  80

Asn Tyr Thr Ser Arg Tyr Pro Ala Leu Ile Gln Gln Arg Phe Gly Tyr
                85                  90                  95

Pro Leu Ala Phe Leu Asp Arg Gln Lys Leu Leu Gln Ile Leu Ala Thr
            100                 105                 110

Gln Pro Val Gln Ser Ser Arg Val Lys Leu Asp His Lys Val Glu Ser
        115                 120                 125

Ile Glu Val Ser Pro Cys Gly Val Thr Val Ile Thr Ser Asn Gly His
    130                 135                 140

Thr Tyr Gln Gly Asp Leu Val Val Gly Ala Asp Gly Val His Ser Arg
145                 150                 155                 160

Val Arg Ala Glu Met Trp Arg Leu Ala Asp Ala Ser Gln Gly Asn Val
                165                 170                 175

Cys Gly Asn Gly Asp Lys Ala Phe Thr Ile Asn Tyr Ala Cys Ile Phe
            180                 185                 190

Gly Ile Ser Ser His Val Asp Gln Leu Asp Pro Gly Glu Gln Ile Thr
        195                 200                 205

Cys Tyr Asn Asp Gly Trp Ser Ile Leu Ser Val Ile Gly Gln Asn Gly
    210                 215                 220

Arg Ile Tyr Trp Phe Leu Phe Ile Lys Leu Glu Lys Glu Phe Val Tyr
225                 230                 235                 240

Asp Gly Ser His Lys Thr Gln Leu His Phe Ser Arg Glu Asp Ala Arg
                245                 250                 255

Ala His Cys Glu Arg Leu Ala Gln Glu Pro Leu Trp Lys Asp Val Thr
            260                 265                 270

Phe Gly Gln Val Trp Ala Arg Cys Glu Val Phe Gln Met Thr Pro Leu
        275                 280                 285

Glu Glu Gly Val Leu Gly Lys Trp His Trp Arg Asn Ile Ile Cys Ile
    290                 295                 300

Gly Asp Ser Met His Lys Phe Ala Pro His Ile Gly Gln Gly Ala Asn
305                 310                 315                 320

Cys Ala Ile Glu Asp Ala Ala Gln Leu Ser Asn Ser Leu His Thr Trp
                325                 330                 335

Leu Ser Gly Ser Gly Lys Glu His Gln Leu Lys Thr Asp Asp Leu Thr
            340                 345                 350

Glu Ile Leu Ala Gln Phe Ala Gln Thr Arg Leu Gln Arg Leu Gly Pro
        355                 360                 365

Thr Ala Met Ala Ala Arg Ser Ala Met Arg Leu His Ala Arg Glu Gly
    370                 375                 380

Leu Lys Asn Trp Ile Leu Gly Arg Tyr Phe Leu Pro Tyr Ala Gly Asp
385                 390                 395                 400

Lys Pro Ala Asp Trp Ala Ser Arg Gly Ile Ala Gly Gly Asn Thr Leu
                405                 410                 415

Asp Phe Val Glu Pro Pro Thr Arg Ala Gly Pro Gly Trp Ile Gln Phe
            420                 425                 430

Ser Gln Ser Gly Lys Arg Thr Ser Phe Pro Met Ala Val Ala Gly Leu
        435                 440                 445

Cys Leu Val Ser Ile Val Ala Arg Ile Met Tyr Leu Lys Leu Val Ala
    450                 455                 460

<210>  273
<211>  317
<212>  PRT
<213>  Penicillium coprobium PF1169

<400>  273

Met Ala Gly Ser Gln Ser Thr Ala Gln Leu Ala Arg Leu Leu Ile Asp
1               5                   10                  15

Ile Ser Arg Phe Asp Lys Tyr Asn Cys Leu Phe Ala Ile Phe Pro Gly
            20                  25                  30

Val Trp Ser Ile Phe Leu Ala Ala Ala Ser Arg His Ala Asp Gly Asp
        35                  40                  45

Pro Val Pro Leu Asp Phe Val Leu Gly Arg Ala Gly Leu Ala Phe Met
    50                  55                  60

Tyr Thr Tyr Met Leu Ser Gly Ala Gly Met Val Trp Asn Asp Trp Ile
65                  70                  75                  80

Asp Arg Asp Ile Asp Ala Gln Val Ala Arg Thr Lys Asn Arg Pro Leu
                85                  90                  95

Ala Ser Gly Arg Leu Ser Thr Arg Ala Ala Leu Ile Trp Met Leu Val
            100                 105                 110

Gln Tyr Ala Ala Ser Val Trp Leu Met Asp Arg Met Val Ser Gly Gln
        115                 120                 125

Asp Val Trp Thr Tyr Met Leu Pro Leu Thr Thr Gly Ile Ile Leu Tyr
    130                 135                 140

Pro Phe Gly Lys Arg Pro Thr Ser Arg Lys Leu Gly Val Tyr Pro Gln
145                 150                 155                 160

Tyr Ile Leu Gly Ala Ser Ser Ala Leu Thr Ile Leu Pro Ala Trp Ala
                165                 170                 175

Ser Val Tyr Thr Gly Arg Ile Ser Leu Lys Asp Leu Gly Met Arg Cys
            180                 185                 190

Leu Pro Leu Cys Leu Phe Leu Phe Leu Trp Thr Ile Tyr Phe Asn Thr
        195                 200                 205

Ala Tyr Ser Tyr Gln Asp Ile Lys Asp Asp Cys Lys Leu Asn Val Asn
    210                 215                 220

Ser Ser Tyr Val Leu Ala Gly Ser His Val Arg Gly Met Leu Leu Leu
225                 230                 235                 240

Gln Ala Ile Ala Val Val Leu Val Ile Pro Trp Ile Leu Tyr Thr Ser
                245                 250                 255

Ala Ser Thr Trp Leu Trp Val Ser Trp Leu Gly Val Trp Thr Ala Ser
            260                 265                 270

Leu Gly Glu Gln Leu Tyr Leu Phe Asp Val Lys Asp Pro Ser Ser Gly
        275                 280                 285

Gly Lys Val His Arg Arg Asn Phe Ala Leu Gly Ile Trp Asn Val Leu
    290                 295                 300

Ala Cys Phe Val Glu Leu Leu Tyr Ala Ser Gly Ser Leu
305                 310                 315

<210> 274
<211> 522
<212> PRT
<213> Penicillium coprobium PF1169

<400> 274

Met Ser Thr Gln Glu Val Cys Leu Pro Val Ser Gln Arg Asp Gln Val
1               5                   10                  15

Lys Glu Gly Pro Val Arg Leu His Gly Leu Cys Glu Asp Gly Met Cys
            20                  25                  30

Asp Ala Arg Arg Thr Gly Asp Arg Ser Ala Tyr Pro Leu Ser Ser Leu
        35                  40                  45

Asp His Asn Pro Leu Gly Met Asn Val Thr Phe Leu Leu Phe Phe Gln
    50                  55                  60

Thr Thr Gln Pro Glu Lys Ser Ile Gly Val Leu Glu Asn Gly Ile Glu
65                  70                  75                  80

Leu Leu Leu Lys Val His Pro Phe Leu Ala Gly Asp Val Thr Arg Arg
                85                  90                  95

Thr Glu Ser Ser Gln Thr Lys Tyr Thr Trp Gln Ile Glu Pro Glu Ala
            100                 105                 110

Ser Glu Ser Leu Val Gln Phe Pro Ile Leu Arg Ile Arg His Tyr Gln
        115                 120                 125

Ala Glu Ser Phe Lys Glu Ile Gln Ser Lys Cys Leu Leu Thr Gly Thr
    130                 135                 140

Glu Glu Gln Glu Ile Ile Ser Arg Leu Ala Pro Leu Pro Ile Asp Met
145                 150                 155                 160

Asp Ile Ser Leu Pro Arg Arg Pro Ile Leu Arg Phe Gln Ala Asn Val
                165                 170                 175

Met Arg Asp Gly Ile Ile Leu Ala Met Thr Phe His His Ser Ala Met
            180                 185                 190

Asp Gly Ala Gly Ala Ala Arg Val Leu Gly Leu Leu Ala Asp Cys Cys
        195                 200                 205

Arg Asp Pro Thr Ala Met Ser Ser Ala Ser Val Ser Pro Asp Arg Gln
    210                 215                 220

Leu Arg Ser Glu Ile Glu Arg Leu Val Pro Glu Ser Ser Ser Gly Leu
225                 230                 235                 240

Ser Arg Met Asp Phe Ser Lys His Tyr Cys Gly Leu Gly Asp Trp Ala
                245                 250                 255

Ala Leu Leu Ala Lys Asn Trp Ser Gly Phe Val Arg Ala Arg Ala Thr
            260                 265                 270

Glu Leu Val Thr Trp Arg Leu Lys Ile Pro Gly Pro Lys Ile Glu Tyr
        275                 280                 285

Leu Lys Glu Ala Cys Asn Thr Leu Ile Lys Gly Gln Thr Ser Phe Gln
    290                 295                 300

Ala Asp Gly Arg Pro Ser Pro Gly Phe Leu Ser Ser Asn Asp Ile Val
305                 310                 315                 320

Ser Ala Leu Leu Ala Met Ile Leu Arg Gln Ala Gly Gln Leu Ala Gly
                325                 330                 335

Lys Ser Thr Glu Leu Ser Ile Ala Val Asp Met Arg Gly Asn Phe Lys
            340                 345                 350

Thr Pro Ala Phe Asp Asp Tyr Leu Gly Asn Met Val Leu Leu Thr Tyr

355                 360                 365

Thr Pro Ile Gln Ala Gly Arg Asn Glu Ala Leu Val Asp Gly Thr Asp
    370                 375                 380

Pro Ser Val Glu Leu Arg Gln Glu Cys Leu Glu Asp Leu Thr Gln Ile
385                 390                 395                 400

Ala Ala Arg Ile Arg Gln Ser Leu Leu Ala Val Asp Ala Glu Tyr Ile
                405                 410                 415

Gln Asp Ala Leu Ser His Leu His Ser Gln Pro Asp Trp Ala Asp Ile
            420                 425                 430

Gly Phe Arg Gly Val Pro Ile Pro Leu Ser Ser Phe Arg Asn Phe Glu
        435                 440                 445

Ile Phe Gly Leu Asp Phe Gly Glu Ser Leu Gly Ala Gln Pro Arg Gly
    450                 455                 460

Phe Gln Leu His Leu Pro Val Leu Gly Gly Met Cys Phe Ile Leu Pro
465                 470                 475                 480

Lys Gly Gln Asp Asp Val Ala Ser Thr Glu Pro Trp Asp Leu His Leu
                485                 490                 495

Thr Leu Asn Arg Asp Asp Gln Leu Leu Leu Ala Lys Asp Pro Leu Phe
            500                 505                 510

Cys Trp Ala Ile Gly Ala Gln Ala Lys Glu
        515                 520

<210>  275
<211>  434
<212>  PRT
<213>  Penicillium coprobium PF1169

<400>  275

Met Asp Ser Leu Leu Thr Ser Pro Leu Trp Leu Lys Ile Ala His Glu
1               5                   10                  15

Leu Ala Leu Tyr Leu Ser Phe Ile Val Pro Thr Ala Phe Leu Ile Ile
            20                  25                  30

Thr Thr Gln Lys Ser Ser Ile Ile Arg Trp Ala Trp Thr Pro Cys Leu
        35                  40                  45

Leu Tyr Ile Leu Tyr Gln Phe Ser Leu Arg Val Pro Ser Leu Ser Thr
    50                  55                  60

Ser Gln Phe Leu Lys Gly Val Ala Ala Gly Gln Ala Thr Val Ala Ala
65                  70                  75                  80

Leu Gln Cys Leu Asn Leu Leu Leu Ile Thr Lys Leu Asp Gln Thr Asp
                85                  90                  95

Leu Leu Arg Ala Asn Leu Tyr Ser Pro Ser Ala Gly Leu Leu Ser Arg
            100                 105                 110

Leu Ala Gln Ser Cys Ala Leu Leu Val Asn Phe Arg Gly Ile Gly Thr
        115                 120                 125

Ile Trp Glu Val Arg Asn Ile Pro Gln His Ala Ala Phe Val Gln Pro
    130                 135                 140

Lys Gly Lys Asp Gln Ser Met Ser Arg Lys Arg Phe Val Leu Arg Glu
145                 150                 155                 160

Ile Ala Ile Ile Val Trp Gln Tyr Leu Leu Leu Asp Phe Ile Tyr Glu
                165                 170                 175

Ser Thr Lys Gly Thr Ser Ala Glu Asp Leu Met Arg Leu Phe Gly Pro
            180                 185                 190

Gly Met Glu Ile Lys Tyr Leu Asp Ala Thr Phe Glu Gln Trp Met Gly
        195                 200                 205

Arg Leu Ser Val Gly Ile Phe Ser Trp Leu Val Pro Ser Arg Val Cys
    210                 215                 220

Leu Asn Ile Thr Ser Arg Leu Tyr Phe Leu Ile Leu Val Val Leu Gly
225                 230                 235                 240

Ile Ser Ser Pro Glu Ser Cys Arg Pro Gly Phe Gly Arg Val Arg Asp
                245                 250                 255

Val Cys Thr Ile Arg Gly Val Trp Gly Lys Phe Trp His Gln Ser Phe
            260                 265                 270

Arg Trp Pro Leu Thr Ser Val Gly Asn Tyr Ile Ala Arg Asp Val Leu
        275                 280                 285

Gly Leu Ala His Pro Ser Leu Leu Glu Arg Tyr Thr Asn Ile Phe Phe
    290                 295                 300

Thr Phe Phe Thr Ser Gly Val Leu His Leu Val Cys Asp Ala Ile Leu
305                 310                 315                 320

Gly Val Pro Pro Ser Ala Ser Gly Ala Met Gln Phe Phe Cys Ser Phe
                325                 330                 335

Pro Leu Ala Ile Met Ile Glu Asp Gly Val Gln Glu Ile Trp Arg Arg
            340                 345                 350

Ala Thr Gly Gln Thr Lys Asp Ser Asp Arg Ala Val Pro Phe Trp Gln
        355                 360                 365

Arg Leu Val Gly Tyr Leu Trp Val Ala Val Trp Met Cys Val Thr Ser
    370                 375                 380

Pro Phe Tyr Leu Tyr Pro Ala Ala Arg Gln His Ala Glu Lys Asn Trp
385                 390                 395                 400

Ile Val Pro Phe Ser Ile Val Glu Glu Ile Gly Leu Gly Thr Ala Gln
                405                 410                 415

Lys Ile Leu Leu Gly Tyr Gly Leu Phe Val Tyr Trp Ala Val Gly Gly
            420                 425                 430

Glu Ile

<210>  276
<211>  1299
<212>  PRT
<213>  Penicillium coprobium PF1169

<400>  276

Met Leu Tyr Arg Ala Lys Leu Val Asp Asp His Gln Ile His Thr Ala
1               5                   10                  15

Ser Leu His Asn Pro Ile Pro Trp Gln Leu His Thr Tyr Val Trp Pro
            20                  25                  30

Phe Leu Ile Ile Trp Pro Val Phe Phe Ala Phe Tyr Leu Ser Pro Glu
        35                  40                  45

Arg Tyr Asp Thr Tyr Ile Gln Gly Gln Glu Trp Thr Phe Val Phe Ala
    50                  55                  60

Gly Ser Ile Ile Thr Val Gln Ser Leu Phe Trp Leu Met Thr Lys Trp
65                  70                  75                  80

Asn Ile Asp Ile Asn Thr Leu Phe Thr Thr Thr Arg Ser Lys Ser Ile
                85                  90                  95

Asp Thr Ala Arg Leu Ile Lys Val Val Pro Ile Thr Asn Ala Gly Ser
            100                 105                 110

Ala Glu Ile Cys Asn Leu Ile Arg Glu His Ile Gly Pro Lys Lys Thr
        115                 120                 125

Leu Ser Phe Leu Phe Gln Lys Arg Arg Phe Leu Phe Tyr Pro Glu Thr
    130                 135                 140

Arg Ser Phe Ala Pro Leu Ser Tyr Ala Leu Asp Ala Glu Pro Lys Pro
145                 150                 155                 160

Ala Leu Lys Thr Phe Gln Gln Ser Glu Gly Phe Thr Ser Lys Ala Glu
                165                 170                 175

Ile Glu Arg Val Gln Asn His Tyr Gly Asp Asn Thr Phe Asp Ile Pro
            180                 185                 190

Val Pro Gly Phe Ile Glu Leu Phe Gln Glu His Ala Val Ala Pro Phe
        195                 200                 205

Phe Val Phe Gln Ile Phe Cys Val Gly Leu Trp Met Leu Asp Glu Tyr
    210                 215                 220

Trp Tyr Tyr Ser Leu Phe Thr Leu Phe Met Leu Val Met Phe Glu Ser
225                 230                 235                 240

Thr Val Val Trp Gln Arg Gln Arg Thr Leu Ser Glu Phe Arg Gly Met
                245                 250                 255

Ser Ile Lys Pro Tyr Asp Val Trp Val Tyr Arg Glu Arg Lys Trp Gln
            260                 265                 270

Glu Ile Thr Ser Asp Lys Leu Leu Pro Gly Asp Leu Met Ser Val Asn
        275                 280                 285

Arg Thr Lys Glu Asp Ser Gly Val Ala Cys Asp Ile Leu Leu Val Glu
    290                 295                 300

Gly Ser Val Ile Val Asn Glu Ala Met Leu Ser Gly Glu Ser Thr Pro
305                 310                 315                 320

Leu Leu Lys Asp Ser Ile Gln Leu Arg Pro Gly Asp Asp Leu Ile Glu
                325                 330                 335

Pro Asp Gly Leu Asp Lys Leu Ser Phe Val His Gly Gly Thr Lys Val
            340                 345                 350

Leu Gln Val Thr His Pro Asn Leu Thr Gly Asp Ala Gly Leu Lys Asn
        355                 360                 365

Leu Ala Ser Asn Val Thr Met Pro Pro Asp Asn Gly Ala Leu Gly Val
    370                 375                 380

Val Val Lys Thr Gly Phe Glu Thr Ser Gln Gly Ser Leu Val Arg Thr
385                 390                 395                 400

Met Ile Tyr Ser Thr Glu Arg Val Ser Ala Asn Asn Val Glu Ala Leu
                405                 410                 415

Leu Phe Ile Leu Phe Leu Leu Ile Phe Ala Ile Ala Ala Ser Trp Tyr
            420                 425                 430

Val Trp Gln Glu Gly Val Ile Arg Asp Arg Lys Arg Ser Lys Leu Leu
        435                 440                 445

Leu Asp Cys Val Leu Ile Ile Thr Ser Val Val Pro Pro Glu Leu Pro
    450                 455                 460

Met Glu Leu Ser Leu Ala Val Asn Thr Ser Leu Ala Ala Leu Ser Lys
465                 470                 475                 480

Tyr Ala Ile Phe Cys Thr Glu Pro Phe Arg Ile Pro Phe Ala Gly Arg
                485                 490                 495

Val Asp Ile Ala Cys Phe Asp Lys Thr Gly Thr Leu Thr Gly Glu Asp
            500                 505                 510

Leu Val Val Asp Gly Ile Ala Gly Leu Thr Leu Gly Glu Ala Gly Ser
        515                 520                 525

Lys Val Glu Ala Asp Gly Ala His Thr Glu Leu Ala Asn Ser Ser Ala
    530                 535                 540

Ala Gly Pro Asp Thr Thr Leu Val Leu Ala Ser Ala His Ala Leu Val
545                 550                 555                 560

Lys Leu Asp Glu Gly Glu Val Val Gly Asp Pro Met Glu Lys Ala Thr
                565                 570                 575

Leu Glu Trp Leu Gly Trp Thr Leu Gly Lys Asn Asp Thr Leu Ser Ser
            580                 585                 590

Lys Gly Asn Ala Pro Val Val Ser Gly Arg Ser Val Glu Ser Val Gln
        595                 600                 605

Ile Lys Arg Arg Phe Gln Phe Ser Ser Ala Leu Lys Arg Gln Ser Thr
    610                 615                 620

Ile Ala Thr Ile Thr Thr Asn Asp Arg Asn Ala Ser Lys Lys Thr Lys
625                 630                 635                 640

Ser Thr Phe Val Gly Val Lys Gly Ala Pro Glu Thr Ile Asn Thr Met
                645                 650                 655

Leu Val Asn Thr Pro Pro Asn Tyr Glu Glu Thr Tyr Lys His Phe Thr
            660                 665                 670

Arg Asn Gly Ala Arg Val Leu Ala Leu Ala Tyr Lys Tyr Leu Ser Ser
        675                 680                 685

Glu Thr Glu Leu Ser Gln Ser Arg Val Asn Asn Tyr Val Arg Glu Glu
    690                 695                 700

Ile Glu Ser Glu Leu Ile Phe Ala Gly Phe Leu Val Leu Gln Cys Pro
705                 710                 715                 720

Leu Lys Asp Asp Ala Ile Lys Ser Val Gln Met Leu Asn Glu Ser Ser
                725                 730                 735

His Arg Val Val Met Ile Thr Gly Asp Asn Pro Leu Thr Ala Val His
            740                 745                 750

Val Ala Arg Lys Val Glu Ile Val Asp Arg Glu Val Leu Ile Leu Asp
        755                 760                 765

Ala Pro Glu His Asp Asn Ser Gly Thr Lys Ile Val Trp Arg Thr Ile
    770                 775                 780

Asp Asp Lys Leu Asn Leu Glu Val Asp Pro Thr Lys Pro Leu Asp Pro
785                 790                 795                 800

Glu Ile Leu Lys Thr Lys Asp Ile Cys Ile Thr Gly Tyr Ala Leu Ala
                805                 810                 815

Lys Phe Lys Gly Gln Lys Ala Leu Pro Asp Leu Leu Arg His Thr Trp
            820                 825                 830

Val Tyr Ala Arg Val Ser Pro Lys Gln Lys Glu Glu Ile Leu Leu Gly
        835                 840                 845

Leu Lys Asp Ala Gly Tyr Thr Thr Leu Met Cys Gly Asp Gly Thr Asn
    850                 855                 860

Asp Val Gly Ala Leu Lys Gln Ala His Val Gly Val Ala Leu Leu Asn
865                 870                 875                 880

Gly Ser Gln Glu Asp Leu Thr Lys Ile Ala Glu His Tyr Arg Asn Thr

                885                 890                 895

Lys Met Lys Glu Leu Tyr Glu Lys Gln Val Ser Met Met Gln Arg Phe
            900                 905                 910

Asn Gln Pro Ala Pro Pro Val Pro Val Leu Ile Ala His Leu Tyr Pro
        915                 920                 925

Pro Gly Pro Thr Asn Pro His Tyr Glu Lys Ala Met Glu Arg Glu Ser
    930                 935                 940

Gln Arg Lys Gly Ala Ala Ile Thr Ala Pro Gly Ser Thr Pro Glu Ala
945                 950                 955                 960

Ile Pro Thr Ile Thr Ser Pro Gly Ala Gln Ala Leu Gln Gln Ser Asn
                965                 970                 975

Leu Asn Pro Gln Gln Gln Lys Lys Gln Gln Ala Gln Ala Ala Ala Ala
            980                 985                 990

Gly Leu Ala Asp Lys Leu Thr Ser Ser Met Met Glu Gln Glu Leu Asp
        995                 1000                1005

Asp Ser Glu Pro Pro Thr Ile Lys Leu Gly Asp Ala Ser Val Ala
    1010                1015                1020

Ala Pro Phe Thr Ser Lys Leu Ala Asn Val Ile Ala Ile Pro Asn
    1025                1030                1035

Ile Ile Arg Gln Gly Arg Cys Thr Leu Val Ala Thr Ile Gln Met
    1040                1045                1050

Tyr Lys Ile Leu Ala Leu Asn Cys Leu Ile Ser Ala Tyr Ser Leu
    1055                1060                1065

Ser Val Ile Tyr Leu Asp Gly Ile Lys Phe Gly Asp Gly Gln Val
    1070                1075                1080

Thr Ile Ser Gly Met Leu Met Ser Val Cys Phe Leu Ser Ile Ser
    1085                1090                1095

Arg Ala Lys Ser Val Glu Gly Leu Ser Lys Glu Arg Pro Gln Pro
    1100                1105                1110

Asn Ile Phe Asn Val Tyr Ile Ile Gly Ser Val Leu Gly Gln Phe
    1115                1120                1125

Ala Ile His Ile Ala Thr Leu Ile Tyr Leu Ser Asn Tyr Val Tyr
    1130                1135                1140

Lys His Glu Pro Arg Asp Ser Asp Ile Asp Leu Glu Gly Glu Phe
    1145                1150                1155

Glu Pro Ser Leu Leu Asn Ser Ala Ile Tyr Leu Leu Gln Leu Ile
    1160                1165                1170

Gln Gln Ile Ser Thr Phe Ser Ile Asn Tyr Gln Gly Arg Pro Phe
    1175                1180                1185

Arg Glu Ser Ile Arg Glu Asn Lys Gly Met Tyr Trp Gly Leu Ile
    1190                1195                1200

Ala Ala Ser Gly Val Ala Phe Ser Cys Ala Thr Glu Phe Ile Pro
    1205                1210                1215

Glu Leu Asn Glu Lys Leu Arg Leu Val Pro Phe Thr Asn Glu Phe
    1220                1225                1230

Lys Val Thr Leu Thr Val Leu Met Ile Phe Asp Tyr Gly Gly Cys
    1235                1240                1245

Trp Leu Ile Glu Asn Val Leu Lys His Leu Phe Ser Asp Phe Arg
    1250                1255                1260

Pro Lys Asp Ile Ala Ile Arg Arg Pro Asp Gln Leu Lys Arg Glu
    1265                1270                1275

Ala Glu Arg Lys Leu Gln Glu Gln Val Asp Ala Glu Ala Gln Lys
    1280                1285                1290

Glu Leu Gln Arg Lys Val
    1295

<210> 277
<211> 27
<212> DNA
<213> Artificial Sequence

<220>
<223> a primer sequence for PCR

<400> 277
gcgcggtacc attgagacaa catggat 27

<210> 278
<211> 26
<212> DNA
<213> Artificial Sequence

<220>
<223> a primer sequence for PCR

<400>  278
atttaaatag ttagacaata gtatca                                            26


<210>  279
<211>  35
<212>  DNA
<213>  Artificial Sequence

<220>
<223>  a primer sequence for PCR

<400>  279
gcgcggggta ccatgttgtc ttagcatttt catta                                  35


<210>  280
<211>  40
<212>  DNA
<213>  Artificial Sequence

<220>
<223>  a primer sequence for PCR

<400>  280
accacgctaa agatggggga ctttgcatac ccagcttcca                             40


<210>  281
<211>  40
<212>  DNA
<213>  Artificial Sequence

<220>
<223>  a primer sequence for PCR

<400>  281
tggaagctgg gtatgcaaag tcccccatct ttagcgtggt                             40


<210>  282
<211>  40
<212>  DNA
<213>  Artificial Sequence

<220>
<223>  a primer sequence for PCR

<400>  282
ggtacctctg ccatgaagtc ctcgatcatt gaccgataat                             40


<210>  283
<211>  40
<212>  DNA
<213>  Artificial Sequence

<220>
<223>  a primer sequence for PCR

<400>  283
attatcggtc aatgatcgag gacttcatgg cagaggtacc                             40

<210>  284
<211>  40
<212>  DNA
<213>  Artificial Sequence

<220>
<223>  a primer sequence for PCR

<400>  284
gagaggtgct agcagttgtt ctagttcgcg agtgatgaca                          40


<210>  285
<211>  40
<212>  DNA
<213>  Artificial Sequence

<220>
<223>  a primer sequence for PCR

<400>  285
tgtcatcact cgcgaactag aacaactgct agcacctctc                          40


<210>  286
<211>  40
<212>  DNA
<213>  Artificial Sequence

<220>
<223>  a primer sequence for PCR

<400>  286
cagtgctacc tcatgccagt ctggtgagtc cgtccaagta                          40


<210>  287
<211>  40
<212>  DNA
<213>  Artificial Sequence

<220>
<223>  a primer sequence for PCR

<400>  287
tacttggacg gactcaccag actggcatga ggtagcactg                          40


<210>  288
<211>  40
<212>  DNA
<213>  Artificial Sequence

<220>
<223>  a primer sequence for PCR

<400>  288
agtaaagcgc gaaaatgctc cggttgtgac tggatgcaac                          40


<210>  289
<211>  40
<212>  DNA
<213>  Artificial Sequence

<220>
<223>  a primer sequence for PCR

<400>  289
gttgcatcca gtcacaaccg gagcattttc gcgctttact                              40


<210>  290
<211>  20
<212>  DNA
<213>  Artificial Sequence

<220>
<223>  a primer sequence for PCR

<400>  290
attttcccta gatcacttct                                                    20


<210>  291
<211>  40
<212>  DNA
<213>  Artificial Sequence

<220>
<223>  a primer sequence for PCR

<400>  291
ccgaattcga gctcggtacc ttgtcttagc attttcatta                              40


<210>  292
<211>  38
<212>  DNA
<213>  Artificial Sequence

<220>
<223>  a primer sequence for PCR

<400>  292
ctactacaga tccccgggtc gtaattttcc ctagatca                                38


<210>  293
<211>  26
<212>  DNA
<213>  Artificial Sequence

<220>
<223>  a primer sequence for PCR

<400>  293
atttaaatgt cgtacatatg ctatgt                                             26


<210>  294
<211>  26
<212>  DNA
<213>  Artificial Sequence

<220>
<223>  a primer sequence for PCR

<400>  294
gcggtaccac aactcaactc aatagg                                             26

<210>  295
<211>  40
<212>  DNA
<213>  Artificial Sequence

<220>
<223>  a primer sequence for PCR

<400>  295
ccgaattcga gctcggtacc tcgctattgt cagttacaca                              40


<210>  296
<211>  38
<212>  DNA
<213>  Artificial Sequence

<220>
<223>  a primer sequence for PCR

<400>  296
ctactacaga tccccgggga acaatcccga cacatgaa                                38

## Description

### [CROSS-REFERENCE TO RELATED APPLICATION]

This patent application claims priority to Japanese Patent Application No.14727/2010 that was filed on January 26, 2010 and Japanese Patent Application No.14336/2010 that was filed on January 26, 2010, and the entire disclosures of all are incorporated herein by reference.

### [BACKGROUND OF THE INVENTION]

### Field of Invention

The present invention relates to a method for producing a pyripyropene derivative which is useful as a pest control agent. More specifically, it relates to a method for producing a pyripyropene derivative which has acyloxy groups at the 1 position and 11 position, and which has a hydroxyl group at the 7-position.

### Background Art

A pyripyropene derivative which has acyloxy groups at the 1 position and 11 position, and which has a hydroxyl group at the 7-position is a compound exerting a control effect against insect pests, as described in WO2006/129714 (Patent Document 1) and WO2008/066153 (Patent Document 2).

As a method for producing the pyripyropene derivative which has acyloxy groups at the 1 position and 11 position, and which has a hydroxyl group at the 7-position, a method of purification and isolation thereof from a plurality of products generated by nonselective hydrolysis of acyloxy groups using a 1,7,11-triacyloxy derivative as a raw material has been disclosed in WO2006/129714 and Japanese Patent Laid-Open Publication No. 259569/1996 (Patent Document 3).

Also, Japanese Patent Laid-Open Publication No. 259569/1996 described use of protective groups in combination for synthesis of pyripyropene derivatives. Journal of Antibiotics Vol. 49, No. 11, p. 1149, 1996 (Non-patent Document 1) and Bioorganic Medicinal Chemistry Letter Vol. 5, No. 22, p. 2683, 1995 (Non-patent Document 2) and Japanese Patent Laid-Open Publication No. 269065/1996 (Patent Document 4) have disclosed synthesis examples wherein an acyl group was introduced into the 7-position using a protective group.

WO2009/022702 (Patent Document 5) has disclosed a method for producing 1,11-diacyl-1,7,11-trideacetyl pyripyropene A from 1,7,11-trideacetyl pyripyropene A using a protective group.

Since hitherto known production of a pyripyropene derivative which has acyloxy groups at the 1 position and 11 position, and which has a hydroxyl group at the 7-position is a production method using nonselective hydrolysis of a 1,7,11-triacyloxy derivative or using protective groups in multiple steps, in industrial production, further improvement of production efficiency such as reduction of production cost, improvement of yield, facilitation of purification and isolation or shortening of the number of the steps has been desired.

### [PRIOR ART REFERENCES]

### [PATENT DOCUMENTS]

[Patent Document 1] WO2006/129714
[Patent Document 2] WO2008/066153
[Patent Document 3] Japanese Patent Laid-Open Publication No. 259569/1996
[Patent Document 4] Japanese Patent Laid-Open Publication No. 269065/1996
[Patent Document 5] WO2009/022702

### [NON-PATENT DOCUMENTS]

[Non-patent Document 1] Journal of Antibiotics Vol. 49, No. 11, p. 1149, 1996
[Non-patent Document 2] Bioorganic Medicinal Chemistry Letter Vol. 5, No. 22, p. 2683, 1995

### [SUMMARY OF THE INVENTION]

The present inventors have found a method for obtaining a desired 1,11-diacyloxy derivative under simpler conditions and in shorter steps using a pyripyropene analog, which is obtained as a naturally-occurring product (Journal of Antibiotics (1996) 49(3), 292-298, Pure Appl. Chem., vol. 71, No6, pp. 1059-1064, 1999.; WO94/09147; Japanese Patent Laid-Open Publication No. 239385/1996; Japanese Patent Laid-Open Publication No. 259569/1996 (Patent Document 3); Bioorganic Medicinal Chemistry Letter Vol. 5, No. 22, p. 2683, 1995 (Non-patent Document 2); and WO2004/060065) as a synthetic raw material by an enzyme method by a microorganism or by a combination of an enzyme method and chemical conversion.

Thus, an object of the present invention is to provide a method for producing a 1,11-diacyloxy derivative using an enzyme method by a microorganism.

According to one embodiment of the present invention, there is provided a method for producing a compound represented by the following formula A: 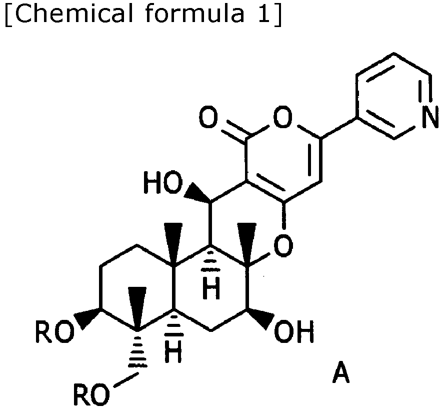 [wherein R represents a linear, branched or cyclic C₂₋₆ alkylcarbonyl group (when the alkyl moiety of this group is branched or cyclic, C₃₋₆ alkyl carbonyl group)],
the method comprising a step of culturing a microorganism into which at least one polynucleotide of (I) to (III) below or a recombinant vector comprising it/them is introduced with a compound represented by the following formula B: 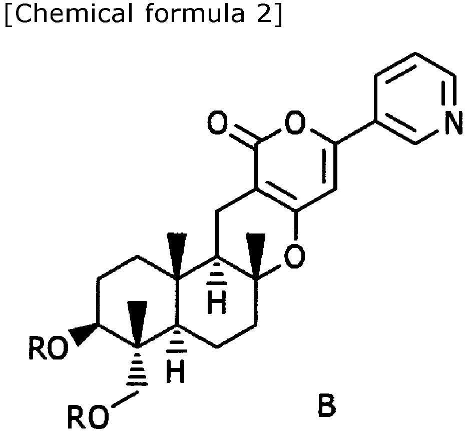 [wherein R represents the same meanings as above],
and isolating the compound represented by formula A:
(I) an isolated polynucleotide having at least one nucleotide sequence selected from the nucleotide sequences of the following (a) to (d):
   (a) a nucleotide sequence of SEQ ID NO:266,
   (b) a nucleotide sequence which is capable of hybridizing with a sequence complementary to the nucleotide sequence of SEQ ID NO:266 under stringent conditions, and which encodes a protein substantially equivalent to the protein encoded by the nucleotide sequence of SEQ ID NO:266,
   (c) a nucleotide sequence of SEQ ID NO:266 in which one or more nucleotides are deleted, substituted, inserted or added, and which encodes a protein substantially equivalent to the protein encoded by the each nucleotide sequence, and
   (d) a nucleotide sequence which has at least 90% identity to the polynucleotide of the nucleotide sequence of SEQ ID NO:266, and which encodes a protein substantially equivalent to the protein encoded by the nucleotide sequence of SEQ ID NO:266;
(II) an isolated polynucleotide having a nucleotide sequence which encodes at least one amino acid sequence selected from SEQ ID NOs:269 and 270 or amino acid sequence substantially equivalent thereto; and
(III) an isolated polynucleotide having at least one nucleotide sequence selected from the nucleotide sequences of the following (1) to (4):
   (1) a nucleotide sequence of the following (a) and (b):
      (a) a nucleotide sequence from 13266 to 15144 of the nucleotide sequence shown in SEQ ID NO:266, and
      (b) a nucleotide sequence from 16220 to 18018 of the nucleotide sequence shown in SEQ ID NO:266,
   (2) a nucleotide sequence which is capable of hybridizing with a sequence complementary to the nucleotide sequence of (1) under stringent conditions, and which encodes a protein substantially equivalent to the protein encoded by the each nucleotide sequence,
   (3) a nucleotide sequence of (1) in which one or more nucleotides are deleted, substituted, inserted or added, and which encodes a protein substantially equivalent to the protein encoded by the each nucleotide sequence, and
   (4) a nucleotide sequence which has at least 90% identity to the polynucleotide of the nucleotide sequence of (1), and which encodes a protein substantially equivalent to the protein encoded by the each nucleotide sequence.

According to another embodiment of the present invention, there is provided the production method comprising a step of culturing a microorganism which comprises plasmid pCC1-PP1, plasmid pPP2 or plasmid pPP3, or one or more vectors selected from the group consisting of these plasmids with a compound represented by the above formula B and isolating the compound represented by the above formula A.

According to a preferred embodiment of the present invention, there is provided a method for producing a compound represented by the above formula A, the method comprising a step of culturing a microorganism into which at least one polynucleotide of (IV) to (V) below or a recombinant vector comprising it/them is introduced with a compound represented by the above formula B and isolating the compound represented by the above formula A:
(IV) an isolated polynucleotide having a nucleotide sequence which encodes the amino acid sequence of SEQ ID NO:270 or an amino acid sequence substantially equivalent thereto; and
(V) an isolated polynucleotide having at least one nucleotide sequence selected from the nucleotide sequences of the following (1) to (4):
   (1) a nucleotide sequence of the following (a):
      (a) a nucleotide sequence from 16220 to 18018 of the nucleotide sequence shown in SEQ ID NO:266,
   (2) a nucleotide sequence which is capable of hybridizing with a sequence complementary to the nucleotide sequence of (1) under stringent conditions, and which encodes a protein substantially equivalent to the protein encoded by the each nucleotide sequence,
   (3) a nucleotide sequence of (1) in which one or more nucleotides are deleted, substituted, inserted or added, which encodes a protein substantially equivalent to the protein encoded by the each nucleotide sequence, and
   (4) a nucleotide sequence which has at least 90% identity to the polynucleotide of the nucleotide sequence of (1), and which encodes a protein substantially equivalent to the protein encoded by the each nucleotide sequence.

According to another embodiment of the present invention, there is provided the production method further comprising a step of acylating the hydroxyl groups at the 1 and 11 positions of a compound represented by the following formula D using an acylating agent: 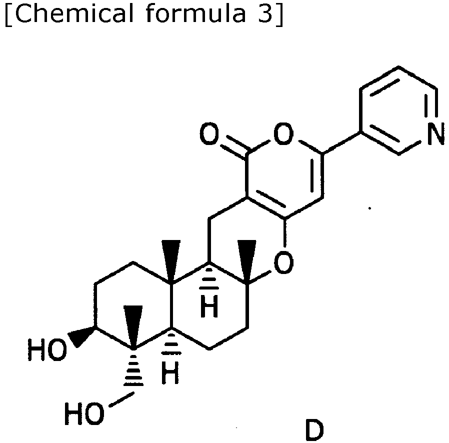 and isolating a compound represented by the above formula B.

According to another embodiment of the present invention, there is provided the production method further comprising a step of hydrolyzing the acetyl group at the 1 position and, when R' is an acetyl group, the acetyl group at the 11 position of a compound represented by the following formula C: 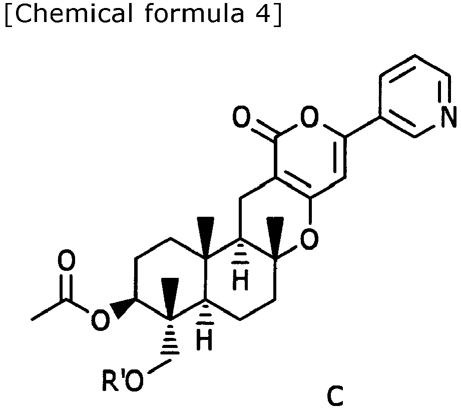 [wherein R' represents an acetyl group or a hydrogen atom]
and isolating a compound represented by the above formula D.

According to another embodiment of the present invention, there is provided the production method further comprising a step of culturing a microorganism into which at least one polynucleotide of (VI) to (VII) below or a recombinant vector comprising it/them is introduced with pyripyropene E and isolating a compound represented by the above formula C:
(VI) an isolated polynucleotide having a nucleotide sequence which encodes the amino acid sequence of SEQ ID NOs:269 and 275 or an amino acid sequence substantially equivalent thereto; and
(VII) an isolated polynucleotide having at least one nucleotide sequence selected from the nucleotide sequences of the following (1) to (4):
   (1) a nucleotide sequence of the following (a) and (b):
      (a) a nucleotide sequence from 13266 to 15144 of the nucleotide sequence shown in SEQ ID NO:266, and
      (b) a nucleotide sequence from 25824 to 27178 of the nucleotide sequence shown in SEQ ID NO:266,
   (2) a nucleotide sequence which is capable of hybridizing with a sequence complementary to the nucleotide sequence of (1) under stringent conditions, and which encodes a protein substantially equivalent to the protein encoded by the each nucleotide sequence,
   (3) a nucleotide sequence of (1) in which one or more nucleotides are deleted, substituted, inserted or added, which encodes a protein substantially equivalent to the protein encoded by the each nucleotide sequence, and
   (4) a nucleotide sequence which has at least 90% identity to the polynucleotide of the nucleotide sequence of (1), and which encodes a protein substantially equivalent to the protein encoded by the each nucleotide sequence.

According to another embodiment of the present invention, there is provided the production method comprising a step of culturing a microorganism which comprises plasmid pPP2 or plasmid pPP9 with pyripyropene E and isolating a compound represented by the above formula C.

According to another embodiment of the present invention, there is provided the production method further comprising a step of culturing a microorganism into which at least one polynucleotide of (VIII) to (IX) below or a recombinant vector comprising it/them is introduced with deacetyl pyripyropene E and isolating a compound represented by the above formula C:
(VIII) an isolated polynucleotide having a nucleotide sequence which encodes at least one amino acid sequence selected from SEQ ID NOs:269, 274 and 275 or amino acid sequence substantially equivalent thereto; and
(IX) an isolated polynucleotide having at least one nucleotide sequence selected from the nucleotide sequences of the following (1) to (4):
   (1) a nucleotide sequence of the following (a), (b) and (c):
      (a) a nucleotide sequence from 13266 to 15144 of the nucleotide sequence shown in SEQ ID NO:266,
      (b) a nucleotide sequence from 23205 to 24773 of the nucleotide sequence shown in SEQ ID NO:266, and
      (c) a nucleotide sequence from 25824 to 27178 of the nucleotide sequence shown in SEQ ID NO:266,
   (2) a nucleotide sequence which is capable of hybridizing with a sequence complementary to the nucleotide sequence of (1) under stringent conditions, and which encodes a protein substantially equivalent to the protein encoded by the each nucleotide sequence,
   (3) a nucleotide sequence of (1) in which one or more nucleotides are deleted, substituted, inserted or added, which encodes a protein substantially equivalent to the protein encoded by the each nucleotide sequence, and
   (4) a nucleotide sequence which has at least 90% identity to the polynucleotide of the nucleotide sequence of (1), and which encodes a protein substantially equivalent to the protein encoded by the each nucleotide sequence.

According to another embodiment of the present invention, there is provided the production method comprising a step of culturing a microorganism which comprises plasmid pPP2 or plasmid pPP9 and also comprises plasmid pPP7 with deacetyl pyripyropene E and isolating a compound represented by the above formula C.

According to another embodiment of the present invention, there is provided use of plasmid pCC1-PP1 (Accession No. FERM BP-11133 of Escherichia coli EPI300^{™}-T1^{R} transformed with plasmid pCC1-PP1), plasmid pPP2 (Accession No. FERM BP-11137 of Aspergillus oryzae transformed with plasmid pPP2), plasmid pPP3 (Accession No. FERM BP-11141 of Aspergillus oryzae transformed with plasmid pPP3), plasmid pPP7 (Accession No. FERM BP-11219 of Aspergillus oryzae transformed with plasmid pPP7), or plasmid pPP9 (Accession No. FERM BP-11220 of Aspergillus oryzae transformed with plasmid pPP9), or one or more vectors selected from the group consisting of these plasmids, for producing a compound represented by formula A.

According to a preferred embodiment of the present invention, there is provided use of plasmid pPP3 (Accession No. FERM BP-11141 of Aspergillus oryzae transformed with plasmid pPP3) or a vector comprising this plasmid, for producing a compound represented by formula A.

According to another embodiment of the present invention, there is provided use of a transformant comprising plasmid pCC1-PP1 (Accession No. FERM BP-11133 of Escherichia coli EPI300^{™}-T1^{R} transformed with plasmid pCC1-PP1), plasmid pPP2 (Accession No. FERM BP-11137 of Aspergillus oryzae transformed with plasmid pPP2), plasmid pPP3 (Accession No. FERM BP-11141 of Aspergillus oryzae transformed with plasmid pPP3), plasmid pPP7 (Accession No. FERM BP-11219 of Aspergillus oryzae transformed with plasmid pPP7), or plasmid pPP9 (Accession No. FERM BP-11220 of Aspergillus oryzae transformed with plasmid pPP9), or a vector selected from the group consisting of these plasmids, for producing a compound represented by formula A.

According to a preferred embodiment of the present invention, there is provided use of a transformant comprising plasmid pPP2 (Accession No. FERM BP-11137 of Aspergillus oryzae transformed with plasmid pPP2), plasmid pPP3 (Accession No. FERM BP-11141 of Aspergillus oryzae transformed with plasmid pPP3), plasmid pPP7 (Accession No. FERM BP-11219 of Aspergillus oryzae transformed with plasmid pPP7), or plasmid pPP9 (Accession No. FERM BP-11220 of Aspergillus oryzae transformed with plasmid pPP9), or a vector comprising one of these plasmids, for producing a compound represented by formula A.

According to another embodiment of the present invention, there is provided a compound represented by the following formula B1:

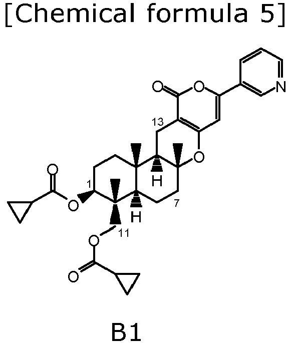

According to another embodiment of the present invention, there is provided a compound represented by the above formula D.

According to another embodiment of the present invention, there is provided a method for producing a compound represented by the above formula A from a compound represented by the above formula B, the method comprising a step of using a protein comprising the amino acid sequence described in SEQ ID NO:270, instead of a microorganism.

The present invention allows for production of a pyripyropene derivative which is useful as a pest control agent and has acyloxy groups at the 1 and 11 positions and a hydroxyl group at the 7 position under simpler conditions and in shorter steps.

### [BRIEF DESCRIPTION OF DRAWINGS]

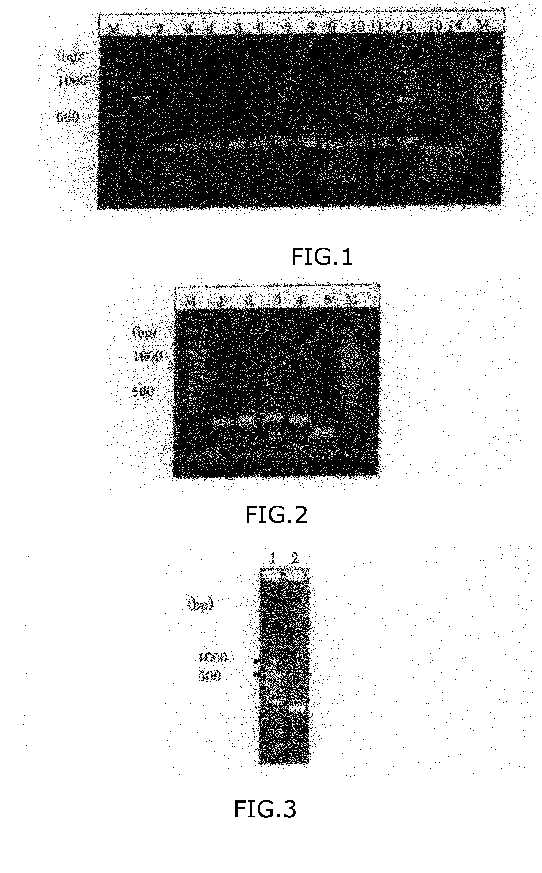

[Figure 1] Figure 1 shows an electrophoresis pattern of PCR products by agarose gel. For the electrophoresis, the PCR products amplified using the following primers were used: M: molecular weight marker (100 bp ladder), lane 1: primers of SEQ ID NOs:1 and 2, lane 2: primers of SEQ ID NOs:239 and 240, lane 3: primers of SEQ ID NOs:237 and 238, lane 4: primers of SEQ ID NOs:241 and 242, lane 5: primers of SEQ ID NOs:247 and 248, lane 6: primers of SEQ ID NOs:251 and 252, lane 7: primers of SEQ ID NOs:245 and 246, lane 8: primers of SEQ ID NOs:243 and 244, lane 9: primers of SEQ ID NOs:249 and 250, lane 10: primers of SEQ ID NOs:235 and 236, lane 11: primers of SEQ ID NOs:233 and 234, lane 12: primers of SEQ ID NOs:227 and 228, lane 13: primers of SEQ ID NOs:229 and 230, lane 14: primers of SEQ ID NOs:231 and 232.
[Figure 2] Similarly to Figure 1, Figure 2 shows an electrophoresis pattern of PCR products by agarose gel. For the electrophoresis, the PCR products amplified using the following primers were used: M: molecular weight marker (100 bp ladder), lane 1: primers of SEQ ID NOs:253 and 254, lane 2: primers of SEQ ID NOs:257 and 258, lane 3: primers of SEQ ID NOs:259 and 260, lane 4: primers of SEQ ID NOs:255 and 256, lane 5: primers of SEQ ID NOs:261 and 262.
[Figure 3] Similarly to Figure 1, Figure 3 shows an electrophoresis pattern of PCR products by agarose gel. For the electrophoresis, the PCR products amplified using the following primers were used: lane 1: molecular weight marker (100 bp ladder), lane 2: primers of SEQ ID NOs:264 and 265 (400 bp amplified fragment).
[Figure 4] Figure 4 shows the plasmid map of pUSA.
[Figure 5] Figure 5 shows the plasmid map of pPP2.
[Figure 6] Figure 6 shows a scheme of P450-2 cDNA amplification.
[Figure 7] Figure 7 shows the plasmid map of pPP3.
[Figure 8] Figure 8 shows ¹H-NMR spectrum of pyripyropene E in deuterated acetonitrile.
[Figure 9] Figure 9 shows ¹H-NMR spectrum in deuterated acetonitrile of a product of the culture of Aspergillus oryzae transformed with plasmid pPP2.
[Figure 10] Figure 10 shows ¹H-NMR spectrum of pyripyropene O in deuterated acetonitrile.
[Figure 11] Figure 11 shows ¹H-NMR spectrum in deuterated acetonitrile of a product of the culture of Aspergillus oryzae transformed with plasmid pPP3.
[Figure 12] Figure 12 shows the plasmid map of plasmids pPP7 and pPP9.

### [DETAILED DESCRIPTION OF THE INVENTION]

### Deposition of Microorganisms

Escherichia coli (Escherichia coli EPI300^{™}-T1^{R}) transformed with plasmid pCC1-PP1 has been deposited with International Patent Organism Depositary, National Institute of Advanced Industrial Science and Technology (Address: AIST Tsukuba Central 6, 1-1-1 Higashi, Tsukuba, Ibaraki, Japan, 305-8566), under accession No. FERM BP-11133 (converted from domestic deposition under accession No. FERM P-21704) (identification reference by the depositors: Escherichia coli EPI300^{™}-T1^{R}/pCC1-PP1) as of October 9, 2008 (original deposition date).

Aspergillus oryzae transformed with plasmid pPP2 has been deposited with International Patent Organism Depositary, National Institute of Advanced Industrial Science and Technology (Address: AIST Tsukuba Central 6, 1-1-1 Higashi, Tsukuba, Ibaraki, Japan, 305-8566), under accession No. FERM BP-11137 (identification reference by the depositors: Aspergillus oryzae PP2-1) as of June 23, 2009.

Aspergillus oryzae transformed with plasmid pPP3 has been deposited with International Patent Organism Depositary, National Institute of Advanced Industrial Science and Technology (Address: AIST Tsukuba Central 6, 1-1-1 Higashi, Tsukuba, Ibaraki, Japan, 305-8566), under accession No. FERM BP-11141 (identification reference by the depositors: Aspergillus oryzae PP3-2) as of July 3, 2009.

Aspergillus oryzae transformed with plasmid pPP7 has been deposited with International Patent Organism Depositary, National Institute of Advanced Industrial Science and Technology (Address: AIST Tsukuba Central 6, 1-1-1 Higashi, Tsukuba, Ibaraki, Japan, 305-8566), under accession No. FERM BP-11219 (identification reference by the depositors: Aspergillus oryzae PP7) as of December 21, 2009.

Aspergillus oryzae transformed with plasmid pPP9 has been deposited with International Patent Organism Depositary, National Institute of Advanced Industrial Science and Technology (Address: AIST Tsukuba Central 6, 1-1-1 Higashi, Tsukuba, Ibaraki, Japan, 305-8566), under accession No. FERM BP-11220 (identification reference by the depositors: Aspergillus oryzae PP9) as of December 21, 2009.

The microorganism used in the present invention may be introduced with a polynucleotide using the recombinant vector described below. However, the polynucleotide may be introduced into the microorganism, for example, by an electroporation method, a polyethylene glycol method, an Agrobacterium method, a lithium method, a calcium chloride method or the like.

The microorganism used in the present invention is not particularly restricted as long as it can be introduced with a polynucleotide or a recombinant vector comprising it/them. Microorganisms belonging to the genus Aspergillus are preferred and more preferred microorganism includes Aspergillus oryzae.

In the present invention, culturing microorganisms can be carried out, for example, by solid culturing under aerobic conditions, shake culturing, culturing with bubbling under stirring or deep part aerobic culturing, in particular, deep part aerobic culturing is preferred. As a medium for culturing microorganisms, commonly used components, for example, as carbon sources, glucose, sucrose, starch syrup, dextrin, starch, glycerol, molasses, animal and vegetable oils or the like, can be used. Also, as nitrogen sources, soybean flour, wheat germ, corn steep liquor, cotton seed meal, meat extract, polypeptone, malto extract, yeast extract, ammonium sulfate, sodium nitrate, urea or the like can be used. Besides, as required, addition of sodium, potassium, calcium, magnesium, cobalt, chlorine, phosphoric acid (dipotassium hydrogen phosphate or the like), sulfuric acid (magnesium sulfate or the like) or inorganic salts which can generate other ions is effective. Also, as required, various vitamins such as thiamin (thiamine hydrochloride or the like), amino acids such as glutamic acid (sodium glutamate or the like) or asparagine (DL-asparagine or the like), trace nutrients such as nucleotides or selection agents such as antibiotics can be added. Further, organic substances or inorganic substances which help the growth of a bacterium and promote the production of the compound represented by formula A can be appropriately added.

The pH of the medium is, for example, about pH 5.5 to pH 8. The appropriate temperature for the culturing is 15°C to 40°C and, in many cases, the growth takes place around 22°C to 30°C. The production of the compound represented by formula A varies depending on the medium and culturing conditions, or the used host. In any method for culturing, the accumulation usually reaches a peak in 2 days to 10 days. The culturing is terminated at the time when the amount of the compound represented by formula A in the culture reaches the peak and a desired substance is isolated and purified from the culture.

To isolate the compound represented by formula A from the culture, it can be extracted and purified by a usual separation means using properties thereof, such as a solvent extraction method, an ion exchange resin method, an adsorption or distribution column chromatography method, a gel filtration method, dialysis, a precipitation method, which may be individually used or appropriately used in combination. The solvent extraction method is preferred.

In the present invention, the term "substantially equivalent amino acid sequence" means an amino acid sequence which does not affect an activity of a polypeptide despite the fact that one or more amino acids are altered by substitution, deletion, addition, or insertion. Preferably, an amino acid sequence which is altered by amino acid substitution, deletion, addition, or insertion has a sequence identity of 70% or more, preferably 80% or more, more preferably 90% or more, still more preferably 95% or more, and still more preferably 98% or more to the amino acid sequence before alteration and the like. Further, the number of the altered amino acid residues is preferably 1 to 40, more preferably 1 to 20, still more preferably 1 to 10, still more preferably 1 to 8, and most preferably 1 to 4.

Further, an example of the alteration which does not affect the activity includes conservative substitution. The term "conservative substitution" means substitution of preferably 1 to 40, more preferably 1 to 20, more preferably 1 to 10, still more preferably 1 to 8, and most preferably 1 to 4 amino acid residues with other chemically similar amino acid residues such that the activity of the polypeptide is not substantially altered. Examples thereof include cases where a certain hydrophobic amino acid residue is substituted with another hydrophobic amino acid residue and cases where a certain polar amino acid residue is substituted with another polar amino acid residue having the same charges. Functionally similar amino acids capable of such a substitution are known in the art for each amino acid. Concretely, examples of non-polar (hydrophobic) amino acids include alanine, valine, isoleucine, leucine, proline, tryptophan, phenylalanine, methionine and the like. Examples of polar (neutral) amino acids include glycine, serine, threonine, tyrosine, glutamine, asparagine, cysteine and the like. Examples of positively charged (basic) amino acids include arginine, histidine, lysine and the like. Examples of negatively charged (acidic) amino acids include aspartic acid, glutamic acid and the like.

The term, "stringent conditions" in the present invention means conditions where a washing operation of membranes after hybridization is carried out at high temperatures in a solution with low salt concentrations, a person skilled in the art would be able to appropriately determine the condition, for example, the condition includes the condition of washing in a solution with 2xSSC (1×SSC: 15 mM trisodium citrate, 150 mM sodium chloride) and 0.5% SDS at 60°C for 20 minutes, and the condition of washing in a solution with 0.2xSSC (1×SSC: 15 mM trisodium citrate, 150 mM sodium chloride) and 0.1% SDS at 60°C for 15 minutes.

Hybridization can be carried out in accordance with a known method. Also, when a commercially-available library is used, it can be carried out in accordance with a method in the attached instructions.

In the present description, the term "identity" (also referred to as homology) for nucleotide sequences means a degree of match of bases constituting each sequence among the sequences to be compared. At that time, the presence of a gap(s) and characteristics of the amino acids are taken into account. Any values of the "identity" shown in the present description may be values calculated using a homology search program known to those skilled in the art. For instance, the value can be readily calculated by using default (initial setting) parameters in FASTA, BLAST or the like.

In the present description, the "identity" for nucleotide sequences is 90% or more, preferably 95% or more, more preferably 98% or more, still more preferably 99% or more.

In the present description, the term, "one or more nucleotides are deleted, substituted, inserted or added in a polynucleotide" means that alteration was made by a known method such as a site specific mutagenesis method, or substitution or the like of a plurality nucleotides in a degree at which they may naturally occur. The number of the altered nucleotides is one or several nucleotides (for example, one to several nucleotides or 1, 2, 3 or 4 nucleotides).

The term "nucleotide sequence which encodes a protein substantially equivalent to the protein encoded by the (each) nucleotide sequence" means a nucleotide sequence encoding a protein which has an activity equivalent to that of "then protein encoded by the (each) nucleotide sequence."

It is preferred that a protein substantially equivalent to a protein encoded by the nucleotide sequence from 13266 to 15144 of the nucleotide sequence shown in SEQ ID NO:266 have Cytochrome P450 monooxygenase (1) (P450-1) activity.

It is preferred that a protein substantially equivalent to a protein encoded by the nucleotide sequence from 16220 to 18018 of the nucleotide sequence shown in SEQ ID NO:266 have Cytochrome P450 monooxygenase (2) (P450-2) activity.

It is preferred that a protein substantially equivalent to a protein encoded by a nucleotide sequence from 23205 to 24773 of a nucleotide sequence shown in SEQ ID NO: 266 have Acetyltransferase (AT) activity.

It is preferred that a protein substantially equivalent to a protein encoded by a nucleotide sequence from 25824 to 27178 of a nucleotide sequence shown in SEQ ID NO: 266 have Acetyltransferase-2 (AT-2) activity.

### Obtainment of Isolated Polynucleotide

The method for obtaining the isolated polynucleotide of the present invention is not particularly restricted. The polynucleotide can be isolated from Penicillium coprobium PF1169 strain (Journal of Technical Disclosure 500997/2008) or filamentous bacterium by the following method. Concretely, based on a homology sequence obtained by the method of Example 9 below or the like, primers capable of specifically amplifying any one or more genes of a polyketide synthase gene, prenyltransferase gene, hydroxylase gene, acetyltransferase gene or adenylate synthetase gene, which are involved in synthesis of pyripyropene, are synthesized. PCR is carried out for a fosmid genomic library of Penicillium coprobium PF1169 strain which is separately prepared and further colony hybridization is carried out, thereby obtaining the isolated polynucleotide used in the present invention.

### Recombinant Vector

The recombinant vector according to the present invention can be prepared by modifying any one or more of the polynucleotides in the above-mentioned (I) to (III) into an appropriate form depending on an object and ligating them to a vector in accordance with a conventional method, for example, gene recombination techniques described in [Sambrook, J. et al., "Molecular cloning: a laboratory manual", (USA), 2nd Edition, Cold Spring Harbor Laboratory, 1989].

The recombinant vector used in the present invention can be appropriately selected from virus, plasmid, fosmid, cosmid vectors or the like. For instance, when a host cell is Escherichia coli, examples thereof include λ phage-based bacteriophage and pBR and pUC-based plasmids. In the case of a Bacillus subtilis, examples include pUB-based plasmids. In the case of yeast, examples include YEp, YRp, YCp and YIp-based plasmids.

It is preferred that at least one plasmid among the used plasmids comprise a selection marker for selecting a transformant. As the selection marker, a gene encoding drug resistance and gene complementing auxotrophy can be used. Concrete preferred examples thereof include, when a host to be used is bacterium, ampicillin resistant genes, kanamycin resistant genes, tetracycline resistant gene and the like; in the case of yeast, tryptophan biosynthetic gene (TRP1), uracil biosynthetic gene (URA3), leucine biosynthetic gene (LEU2) and the like; in the case of a fungus, hygromycin resistant genes, bialaphos resistant genes, bleomycin resistant genes, aureobasidin resistant genes and the like; and in the case of a plant, kanamycin resistant genes, bialaphos resistant genes and the like.

In addition, DNA molecules serving as an expression vector used in the present invention preferably has DNA sequences necessary to express each gene, for example, transcription regulatory signals and translation regulatory signals such as promoters, transcription initiation signals, liposome binding sites, translation stop signals, terminators. Preferred examples of the promoters include promoters of lactose operon, tryptophan operon and the like in Escherichia coli; promoters of alcohol dehydrogenase gene, acid phosphatase gene, galactose metabolizing gene, glyceraldehyde 3-phosphate dehydrogenase gene or the like in yeast; promoters of a-amylase gene, glucoamylase gene, cellobiohydrolase gene, glyceraldehyde 3-phosphate dehydrogenase gene, abp1 gene or the like in fungi; a CaMV 35S RNA promoter, a CaMV 19S RNA promoter or a nopaline synthetase gene promoter in plants.

### Transformant

A host in which the isolated polynucleotide according to the present invention is introduced may be appropriately selected, depending on the type of the used vector, from actinomycetes, Escherichia coli, Bacillus subtilis, yeast, filamentous fungus, plant cells or the like.

A method of introducing a recombinant vector into a host may be selected, depending on a host cell under test, from conjugal transfer, transduction by phage, as well as methods of transformation such as a calcium ion method, a lithium ion method, an electroporation method, a PEG method, an Agrobacterium method or a particle gun method.

When a plurality of genes is introduced into host cells in the present invention, the genes may be comprised in a single DNA molecule or individually in different DNA molecules. Further, when a host cell is a bacterium, each gene can be designed so as to be expressed as polycistronic mRNA and made into one DNA molecule.

### Production method

In the present description, the term "alkyl" as a substituent group or part thereof individually means a linear, branched, cyclic alkyl or a combination thereof unless otherwise defined.

In the present description, the symbol "C_{a-b}" affixed to a substituent group means the number of the carbon atoms comprised in the substituent group is from a to b. In the case of "C_{a-b} alkyl carbonyl", the symbol "C_{a-b}" means the number of the carbon atoms comprised in the alkyl moiety with the carbon atoms of the carbonyl moiety being excluded is from a to b.

Concrete examples of the linear, branched, cyclic C₂₋₆ alkyl carbonyl group (C₃₋₆ alkyl carbonyl group when an alkyl moiety of this group is branched or cyclic) represented by R include a cyclopropane carbonyl group, a propionyl group and the like.

Pyripyropene E can be produced, for example, by a method for culturing microorganisms based on the method described in Japanese Patent Laid-Open Publication No. 239385/1996, WO94/09147 or US Patent No. 5597835; or the total synthesis method described in Tetrahedron Letters, vol. 37, No. 36, 6461-6464, 1996.

### 1. Production of Compound Represented by formula C

Among the compounds represented by formula C, pyripyropene O wherein R' is an acetyl group can be obtained, for example, by a method for culturing microorganisms based on the method described in Journal of Antibiotics (1996) 49(3), 292-298 or WO94/09147.

Among the compounds represented by formula C, 11-deacetylpyripyropene O wherein R' is a hydrogen atom can be synthesized, for example, by the method described in Reference Example 1 below.

According to a preferred embodiment of the present invention, it is preferred to use a microorganism to produce a compound represented by the above formula C. When using a microorganism, one can use the culture medium itself, or one can use a microbial cell suspension obtained by isolating microbial cells from the culture medium and washing them, followed by suspending the obtained living microbial cells in water, physiological saline, or appropriate buffer at a prescribed concentration. The compound can be obtained by adding deacetyl pyripyropene E or pyripyropene E to a suspension of the microorganism used or the microbial cell suspension thereof and allowing the resultant to react in the presence of an enzyme at the optimum temperature and pH for an appropriate period of time. The microorganism can be cultured as described above. A preferred concentration of deacetyl pyripyropene E or pyripyropene E to be added ranges from 1µg/mL to 50,000µg/mL.

When adding pyripyropene E, a protein having a function of hydroxylation (hereinafter referred to as hydroxylase) can be used instead of a microorganism. When using the hydroxylase, the compound can be obtained by dissolving pyripyropene E in a solution of the hydroxylase in water or appropriate buffer and allowing the resultant to react in the presence of an enzyme at the optimum temperature and pH for an appropriate period of time.

When adding deacetyl pyripyropene E, a protein having a function of acetylation (hereinafter referred to as acetylase) and hydroxylase can be used instead of a microorganism. When using acetylase and hydroxylase, the compound can be obtained by dissolving deacetyl pyripyropene E in a solution of acetylase and hydroxylase in water or appropriate buffer and allowing the resultant to react in the presence of an enzyme at the optimum temperature and pH for an appropriate period of time.

Both when adding pyripyropene E and when adding deacetyl pyripyropene E, preferred reaction conditions are a temperature from 10°C to 40°C, a pH from 5 to 9, and a reaction time from 30 minutes to 24 hours. More preferred conditions are a temperature from 20°C to 35°C, a pH from 6 to 8, and a reaction time from an hour to 12 hours.

As the acetylase, a purified acetylase may be used, or a crude enzyme solution obtained by disrupting bacteria, actinomycetes, yeast or fungi containing acetylase can be used. Further, a crude enzyme solution in the form of a solution of disrupted fungi and the like can also be used. As the acetylase, preferably, an acetylase derived from a microorganism having the ability to produce pyripyropene, such as, for example, Aspergillus fumigatus strain FO-1289 (Japanese Patent Laid-Open Publication No. 360895/1992), Eupenicillium reticulosporum strain NRRL-3446 (Applied and Environmental Microbiology (1995), 61(12), 4429-35), Penicillium griseofulvum strain F1959 (WO2004/060065) and Penicillium coprobium strain PF1169 (Journal of Technical Disclosure 500997/2008) can be used.

As the hydroxylase, a purified hydroxylase may be used, or a crude enzyme solution obtained by disrupting bacteria, actinomycetes, yeast or fungi containing hydroxylase can be used. Further, a crude enzyme solution in the form of a solution of disrupted fungi and the like can also be used. As the hydroxylase, preferably, a hydroxylase derived from a microorganism having the ability to produce pyripyropene, such as, for example, Aspergillus fumigatus strain FO-1289 (Japanese Patent Laid-Open Publication No. 360895/1992), Eupenicillium reticulosporum strain NRRL-3446 (Applied and Environmental Microbiology (1995), 61(12), 4429-35), Penicillium griseofulvum strain F1959 (WO2004/060065), and Penicillium coprobium strain PF1169 (Journal of Technical Disclosure 500997/2008) can be used.

A compound represented by the above formula C which is generated by conversion can be isolated as described above.

According to a preferred embodiment of the present invention, one can culture a microorganism into which at least one polynucleotide described of the above (VI) to (VII) or a recombinant vector comprising it/them is introduced with pyripyropene E and isolate a compound represented by the above formula C.

According to another preferred embodiment of the present invention, one can culture a microorganism which comprises plasmid pPP2 or plasmid pPP9 with pyripyropene E and isolate a compound represented by the above formula C.

An isolated compound represented by the above formula C which is obtained by culturing a microorganism which comprises plasmid pPP2 with pyripyropene E is preferably, but not limited to, 11-deacetyl pyripyropene O wherein R' is a hydrogen atom in the above formula C. Further, an isolated compound represented by the above formula C which is obtained by culturing a microorganism which comprises plasmid pPP9 with pyripyropene E is preferably, but not limited to, a compound wherein R' is an acetyl group in the above formula C.

According to another preferred embodiment of the present invention, one can culture a microorganism into which at least one polynucleotide of the above (VIII) to (IX) or a recombinant vector comprising it/them is introduced with deacetyl pyripyropene E and isolate a compound represented by the above formula C.

According to another embodiment of the present invention, one can culture a microorganism which comprises plasmid pPP2 or plasmid pPP9 and also comprises plasmid pPP7 with deacetyl pyripyropene E, and isolate a compound represented by the above formula C.

An isolated compound represented by the above formula C which is obtained by culturing a microorganism which comprises plasmid pPP2 with deacetyl pyripyropene E is preferably, but not limited to, 11-deacetyl pyripyropene O wherein R' is a hydrogen atom in the above formula C. Further, an isolated compound represented by the above formula C which is obtained by culturing a microorganism which comprises plasmid pPP9 with deacetyl pyripyropene E is preferably, but not limited to, a compound wherein R' is an acetyl group in the above formula C.

According to another preferred embodiment of the present invention, one can culture a microorganism into which at least one polynucleotide of the above (VIII) to (IX) or a recombinant vector comprising it/them is introduced with deacetyl pyripyropene E, and isolate a compound represented by the above formula C.

### 2. Method for Producing a Compound Represented by Formula B (Hereinafter Sometimes Referred to as Compound B) from a Compound Represented by Formula C (Hereinafter Referred to as Compound C, Including Pyripyropene O wherein R' is Acetyl Group and 11-Deacetyl Pyripyropene O wherein R' is Hydrogen Atom)

Compound B is obtained by hydrolyzing compound C and then acylating the resulting compound represented by the above formula D (hereinafter sometimes referred to as Compound D).

The hydrolysis of Compound C can be carried out under conditions where an acid or base is used. Concrete examples of the acid which can be used include hydrochloric acid, sulfuric acid, p-toluenesulfonic acid, p-toluenesulfonic acid monohydrate , pyridinium p-toluenesulfonate, 10-camphorsulfonic acid and the like. Concrete examples of the base which can be used include inorganic bases such as sodium carbonate, potassium carbonate, sodium hydrogen carbonate, potassium bicarbonate, sodium hydroxide, potassium hydroxide, sodium hydride, potassium hydride, sodium cyanide, potassium cyanide, magnesium hydroxide, calcium hydroxide, lithium hydroxide or barium hydroxide; alkoxides of alkali metals or alkaline earth metals such as sodium methoxide, sodium ethoxide or tert-butoxypotassium; and organic bases such as triethylamine, diisopropylethylamine, pyridine, hydrazine or guanidine. The hydrolysis can be carried out in an appropriate solvent. Concrete examples of the solvent which can be used include alcohol solvents having 1 to 4 carbon atoms such as methanol; ether solvents such as diethyl ether, diisopropyl ether, tetrahydrofuran or dioxane; aprotic polar organic solvents such as N,N-dimethylformamide, dimethyl sulfoxide, N,N-dimethylacetamide or acetonitrile; halogenated solvents such as dichloromethane or chloroform; or water; and mixtures thereof.

Examples of a solvent which can be used in a method for obtaining Compound B by acylating Compound D (1,11-dideacetyl pyripyropene O) include ether solvents such as diethyl ether, diisopropyl ether, tetrahydrofuran or dioxane; aprotic polar organic solvents such as N,N-dimethylformamide, dimethyl sulfoxide, N,N-dimethylacetamide or acetonitrile; halogenated solvents such as dichloromethane or chloroform; aromatic hydrocarbon solvents such as toluene; and mixtures thereof.

The reaction can be carried out without using a base. However, in the case of using the base, examples of the base which can be used include inorganic bases such as sodium carbonate, potassium carbonate, sodium hydrogen carbonate, potassium bicarbonate, sodium hydroxide, potassium hydroxide, sodium hydride, potassium hydride, sodium cyanide, potassium cyanide, magnesium hydroxide, calcium hydroxide, lithium hydroxide or barium hydroxide; and organic bases such as triethylamine, diisopropylethylamine, pyridine or guanidine.

As an acylation agent corresponding to a desired R, ROH, RCl, (R)₂O or mixed acid anhydride can be used with RCl, (R)₂O being preferred. The reaction can be carried out in the presence or absence of a base, or by using a condensing agent such as dicyclohexylcarbodiimide, 1-ethyl-3-(3-dimethylaminopropyl)carbodiimide hydrochloride, carbonyldiimidazole, dipyridyl disulfide, diimidazolyl disulfide, 1,3,5-trichlorobenzoylchloride, 1,3,5-trichlorobenzoyl anhydride, PyBop or PyBrop; and preferably carried out in the presence or absence of a base by using RCl or (R)₂O. An example of a preferred acylation agent includes cyclopropanecarbonyl chloride.

When the base is used, the amount thereof is preferably 2 to 10 equivalents, more preferably 3 to 6 equivalents, based on Compound D.

The amount of the acylation agent to be used is preferably 2 to 10 equivalents, more preferably 2 to 5 equivalents, based on Compound D. It is preferred that the reaction temperature be within a range of -20°C to 50°C. It is preferred that the reaction time be within a range 0.1 hour to 7 days.

### 3. Production of Compound Represented by formula A (hereinafter, described as Compound A) from Compound B

When the production is carried out using a microorganism, a culture medium per se can be used or a fungal cell suspension obtained by suspending living fungal cells in water, physiological saline or an appropriate buffer at a prescribed concentration, which living fungal cells is obtained by isolating fungal cells from the culture medium and washing them, can also be used. Compound B can be added to the culture medium of the used microorganism or the fungal cell suspension thereof and allowed to react at the optimum temperature and pH of an enzyme for an appropriate period of time, thereby obtaining Compound A. A method for culturing microorganisms can be carried out in accordance with the above. A preferred concentration of the compound B to be added is 1 µg/mL to 50,000 µg/mL.

When the production is carried out by, instead of the microorganism, a protein having a function of hydroxylation (hereinafter, referred to as hydroxylase), Compound B can be dissolved in an enzyme solution obtained by dissolving the hydroxylase in water or an appropriate buffer and allowed to react at the optimum temperature and pH of the enzyme for an appropriate period of time, thereby obtaining Compound A.

As a preferred reaction condition, the temperature is 10°C to 40°C; the pH is pH 5 to pH 9; and the reaction time is 30 minutes to 24 hours. As a more preferred reaction condition, the temperature is 20°C to 35°C; the pH is pH 6 to pH 8; and the reaction time is 1 hour to 12 hours. As the hydroxylase, a purified hydroxylase may be used or a crude enzyme solution obtained by disrupting bacteria, actinomycetes, yeast or fungi containing the hydroxylase can be used. Further, a crude enzyme solution in the form of a solution of disrupted fungi and the like can also be used. As the hydroxylase, preferably hydroxylases derived from microorganisms having ability to produce pyripyropene, Aspergillus fumigatus FO-1289 strain (Japanese Patent Laid-Open Publication No. 360895/1992), Eupenicillium reticulosporum NRRL-3446 strain (Applied and Environmental Microbiology (1995), 61(12), 4429-35), Penicillium griseofulvum F1959 strain (WO2004/060065) and Penicillium coprobium PF1169 strain (Journal of Technical Disclosure 500997/2008) can be used. Compound A generated by conversion can be isolated in accordance with the above.

According to the present invention, there is provided a method for producing a compound represented by the above formula A, the method comprising a step of culturing a microorganism into which at least one polynucleotide of the above (I) to (III) or a recombinant vector comprising it/them is introduced with a compound represented by the above formula B and isolating the compound represented by the above formula A.

According to a preferred embodiment of the present invention, there is provided the production method wherein the compound represented by the above formula B is a compound represented by the above formula B1.

There is provided the production method comprising a step of culturing a microorganism which comprises plasmid pCC1-PP1, plasmid pPP2 or plasmid pPP3, or one or more vectors selected from the group consisting of these plasmids with a compound represented by the above formula B and isolating the compound represented by the above formula A.

According to a more preferred embodiment of the present invention, there is provided a method for producing a compound represented by the above formula A, the method comprising a step of culturing a microorganism into which at least one polynucleotide of the above (IV) to (V) or a recombinant vector comprising it/them is introduced with a compound represented by the above formula B and isolating the compound represented by the above formula A.

According to a preferred embodiment of the present invention, there is provided a method for producing 1,11-di-O-cyclopropanecarbonyl-1,7,11-tri-deacetyl pyripyropene A by acylating the hydroxyl groups at the 1 and 11 positions of a compound represented by the above formula D using an acylating agent to produce a compound represented by the above formula B1 and culturing a microorganism which comprises plasmid pPP3 with the compound represented by the above formula B 1 (1,11-di-O-cyclopropanecarbonyl-1,11-dideacetyl pyripyropene O).

### [EXAMPLES]

The present invention will be further illustrated in detail by the following examples, which are not intended to restrict the present invention.

### Example 1: Preparation of Genomic DNA of Penicillium coprobium PF1169 Strain

A sterilized NB medium (500 ml) was placed in an Erlenmeyer flask (1 L). Penicillium coprobium PF1169 strain (Journal of Technical Disclosure No. 500997/2008) precultured in 1/2 CMMY agar medium at 28°C for 4 days was added to the above-mentioned medium and subjected to liquid culture at 28°C for 4 days. Filtration was carried out with Miracloth to obtain 5 g of fungal cells. From these fungal cells, 30 µg of genomic DNA was obtained in accordance with the manual attached to genomic DNA purification kit Genomic-tip 100/G (manufactured by Qiagen K.K.).

### Example 2: Degenerate primers for Amplification of Polyketide Synthase (PKS) and Amplified Fragment Thereof

Based on an amino acid sequence conserved among various filamentous bacterium polyketide synthases, the following primers were designed and synthesized as degenerate primers for amplification:
LC1: GAYCCIMGITTYTTYAAYATG (SEQ ID NO:1)
LC2c: GTICCIGTICCRTGCATYTC (SEQ ID NO:2)
(wherein R=A/G, Y=C/T, M=A/C, I=inosine).
Using these degenerate primers, the genomic DNA prepared in Example 1 and ExTaq polymerase (manufactured by Takara Bio Inc.) were allowed to react in accordance with the attached manual. An amplified fragment of about 700 bp was detected (see Figure 1). Further then, the above-mentioned amplified fragment was analyzed to specify the sequence of its internal 500 bp (SEQ ID NO:3).

### Example 3: Large-scale Sequencing of Genomic DNA and Amino Acid Sequence Homology Search

The genomic DNA of Penicillium coprobium PF1169 strain obtained in Example 1 was subjected to large-scale sequencing and homology search for amino acid sequences. Specifically, part of 50µg of genomic DNA was pretreated and thereafter subjected to Roche 454FLX DNA sequencer to obtain about 250 bp, 103 thousands of fragment sequences (in total, 49 Mb of sequence).

For these sequences, as known sequences among polyketide synthases and prenyltransferases, the following five sequences (sequences derived from polyketide synthases: Aspergillus (A.) fumigatus PKS 2146 a.a. and Penicillium (P.) griseofluvum 6-methylsalycilic acid synthase 1744 a.a.; as well as prenyltransferases: Aspergillus (A.) fumigatus Prenyltransferase, Aspergillus (A.) fumigatus Prenyltransferase (4-hydroxybezoate octaprenyltransferase) and Penicillium (P.) marneffei Prenyltransferase) were selected and search by homology sequence search software blastx was carried out, thereby obtaining 89, 86, 2, 1 and 3 of homology sequences, respectively (see Table 1). Further, from the homology sequences of A. fumigatus PKS 2146 a.a. and P. griseofluvum 6-methylsalycilic acid synthase 1744 a.a., 19 and 23 of contig sequences were respectively obtained (the contig sequences of A. fumigatus PKS 2146 a.a.: SEQ ID NOs:179 to 197; the contig sequences of P. griseofluvum 6-methylsalycilic acid synthase 1744 a.a.: SEQ ID NOs:198 to 220) (see Table 1).

**[Table 1]**

| Enzyme Name | Origin | Number of Homology Sequences | SEQ ID NO. |
|---|---|---|---|
| Polyketide Synthases | A. fumiqatus PKS 2146 a.a. | 89 | 4 to 92 |
| | P. qriseofluvum 6-methylsalycilic acid synthase 1744 a.a. | 86 | 93 to 178 |
| | A. fumigatus PKS 2146 a.a. | 19 (Contig sequences) | 179 to 197 |
| | P. qriseofluvum 6-methylsalycilic acid synthase 1744 a.a. | 23 (Contig sequences) | 198 to 220 |
| Prenyltransferases | A. fumigatus Prenyltransferase | 2 | 221, 222 |
| | A. fumigatus Prenyltransferase (4-hydroxybezoate octaprenyltransferase) | 1 | 223 |
| | P. marneffei Prenyltransferase | 3 | 224 to 226 |

### Example 4: PCR Amplification from Genomic DNA

From the search results of blastx obtained in Example 3, for polyketide synthases, 13 types of primer pairs shown in SEQ ID NOs:227 to 252 were synthesized. Similarly, for prenyltransferases, 5 types of primer pairs shown in SEQ ID NOs:253 to 262 were synthesized. When PCR was carried out for the genomic DNA using these primers, amplified fragments with the expected size were seen for all of the primer pairs (see Figure 1 and Figure 2).

### Example 5: Construction of Phage Genomic Library

A λ phage genomic library of Penicillium coprobium PF1169 strain was constructed using ABlueSTAR XhoI Half-site Arms Kit (manufactured by Takara Bio Inc., Cat. No. 69242-3) in accordance with the attached manual. That is, genomic DNA was partially digested using a restriction enzyme, Sau3A1. The DNA fragment with about 20 kb (0.5 µg) was ligated to 0.5 µg of ABlueSTAR DNA attached to the kit. This ligation solution was subjected to in vitro packaging using Lambda INN Packaging kit (manufactured by Nippon Gene Co., Ltd.) based on the manual attached to the kit to obtain 1 ml of a solution. This solution with packaged phages (10 µl) was infected into 100 µl of E. coli ER1647 strain and cultured on a plaque-forming medium at 37°C overnight, thereby obtaining about 500 clones of plaques. Thus, the genomic library composed of about 50000 clones of phages in which 10 to 20 kb genomic DNA of Penicillium coprobium PF1169 strain were introduced by infection was constructed.

### Example 6: Screening from Phage Library

For 10000 clones of the phage library prepared in Example 5, the primary screening was carried out by plaque hybridization using, as a probe, the PCR product amplified by LC1-LC2c primer pair prepared above. For labeling and detection of the probe, AlkPhos Direct Labelling and Detection System with CDP-Star (manufactured by GE Healthcare, Cat. No. RPN3690) was used. The above-mentioned hybridization was carried out in accordance with the attached manual.

By the primary screening, 6 clones remained as candidates. Further, as the result of the secondary screening by plaque hybridization, 4 clones were obtained. These positive clones were infected into E. coli BM25.8 strain and the phages were converted to plasmids in accordance with the attached manual, thereby obtaining 4 types of plasmids containing a desired region.

### Example 7: Preparation of Fosmid Genome Library

A genomic library of Penicillium coprobium PF1169 strain was constructed in accordance with the manual attached to CopyControl Fosmid Library Production Kit (manufactured by EPICENTRE, Cat. No. CCFOS110). That is, 0.25 µg of DNA fragment of about 40 kb genomic DNA was blunt-ended and then incorporated into fosmid vector pCCFOS (manufactured by Epicentre). This ligation solution was subjected to in vitro packaging using MaxPlax Lambda Packaging Extract attached to the kit based on the manual attached to the kit. This solution with packaged virus (10 µl) was infected into 100 µl of E. coli EPI300^{™}-T1^{R} strain and cultured on a medium containing chloramphenicol at 37°C overnight and selected, thereby obtaining 300 clones of colonies. Thus, about 30000 clones of the fosmids in which 40 kb the genomic DNA of Penicillium coprobium PF1169 strain were introduced by infection were obtained. They were aliquoted in a 96 well plate so as to be about 50 clones per well. Thus, the genomic library composed of 96 pools, about 4800 clones constructed.

### Example 8: Fosmid Library Screening

In accordance with the manual attached to the fosmid, plasmid DNAs were individually prepared from 96 pools of the library prepared in Example 7. Using the degenerate primers for polyketide synthase amplification synthesized in Example 2, PCR was carried out for 96 pools of these plasmid DNA samples. As a result, DNA fragments of about 700 bp were amplified from 9 pools. Further, a petri dish containing colonies of about 300 clones or more was prepared from the positive pools and re-screening was carried out by colony hybridization. As a result, by using LC1-LC2c primer pair, 9 types of fosmids were obtained from about 4800 clones.

### Example 9: Large-Scale Sequencing of Genomic DNA and Amino Acid Sequence Homology Search

Genomic DNA of Penicillium coprobium PF1169 strain obtained in Example 1 was subjected to large-scale sequencing and homology search for amino acid sequences. Specifically, part of 50 µg of genomic DNA was pretreated and then subjected to Roche 454FLX DNA sequencer to obtain 1405 fragment sequences with an average contig length of 19.621 kb (sequence of a total base length of 27.568160 Mb).
For these sequences, as known sequences among polyketide synthases and prenyltransferases, the following five sequences (sequences derived from polyketide synthases: Penicillium(P.) griseofluvum 6-methylsalycilic acid synthase 1744 a.a. (P22367) and Aspergillus(A.) fumigatus PKS 2146 a.a. (Q4WZA8); as well as prenyltransferases: Penicillium(P.) marneffei Prenyltransferase (Q0MRO8), Aspergillus (A.) fumigatus Prenyltransferase (Q4WBI5) and Aspergillus(A.) fumigatus Prenyltransferase (4-hydroxybezoate octaprenyltransferase) (Q4WLD0)) were selected and search by homology sequence search software blastx was carried out, thereby obtaining 22 (P22367), 21 (Q4WZA8), 2 (Q0MRO8), 3 (Q4WBI5) and 3 (Q4WLD0) of the homologous sequences, respectively.

### Example 10: Fosmid Library Screening and Sequence Analysis of Cluster Genes

In accordance with the manual attached to a fosmid kit (manufactured by EPICENTRE, CopyControl Fosmid Library Production Kit), plasmid DNAs were individually prepared from 96 pools of the library prepared in Example 7. Based on base sequences determined by Roche 454FLX DNA sequencer, homology search for amino acid sequences was carried out to search regions adjacent to polyketide synthase and prenyltransferase. Based on the base sequence of prenyltransferase of the obtained region, a primer pair (No. 27) capable of amplifying 400 bp DNA fragment was synthesized. Using the primers, PCR was carried out for these 48 pools of plasmid DNA samples. As a result, expected DNA fragments of about 400 bp (SEQ ID NO:263) were amplified from 11 pools (see Figure 3). Further, a petri dish containing colonies of about 300 clones or more was prepared from 6 pools of the positive pools and re-screening was carried out by colony hybridization. As a result, by using 27F + 27R primer pair (27F primer: SEQ ID NO:264, 27R primer: SEQ ID NO:265), 4 types of fosmids were obtained from about 4800 clones. One of them was named pCC1-PP1 and the entire sequence of the inserted fragment was determined (SEQ ID NO:266).
The obtained pCC1-PP1 was transformed into Escherichia coli EPI300^{™}-T1^{R} strain (attached to the fosmid kit), thereby obtaining Escherichia coli EPI300^{™}-T1^{R} strain/pCC1-PP1.
When a homology search was carried out between the above-mentioned sequence of SEQ ID NO:266 and each of CoA ligase; LovB-like polyketide synthase (PKS); Cytochrome P450 monooxygenase, Cyclase (IMP: Integral membrane protein), FAD-dependent monooxygenase (FMO), which are hydroxylases; UbiA-like prenyltransferase (UbiAPT); Acetyltransferase (AT), Acetyltransferase-2 (AT-2), which are acetyltransferases; and Cation transporting ATPase (the above-mentioned enzymes are all derived from Aspergillus fumigatus Af293 strain), a high homology of 70% or more was seen in any search.
The nucleotides 3342 to 5158 of SEQ ID NO:266 encode CoA ligase and the corresponding polypeptide is shown with the amino acid sequence depicted in SEQ ID NO:267; the nucleotides 5382 to 12777 of SEQ ID NO:266 encode LovB-like polyketide synthase (PKS) and the corresponding polypeptide is shown with the amino acid sequence depicted in SEQ ID NO:268; the nucleotides 13266 to 15144 of SEQ ID NO:266 (hereinafter, a protein encoded by this polynucleotide sequence (P450-1) is referred to as Cytochrome P450 monooxygenase (1) (P450-1)) and the nucleotides 16220 to 18018 (hereinafter, a protein encoded by this polynucleotide sequence (P450-2) is referred to as Cytochrome P450 monooxygenase (2) (P450-2)) encode Cytochrome P450 monooxygenases and the corresponding polypeptides are shown with the amino acid sequences depicted in SEQ ID NOs:269 and 270, respectively; the nucleotides 18506 to 19296 of SEQ ID NO:266 encode Cyclase and the corresponding polypeptide is shown with the amino acid sequence depicted in SEQ ID NO:271; the nucleotides 19779 to 21389 of SEQ ID NO:266 encode FAD-dependent monooxygenase (FMO) and the corresponding polypeptide is shown with the amino acid sequence depicted in SEQ ID NO:272; the nucleotides 21793 to 22877 of SEQ ID NO:266 encode UbiA-like prenyltransferase (UbiAPT) and the corresponding polypeptide is shown with the amino acid sequence depicted in SEQ ID NO:273; the nucleotides 23205 to 24773 of SEQ ID NO:266 encode Acetyltransferase (AT) and the corresponding polypeptide is shown with the amino acid sequence depicted in SEQ ID NO:274; the nucleotides 25824 to 27178 of SEQ ID NO:266 encode Acetyltransferase-2 (AT-2) and the corresponding polypeptide is shown with the amino acid sequence depicted in SEQ ID NO:275; and the nucleotides 27798 to 31855 of SEQ ID NO:266 encode Cation transporting ATPase and the corresponding polypeptide is shown with the amino acid sequence depicted in SEQ ID NO:276.

### Example 11: Hydroxylation of Pyripyropene E or Pyripyropene O by Transformation of Aspergillus Oryzae

Pyripyropene E used below was able to be produced by a method for culturing a microorganism based on the method described in Japanese Patent Laid-Open Publication No. 239385/1996, WO94/09147 or U.S. Patent No. 5597835, or the total synthesis method described in Tetrahedron Letters, vol. 37, No. 36, 6461-6464, 1996. Also, pyripyropene O used below was able to be produced by a method for culturing a microorganism based on the method described in J. Antibiotics 49, 292-298, 1996 or WO94/09147.

### (1) Preparation of Expression Vector for Introducing into Filamentous Bacterium

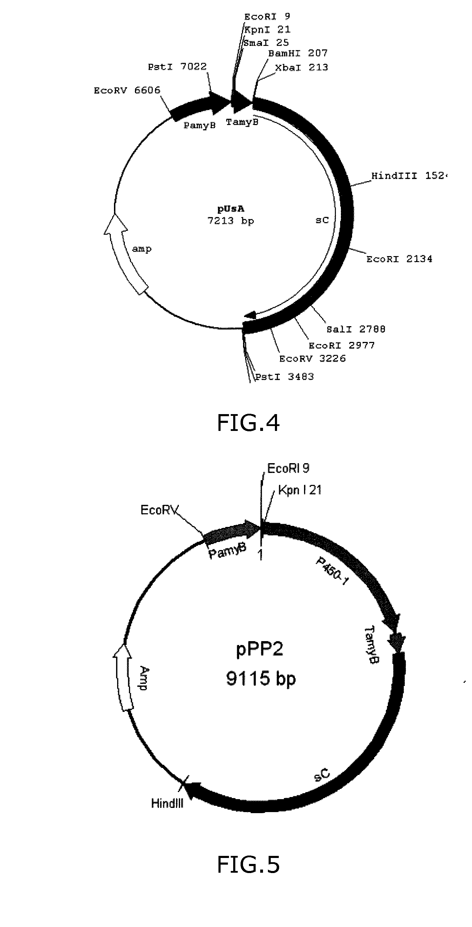

pUSA (Figure 4) and pHSG399 (Takara Bio Inc.) were individually digested with KpnI and ligated, thereby obtaining pUSA-HSG. This plasmid was digested with SmaI and KpnI in the order mentioned, and subjected to gel purification, thereby obtaining a linear vector DNA having a KpnI cohesive end and SmaI blunt end.

### (2) Preparation of Plasmid pPP2

With fosmid pCC1-PP1 as a template, the polynucleotide of the above-mentioned P450-1 was amplified using a primer pair P450-1 with Kpn F (SEQ ID NO:277)/P450-1 with Swa R (SEQ ID NO:278). The purified DNA fragment was cloned into pCR-Blunt (Invitorogen, Cat. No. K2700-20). The plasmid obtained was digested with KpnI and SwaI. The above-mentioned P450-1 fragment was ligated to the above-described vector pUSA-HSG, thereby obtaining a plasmid pPP2 shown in Figure 5.

### (3) Preparation of Plasmid pPP3

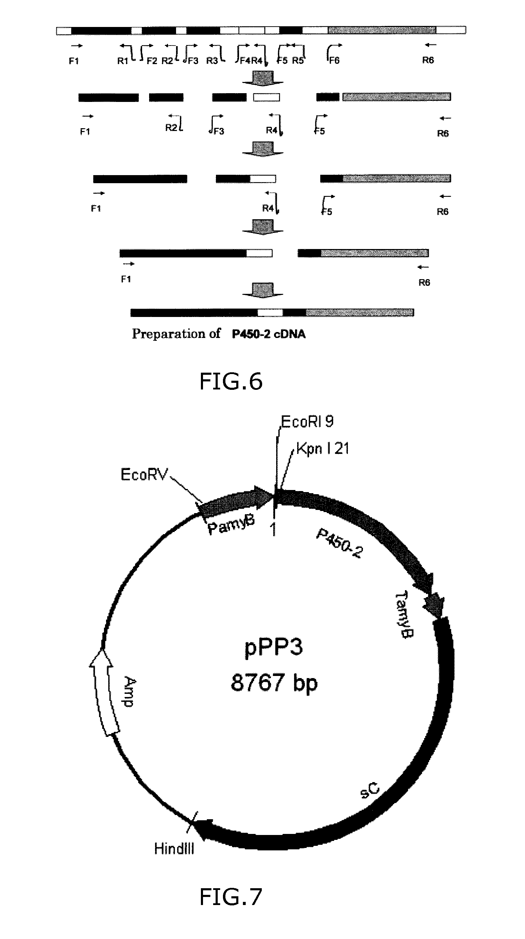

With fosmid pCC1-PP1 as a template, in accordance with the flow shown in Figure 6, exons alone were first amplified using primer pairs F1(SEQ ID NO:279)/R1(SEQ ID NO:280), F2(SEQ ID NO:281)/R2(SEQ ID NO:282), F3(SEQ ID NO:283)/R3(SEQ ID NO:284), F4(SEQ ID NO:285)/R4(SEQ ID NO:286), F5(SEQ ID NO:287)/R5(SEQ ID NO:288) and F6(SEQ ID NO:289)/R6(SEQ ID NO:290), thereby obtaining six fragments. Next, amplification was carried out with these fragments as templates using primer pairs of F1/R2, F3/R4 and F5/R6, thereby obtaining longer fragments. Further, by repeating amplification using primer pairs of F1/R4 and F1/R6, cDNA which did not contain introns of the polynucleotide of the above-mentioned P450-2 was prepared. This cDNA fragment was inserted into pCR-Blunt (Invitorogen, Cat. No. K2700-20) and the obtained plasmid was used as a template for amplification by a primer pair, infusion F of P450-2-cDNA (SEQ ID NO:291)/infusion R of P450-2-cDNA (SEQ ID NO:292). Based on the manual of the kit, a plasmid pPP3 shown in Figure 7 was obtained using In-Fusion Advantage PCR Cloning Kit (Clontech).

### (4) Preparation of Plasmid pPP7 (AT)

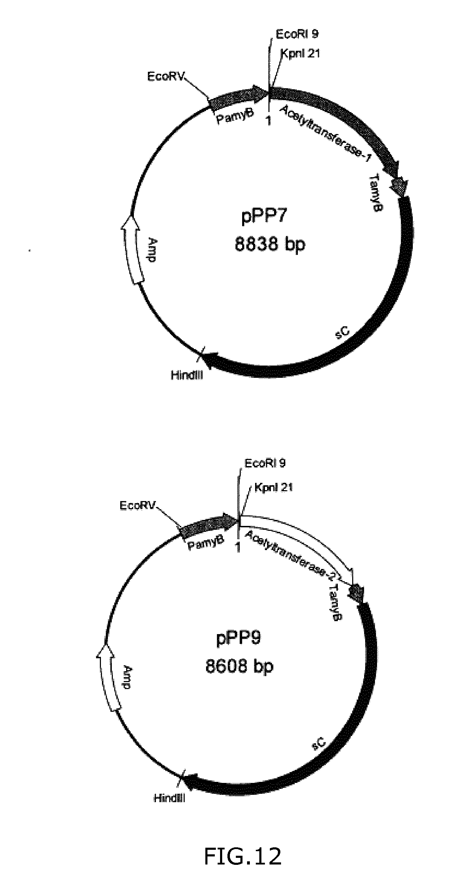

Using vector pUSA-HSG for filamentous fungus transformation obtained in the above-mentioned Example 11(1), plasmid pPP7 was obtained.
With fosmid pCC1-PP1 as a template, the polynucleotide of AT was each amplified using a primer pair AT F with Swa (SEQ ID NO:293) and AT R with Kpn (SEQ ID NO:294). A purified fragment was cloned into a vector for PCR fragments, pCR-Blunt (Invitorogen, Cat. No. K2700-20). The plasmid obtained was digested with KpnI and SwaI. Each fragment was ligated between the KpnI and SmaI sites of the above-described filamentous bacterium vector pUSA-HSG, thereby obtaining a plasmid pPP7 shown in Figure 12.

### (5) Preparation of Plasmid pPP9 (AT-2)

With fosmid pCC1-PP1 as a template, Toxin fragment was amplified using a primer pair infusion F of Toxin (SEQ ID NO:295) and infusion R of Toxin (SEQ ID NO:296), and inserted between the KpnI and SmaI sites of f the above-described filamentous bacterium vector pUSA-HSG using In-Fusion Advantage PCR Cloning Kit (manufactured by Clontech, Cat. No. 639619), based on the manual of the kit, thereby obtaining a plasmid pPP9 shown in Figure 12.

### (6) Transformation of Aspergillus Oryzae (A. oryzae)

In a CD-Met (containing L-Methionine 40 µg/ml) agar medium, A. oryzae (HL-1105 strain) was cultured at 30°C for one week. From this petri dish, conidia (> 10⁸) were collected and seeded in 100 ml of YPD liquid medium in a 500 ml-flask. After 20-hour culturing (30°C, 180 rpm), fungal cells having a moss ball shape were obtained. The fungal cells were collected with a 3G-1 glass filter, washed with 0.8 M NaCl, and water was removed well. The resultant was suspended with TF solution I (protoplast formation solution) and then shook at 30°C, at 60 rpm for 2 hours. At a 30-minute interval, observation under the microscope was carried out and the presence of protoplasts was checked. Thereafter, the culture medium was filtered and subjected to centrifugation (2000 rpm, 5 minutes) to collect protoplasts, which were then washed with TF solution II. After washing, 0.8 volume of TF solution II and 0.2 volume of TF solution III were added and mixed, thereby obtaining a protoplast suspension.

To 200 µl of this suspension, 10 µg of plasmid DNA (pPP2 or pPP3) was added. The mixture was left to stand on ice 30 minutes and added with TF solution III (1 mL). The resulting mixture was gently mixed and then left to stand at room temperature for 15 minutes. Thereafter, the plasmid DNA was introduced into the above-mentioned protoplasts. To this, TF solution II (8 mL) was added and subjected to centrifugation (at 2000 rpm for 5 minutes). Further, protoplasts were then recovered with 1 to 2 ml being left over. The recovered protoplast solution was dropped to a regeneration medium (lower layer) and a regeneration medium (upper layer) was poured. The resultant was mixed by turning a petri dish and then cultured at 30°C for 4 to 5 days. Generated clones were isolated in the regeneration medium (lower layer), subcultured and purified, thereby obtaining a transformant (Aspergillus oryzae PP2-1 and Aspergillus oryzae PP3-2).

Based on the method described in the above-mentioned Example 11 (6), transformants in which each of the plasmid DNAs (pPP7 and pPP9) was introduced were obtained (Aspergillus oryzae PP7 and Aspergillus oryzae PP9).

The above-mentioned TF solution I (protoplast formation solution) was prepared with the following compositions.

| Name of Compound | | Concentration |
|---|---|---|
| Yatalase (manufactured by Takara Bio Inc.) | | 20 mg/ml |
| | Ammonium sulfate | 0.6 M |
| | Maleic acid-NaOH | 50 mM |

After the above-mentioned compositions (pH 5.5) were prepared, filter sterilization was carried out.

The above-mentioned TF solution II was prepared with the following compositions.

| Name of Compound | Amount |
|---|---|
| 1.2 M Sorbitol (MW=182.17) | 43.72 g |
| 50 mM CaCl₂ | 10 ml 1 M CaCl₂ (1/20) |
| 35 mM NaCl | 1.4 ml 5 M NaCl |
| 10 mM Tris-HCl | 2 ml 1 M Tris-HCl (1/100) |

Water was further added to attain a total volume of 200 ml.
After the above-mentioned compositions were prepared, autoclave sterilization was carried out.

The above-mentioned TF solution III was prepared with the following compositions.

| Name of Compound | Amount |
|---|---|
| 60% PEG4000 | 6 g |
| 50 mM CaCl₂ | 500 µl 1 M CaCl₂ (1/20) |
| 50 mM Tris-HCl | 500 µl 1 M Tris-HCl (1/100) |

Water was further added to attain a total volume of 10 ml.
After the above-mentioned compositions were prepared, filter sterilization was carried out.

The above-mentioned regeneration medium was prepared with the following compositions.

| Name of Compound | Amount | Concentration |
|---|---|---|
| Sorbitol (MW=182.17) | 218.6 g | 1.2 M |
| NaNO₃ | 3.0 g | 0.3% (w/v) |
| KCI | 2.0 g | 0.2% (w/v) |
| KH₂PO₄ | 1.0 g | 0.1% (w/v) |
| MgSO₄·7H₂O | 2 ml of 1 M MgSO₄ | 0.05% 2 mM |
| Trace elements solution | 1 ml | |
| Glucose | 20.0 g | 2% (w/v) |

Water was further added to attain a total volume of 1 L.
After the above-mentioned compositions (pH 5.5) were prepared, autoclave sterilization was carried out.

In addition, the Trace elements solution used above was prepared with the following composition.

| Name of Compound | Amount |
|---|---|
| FeSO₄·7H₂O | 1.0 g |
| ZnSO₄·7H₂O | 8.8 g |
| CuSO₄·5H₂O | 0.4 g |
| Na₂B₄O₇·10H₂O | 0.1 g |
| (NH₄)₆Mo₇O₂₄·4H₂O | 0.05 g |

Water was further added to attain a total volume of 1 L.
After the above-mentioned compositions were prepared, autoclave sterilization was carried out.

### (7) Function Analysis and Addition Culture Test of P450-1

To a YPD medium (1% (w/v) Yeast Extract, 2% (w/v) Peptone, 2% (w/v) Dextrose) containing 1% (w/v) maltose, a 1/100 volume of 2 mg/mL dimethyl sulfoxide solution of pyripyropene E was added to provide medium A. From flora of Aspergillus oryzae PP2-1 cultured in Czapek Dox agar medium, conidia thereof were collected and suspended in sterilized water. This conidia suspension was adjusted to 10⁴ spores/mL. Further, 100 µL of this adjusted conidia suspension was added to 10 mL of medium A and cultured with shaking at 25°C for 96 hours. To this culture medium, 10 mL of acetone was added and the mixture was mixed well. Thereafter, acetone was removed using a centrifugal concentrator. To this, 10 mL of ethyl acetate was added and the resulting mixture was mixed well and then only the ethyl acetate layer was recovered. A dried product obtained by removing ethyl acetate using the centrifugal concentrator was dissolved in 1000 µL of methanol. This was used as a sample and analyzed by LC-MS (Waters, Micromass ZQ, 2996PDA, 2695 Separation module, Column: Waters XTerra C18 (Φ4.5×50 mm, 5 µm)) and LC-NMR (manufactured by Burker Daltonik, Avance500).

As the results of the above-mentioned LC-MS measurement, it was confirmed that the obtained compound was single Compound E which increased by a molecular weight of 16 compared with pyripyropene E. In addition, as the results of the LC-NMR measurement, it was confirmed that this Compound E was an 11-position hydroxide of pyripyropene E. It was confirmed that the above-mentioned Cytochrome P450 monooxygenase (1) was an enzyme hydroxylating the 11-position of pyripyropene E with pyripyropene E as a substrate.

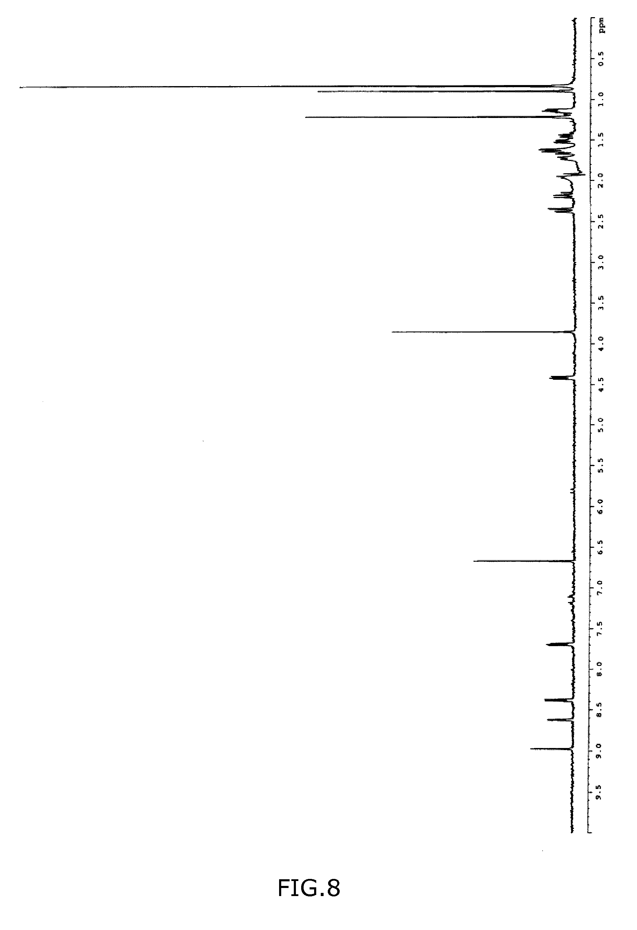

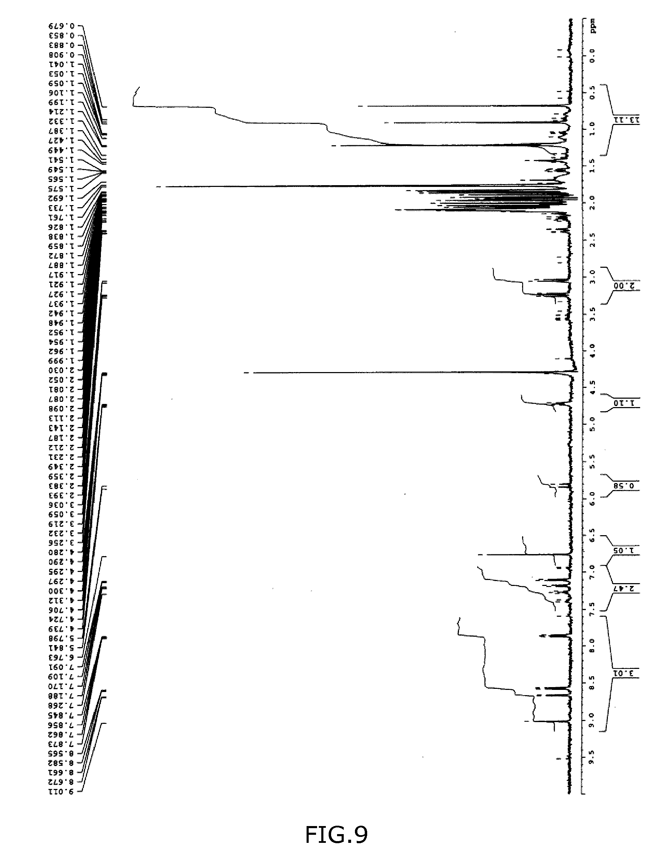

Physicochemical properties of the above-mentioned Compound E are shown below:
1. Mass spectrum: ES-MS 468M/Z (M+H)⁺
2. Molecular formula: C₂₇H₃₃NO₆
3. HPLC: Column: Waters XTerra Column C18 (5 µm, 4.6 mmx50 mm), 40°C, Mobile phase: From 20% aqueous acetonitrile solution to 100% acetonitrile in 10 minutes (linear gradient), Flow rate: 0.8 ml/min, Detection: Retention time 6.696 minutes at UV 323 nm
4. ¹H-NMR spectrum (CD₃CN, 2H: 3.134, 3.157 H-11)
The charts of the ¹H-NMR spectrum of pyripyropene E and ¹H-NMR spectrum according to the above-mentioned 4 are shown in Figure 8 and Figure 9, respectively.

### (8) Function Analysis and Addition Culture Test of P450-2

To a YPD medium (1% (w/v) Yeast Extract, 2% (w/v) Peptone, 2% (w/v) Dextrose) containing 1% (w/v) maltose, a 1/100 volume of 2 mg/mL dimethyl sulfoxide solution of pyripyropene E was added to provide medium A, and similarly a 1/100 volume of 2 mg/mL dimethyl sulfoxide solution of pyripyropene O was added to provide medium B. From flora of Aspergillus oryzae PP3-2 cultured in Czapek Dox agar medium, conidia thereof were collected and suspended in sterilized water. This conidia suspension was adjusted to 10⁴ spores/mL. Further, 500 µL of the adjusted conidia suspension was added to 50 mL of medium A or medium B and cultured with shaking at 25°C for 96 hours. To this culture medium, 50 mL of acetone was added and the mixture was mixed well. Thereafter, acetone was removed using a centrifugal concentrator. To this, 50 mL of ethyl acetate was added and the resulting mixture was mixed well and then only the ethyl acetate layer was recovered. A dried product obtained by removing ethyl acetate using the centrifugal concentrator was dissolved in 1500 µL of methanol. This was used as a sample and analyzed by LC-MS (manufactured by Waters, Micromass ZQ, 2996PDA, 2695 Separation module, Column: Waters XTerra C18 (Φ4.5×50 mm, 5 µm)) and LC-NMR (manufactured by Burker Daltonik, Avance500). As the results of the LC-MS measurement, from a sample obtained from the medium A, Compound F which increased by a molecular weight of 32 compared with pyripyropene E was detected. Also, from a sample obtained from the medium B, Compound G which increased by a molecular weight of 32 compared with pyripyropene O was detected. Further, as the results of the LC-NMR measurement, it was confirmed that Compound G was a 7-position and 13-position hydroxide of pyripyropene O. It was confirmed that the above-mentioned Cytochrome P450 monooxygenase (2) had a hydroxylase activity of the 7-position and 13-position of each of pyripyropene E or pyripyropene O.

Physicochemical properties of the above-mentioned Compound F are shown below:
1. Mass spectrum: ES-MS 484M/Z (M+H)⁺
2. Molecular formula: C₂₇H₃₃NO₇
3. HPLC: Column: Waters XTerra Column C18 (5 µm, 4.6 mm×50 mm), 40°C, Mobile phase: From 20% aqueous acetonitrile solution to 100% acetonitrile in 10 minutes (linear gradient), Flow rate: 0.8 ml/min, Detection: Retention time 5.614 minutes at UV 323 nm

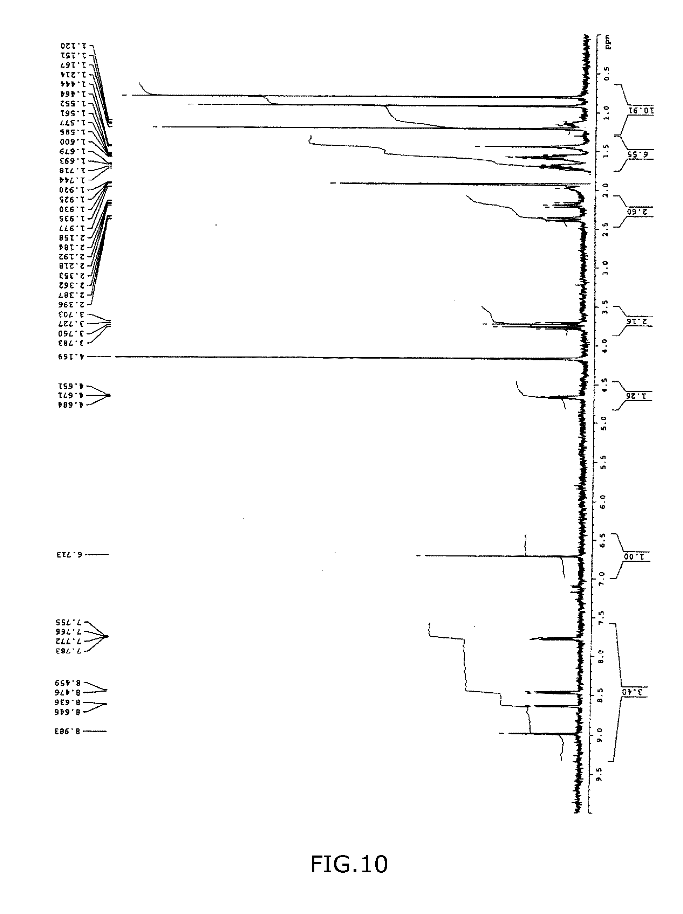

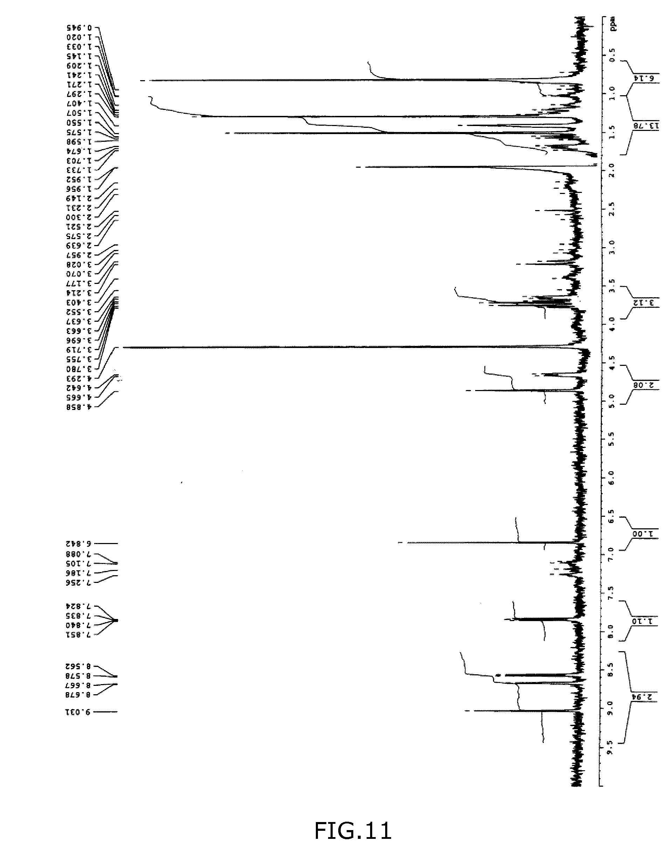

Physicochemical properties of the above-mentioned Compound G are shown below:
1. Mass spectrum: ES-MS 542M/Z (M+H)⁺
2. Molecular formula: C₂₉H₃₅NO₉
3. HPLC: Column: Waters XTerra Column C18 (5 µm, 4.6 mm×50 mm), 40°C, Mobile phase: From 20% aqueous acetonitrile solution to 100% acetonitrile in 10 minutes (linear gradient), Flow rate: 0.8 ml/min, Detection: Retention time 5.165
   minutes at UV 323 nm
4. ¹H-NMR spectrum (CD₃CN, 1H 4.858 H-13), (CD₃CN, 1H 3.65 H-7)
The charts of the ¹H-NMR spectrum of pyripyropene O and the above-mentioned Compound G are shown in Figure 10 and Figure 11, respectively.

### (9) Function Analysis and Addition Culture Test of Acetyltransferase-1

To a YPD medium (1% (w/v) Yeast Extract, 2% (w/v) Peptone, 2% (w/v) Dextrose) containing 1% (w/v) maltose, a 1/100 volume of 2 mg/mL dimethyl sulfoxide solution of deacetyl pyripyropene E (see Reference Example 3) was added to provide medium D; a 1/100 volume of 2 mg/mL dimethyl sulfoxide solution of 11-deacetyl pyripyropene O (see Reference Example 4) was added to provide medium E and 2 mg/mL dimethyl sulfoxide solution of 7-deacetyl pyripyropene A (see Reference Example 5) was added to provide medium F. From flora of Aspergillus oryzae PP7 cultured in Czapek Dox agar medium, conidia thereof were collected and suspended in sterilized water. This conidia suspension was adjusted to 10⁴ spores/mL. Further, 200 µL of this was added to 20 mL of medium D, medium E or medium F and cultured with shaking at 25°C for 96 hours. To this culture medium, 20 mL of acetone was added and the mixture was mixed well. Thereafter, acetone was removed using a centrifugal concentrator. To this, 20 mL of ethyl acetate was added and the resulting mixture was mixed well and then only the ethyl acetate layer was recovered. A dried product obtained by removing ethyl acetate using the centrifugal concentrator was dissolved in 1000 µL of methanol. This was used as a sample and analyzed by LC-MS (Waters, Micromass ZQ, 2996PDA, 2695 Separation module, Column: Waters XTerra C18 (Φ4.5×50 mm, 5 µm)).

As the results of the LC-MS measurement, a single compound which increased a molecular weight of 42 compared with deacetyl pyripyropene E was detected from the medium D. It was confirmed that the compound had the same retention time, molecular ion peaks and UV absorption as pyripyropene E (see Reference Example 6). Meanwhile, no newly-generated compounds were detected from the medium E and medium F. From this, it was confirmed that Acetyltransferase-1 had an acetyl transferase activity which acetylated specifically the 1-position of deacetyl pyripyropene E.

### (10) Function Analysis and Addition Culture Test of Acetyltransferase-2

To a YPD medium (1% (w/v) Yeast Extract, 2% (w/v) Peptone, 2% (w/v) Dextrose) containing 1% (w/v) maltose, a 1/100 volume of 2 mg/mL dimethyl sulfoxide solution of deacetyl pyripyropene E (see Reference Example 3) was added to provide medium D; a 1/100 volume of 2 mg/mL dimethyl sulfoxide solution of 11-deacetyl pyripyropene O (see Reference Example 4) was added to provide medium E and 2 mg/mL dimethyl sulfoxide solution of 7-deacetyl pyripyropene A (see Reference Example 5) was added to provide medium F. From flora of Aspergillus oryzae PP9 cultured in Czapek Dox agar medium, conidia thereof were collected and suspended in sterilized water. This conidia suspension was adjusted to 10⁴ spores/mL. Further, 200 µL of this was added to 20 mL of medium D, medium E or medium F and cultured with shaking at 25°C for 96 hours. To this culture medium, 20 mL of acetone was added and the mixture was mixed well. Thereafter, acetone was removed using a centrifugal concentrator. To this, 20 mL of ethyl acetate was added and the resulting mixture was mixed well and then only the ethyl acetate layer was recovered. A dried product obtained by removing ethyl acetate using the centrifugal concentrator was dissolved in 1000 µL of methanol. This was used as a sample and analyzed by LC-MS (Waters, Micromass ZQ, 2996PDA, 2695 Separation module, Column: Waters XTerra C18 (Φ4.5×50 mm, 5 µm)).

As the results of the LC-MS measurement, a single compound which increased a molecular wieght of 42 compared with 11-deacetyl pyripyropene O was detected from the medium E. It was comfimred that this compound had the same retention time, molecular ion peaks and UV absorption as pyripyropene O (see Reference Example 7). Further, a single compound which increased a molecular wieght of 42 compared with 7-deacetyl pyripyropene A was detected from the medium F. It was comfimred that the compound had the same retention time, molecular ion peaks and UV absorption as pyripyropene A (see Reference Example 8). Meanwhile, no newly-generated compounds were detected from the medium D. From this, it was confirmed that Acetyltransferase-2 had an acetyltransferase activity which acetylated specifically the 11-position of 11-deacetyl pyripyropene O and the 7-position of 7-deacetyl pyripyropene A.

### Example 12:Synthesis and Structural Analysis of Compound D (1,11-dideacetyl Pyripyropene O)

Pyripyropene O (30 mg) was dissolved in methanol-water (19:1, 2 mL) and potassium carbonate (20 mg) was added thereto. The resultant was stirred at room temperature for 22 and half hours, and thereafter acetic acid (0.1 mL) was added to concentrate. Ethyl acetate and water were added and then extraction was carried out with ethyl acetate. The ethyl acetate layer was washed with saturated sodium chloride solution and dried with anhydrous sodium sulfate. The solvent was evaporated under reduced pressure, thereby obtaining a crude product of 1,11-dideacetyl pyripyropene O. The crude product was purified by preparative thin layer chromatography (Merck silica gel 60F254, 0.5 mm, hexane:acetone=1:1), thereby obtaining 1,11-dideacetyl pyripyropene O (23 mg).
ESI-MS; 426 m/z (M+H)⁺; ¹H-NMR (CDCl₃) δ 0.89 (3H, s), 0.97 (3H, s), 1.14 (1H, dt, J = 4.2, 12.8 Hz), 1.20-1.25 (1H, m), 1.28 (3H, s), 1.45-1.59 (3H, m), 1.64-1.75 (3H, m), 1.82 (1H, dt, J = 3.5, 9.6 Hz), 2.11-2.14 (1H, m), 2.25 (1H, dd, J = 12.8, 17.1 Hz), 2.54 (1H, dd, J = 4.6, 17.1 Hz), 3.45 (1H, d, J = 10.3 Hz), 3.68 (1H, dd, J = 5.0, 11.2 Hz), 3.75 (1H, d, J = 10.3 Hz), 6.42 (1H, s), 7.39 (1H, dd, J = 4.8, 8.0 Hz), 8.10 (1H, ddd, J = 1.6, 2.0, 8.0 Hz), 8.65 (1H, dd, J = 1.6, 4.8 Hz), 8.99 (1H, d, J = 2.0 Hz)

### Example 13:synthesis and Structural Analysis of Compound B1 (1,11-di-O-cyclopropanecarbonyl-1,11-dideacetvl Pyripyropene O)

1,11-dideacetyl pyripyropene O (22 mg) was suspended in ethyl acetate (1 mL), and pyridine (20 mg) and cyclopropanecarbonyl chloride (22 mg) were added thereto. The resultant was stirred at room temperature for 4 hours, and water was added and then extraction was carried out with ethyl acetate. The ethyl acetate layer was washed with saturated sodium chloride solution and dried with anhydrous sodium sulfate. The solvent was evaporated under reduced pressure, thereby obtaining a c r u d e product of 1,11-di-O-cyclopropanecarbonyl-1,11-dideacetyl pyripyropene O. The crude product was purified by preparative thin layer chromatography (Merck silica gel 60F254, 0.5 mm, chloroform:methanol=10:1), thereby obtaining 1,11-di-O-cyclopropanecarbonyl-1,11-dideacetyl pyripyropene O (17 mg).
ESI-MS; 562 m/z (M+H)⁺; ¹H-NMR (CDCl₃) δ 0.88 (3H, s), 0.99 (3H, s), 0.84-1.08 (8H, m), 1.21 (1H, dt, J = 3.6, 13.4 Hz), 1.28 (3H, s), 1.43-1.48 (2H, m), 1.56-1.73 (6H, m), 1.81-1.85 (2H, m), 2.13-2.16 (1H, m), 2.26 (1H, dd, J = 12.8, 17.1 Hz), 2.55 (1H, dd, J = 4.6, 17.1 Hz), 3.71 (1H, d, J = 11.7 Hz), 3.93 (1H, d, J = 11.7 Hz), 4.82 (1H, dd, J = 4.7, 12.0 Hz), 6.44 (1H, s), 7.41 (1H, dd, J = 4.8, 8.0 Hz), 8.12 (1H, ddd, J = 1.4, 2.0, 8.0 Hz), 8.66 (1H, dd, J = 1.4, 4.8 Hz), 9.00 (1H, d, J = 2.0 Hz)

### Example 14:Synthesis o f 1,11-di-O-cyclopropanecarbonyl-1,7,11-tri-deacetyl pyripyropene A

From flora of Aspergillus oryzae PP3-2 (FERM BP-11141) cultured in Czapek Dox agar medium, conidia thereof were collected and suspended in sterilized water to obtain a conidia suspension of 10⁴ spores/mL. To 20 mL of YPD medium (1% (w/v) Yeast Extract, 2% (w/v) Peptone, 2% (w/v) Dextrose) containing 1% (w/v) maltose, 200 µL of the conidia suspension and 200 µL of 2 mg/mL dimethyl sulfoxide solution of 1,11-di-O-cyclopropanecarbonyl-1,11-dideacetyl pyripyropene O obtained in Example 13 were added and the resulting mixture was cultured with shaking at 25°C for 96 hours. To this culture medium, 20 mL of acetone was added and the mixture was mixed well. Thereafter, acetone was removed using a centrifugal concentrator. To this, 20 mL of ethyl acetate was added and the resulting mixture was mixed well and then the ethyl acetate layer was recovered. Ethyl acetate was removed using the centrifugal concentrator, thereby obtaining a product. By analyzing under the following conditions, it was confirmed that the product was the captioned compound described in WO2006/129714 (Conversion rate 90%).
LC-MS (Waters, Micromass ZQ, 2996PDA, 2695 Separation module, Column: Waters XTerra C18 (Φ4.5×50 mm, 5 µm)), Mobile phase: From 20% aqueous acetonitrile solution to 100% acetonitrile in 10 minutes (linear gradient), Flow rate: 0.8 ml/min, Column oven: 40°C, Detection: UV 330 nm.

### Reference Example 1 Synthesis and Structural Analysis of 11-deacetyl Pyripyropene O

Pyripyropene O (30 mg) was dissolved in methanol-water (19:1, 2 mL) and potassium carbonate (20 mg) was added thereto. The resultant was stirred for 22 hours, and thereafter acetic acid (0.1 mL) was added and the solvent was evaporated under reduced pressure. Ethyl acetate and water were added and then extraction was carried out with ethyl acetate. The ethyl acetate layer was washed with saturated sodium chloride solution and dried with anhydrous sodium sulfate. The solvent was evaporated under reduced pressure, thereby obtaining a crude product of 1,11-deacetyl pyripyropene O (30 mg). The crude product of 1,11-deacetyl pyripyropene O (23 mg) was dissolved in N,N-dimethylformamide (0.4 mL) and
triethylamine (8 mg) and acetic acid anhydride (7 mg) were added thereto. After the resulting mixture was stirred at room temperature for 23 hours, water was added and then extraction was carried out with ethyl acetate. The ethyl acetate layer was washed with saturated sodium chloride solution and dried with anhydrous magnesium sulfate. The solvent was evaporated under reduced pressure, thereby obtaining a crude product of 1-deacetyl pyripyropene O (28 mg). The crude product of 1-deacetyl pyripyropene O (28 mg) was dissolved in toluene and 1,8-diazabicyclo [5,4,0]-7-undecene (20 mg) was added. The mixture was stirred at 70°C for 20 hours and allowed to cool. Ethyl acetate and water were added and then extraction was carried out with ethyl acetate. The ethyl acetate layer was washed with saturated sodium chloride solution and dried with anhydrous magnesium sulfate. The solvent was evaporated under reduced pressure, thereby obtaining a crude product of 11-deacetyl pyripyropene O (20 mg).
After dissolved in methanol, this was used as a sample and HPLC (manufactured by SHIMADZU, LC-6AD, SPD-M20A PDA, CBM-20A, Column; Waters XTerra C18 (Φ4.5×50 mm, 5 µm, mobile phase 30% aqueous acetonitrile solution to 55% aqueous acetonitrile solution in 25 minutes (linear gradient), flow rate: 1.0 ml/min, retention time 18 to 19 minutes) was repeated to preparative separation, thereby obtaining 11-deacetyl pyripyropene O (4.0 mg).
ESI-MS; m/z 468 (M+H)⁺
¹H-NMR (CDCl₃) δ (ppm); 0.68 (3H, s), 0.95 (3H, s), 1.21-2.21 (10H, m), 1.25 (3H, s), 2.05 (3H, s), 2.20 (1H, dd, J = 4.63, 17.3 Hz), 2.50 (1H, dd, J = 4.63, 17.3 Hz), 2.94 (1H, d, J = 12.5 Hz), 3.33 (1H, d, J = 12.5 Hz), 4.87 (1H, dd, J = 4.6, 12.2 Hz), 6.48 (1H, s), 7.57 (1H, dd, J = 5.1, 8.1 Hz), 8.29 (1H, d, J = 8.3 Hz), 8.68 (1H, d, J = 4.6 Hz), 9.04 (1H, s)

### Reference Example 2: Synthesis and Structural Analysis of Deacetyl Pyripyropene E

Pyripyropene E (29 mg) (Pyripyropene E was obtained by the method described in Japanese Patent Laid-Open Publication No. 239385/1996.) was dissolved in methanol-water (19:1, 1 mL) and potassium carbonate (53 mg) was added thereto. The resultant was stirred for 44 hours. Thereafter, the solvent was evaporated under reduced pressure and a mixed solvent of chloroform-methanol (10:1) was added. Insoluble matter was removed by filtration and the solvent was evaporated under reduced pressure, thereby obtaining a crude product. The crude product was purified by preparative thin layer chromatography (Merck silica gel 60F254, 0.5 mm, chloroform:methanol=10:1), thereby obtaining deacetyl pyripyropene E (18 mg).
ESI-MS; m/z 410 (M+H)⁺
¹H-NMR (CDCl₃) δ (ppm) 0.82 (3H, s), 0.92 (3H, s), 1.00-1.03 (1H, m), 1.04 (3H, s), 1.12 (1H, dt, J = 4.0, 13.2 Hz), 1.27 (3H, s), 1.41-1.53 (2H, m), 1.59-1.75 (3H, m), 1.80-1.84 (2H, m), 2.15 (1H, dt, J = 3.2, 12.4 Hz), 2.18-2.29 (1H, m), 2.54 (1H, dd, J = 3.2, 17.6 Hz), 3.25 (1H, dd, J = 4.4, 11.2 Hz), 6.43 (1H, s), 7.39 (1H, dd, J = 4.8, 8.0 Hz), 8.11 (1H, d, J = 8.0 Hz), 8.65 (1H, d, J = 4.8 Hz), 8.99 (1H, d, J = 1.6 Hz)

### Reference Example 3: Synthesis of 7-Deacetyl Pyripyropene A

7-deacetyl pyripyropene A was synthesized by the method described in Japanese Patent Laid-Open Publication No. 259569/1996.

### Reference Example 4: Obtainment of Pyripyropene E

Pyripyropene E was obtained by the method described in Japanese Patent Laid-Open Publication No. 239385/1996.

### Reference Example 5: Obtainment o f Pyripyropene O

Pyripyropene O was obtained by the method described in J. Antibiot. 1996, 49, 292.

### Reference Example 6: Synthesis of pyripyropene A

Pyripyropene A was obtained by the method described in WO94/09147.

### [Accession Numbers]

FERM BP-11133
FERM BP-11137
FERM BP-11141
FERM BP-11219
FERM BP-11220

## Claims

1. A method for producing a compound represented by the following formula A: 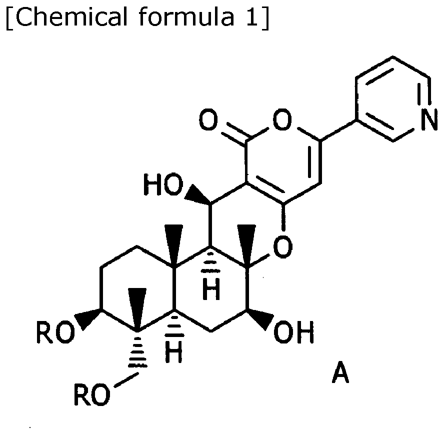 [wherein R represents a linear, branched or cyclic C₂₋₆ alkylcarbonyl group (when the alkyl moiety of this group is branched or cyclic, C₃₋₆ alkyl carbonyl group)],
the method comprising a step of culturing a microorganism into which at least one polynucleotide of (I) to (III) below or a recombinant vector comprising it/them is introduced with a compound represented by the following formula B: 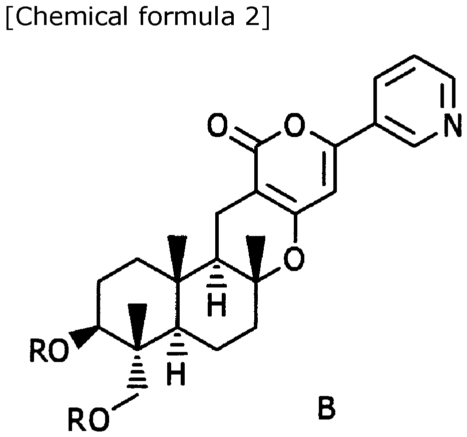 [wherein R represents the same meanings as above],
and isolating the compound represented by formula A:
(I) an isolated polynucleotide having at least one nucleotide sequence selected from the nucleotide sequences of the following (a) to (d):
(a) a nucleotide sequence of SEQ ID NO:266,
(b) a nucleotide sequence which is capable of hybridizing with a sequence complementary to the nucleotide sequence of SEQ ID NO:266 under stringent conditions, and which encodes a protein substantially equivalent to the protein encoded by the nucleotide sequence of SEQ ID NO:266,
(c) a nucleotide sequence of SEQ ID NO:266 in which one or more nucleotides are deleted, substituted, inserted or added, and which encodes a protein substantially equivalent to the protein encoded by the each nucleotide sequence, and
(d) a nucleotide sequence which has at least 90% identity to the polynucleotide of the nucleotide sequence of SEQ ID NO:266, and which encodes a protein substantially equivalent to the protein encoded by the nucleotide sequence of SEQ ID NO:266;
(II) an isolated polynucleotide having a nucleotide sequence which encodes at least one amino acid sequence selected from SEQ ID NOs:269 and 270 or amino acid sequence substantially equivalent thereto; and
(III) an isolated polynucleotide having at least one nucleotide sequence selected from the nucleotide sequences of the following (1) to (4):
(1) a nucleotide sequence of the following (a) and (b):
(a) a nucleotide sequence from 13266 to 15144 of the nucleotide sequence shown in SEQ ID NO:266, and
(b) a nucleotide sequence from 16220 to 18018 of the nucleotide sequence shown in SEQ ID NO:266,
(2) a nucleotide sequence which is capable of hybridizing with a sequence complementary to the nucleotide sequence of (1) under stringent conditions, and which encodes a protein substantially equivalent to the protein encoded by the each nucleotide sequence,
(3) a nucleotide sequence of (1) in which one or more nucleotides are deleted, substituted, inserted or added, which encodes a protein substantially equivalent to the protein encoded by the each nucleotide sequence, and
(4) a nucleotide sequence which has at least 90% identity to the polynucleotide of the nucleotide sequence of (1), and which encodes a protein substantially equivalent to the protein encoded by the each nucleotide sequence.

2. The production method according to claim 1, wherein the compound represented by formula B according to claim 1 is a compound represented by the following formula B1:

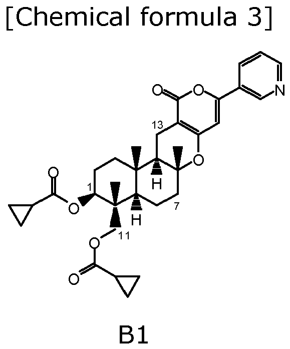

3. The production method according to claim 1, comprising a step of culturing a microorganism which comprises plasmid pCC1-PP1, plasmid pPP2 or plasmid pPP3, or one or more vectors selected from the group consisting of these plasmids with a compound represented by formula B according to claim 1 and isolating a compound represented by formula A according to claim 1.

4. The production method according to claim 1 or 2, comprising a step of culturing a microorganism into which at least one polynucleotide of (IV) to (V) below or a recombinant vector comprising it/them is introduced with a compound represented by formula B according to claim 1 and isolating a compound represented by formula A according to claim 1:
(IV) an isolated polynucleotide having a nucleotide sequence which encodes the amino acid sequence of SEQ ID NO:270 or an amino acid sequence substantially equivalent thereto; and
(V) an isolated polynucleotide having at least one nucleotide sequence selected from the nucleotide sequences of the following (1) to (4):
(1) a nucleotide sequence of the following (a),
(a) a nucleotide sequence from 16220 to 18018 of the nucleotide sequence shown in SEQ ID NO:266,
(2) a nucleotide sequence which is capable of hybridizing with a sequence complementary to the nucleotide sequence of (1) under stringent conditions, and which encodes a protein substantially equivalent to the protein encoded by the each nucleotide sequence,
(3) a nucleotide sequence of (1) in which one or more nucleotides are deleted, substituted, inserted or added, which encodes a protein substantially equivalent to the protein encoded by the each nucleotide sequence, and
(4) a nucleotide sequence which has at least 90% identity to the polynucleotide of the nucleotide sequence of (1), and which encodes a protein substantially equivalent to the protein encoded by the each nucleotide sequence.

5. The production method according to any one of claims 1 to 4, further comprising a step of acylating the hydroxyl groups at the 1 and 11 positions of a compound represented by the following formula D using an acylating agent: 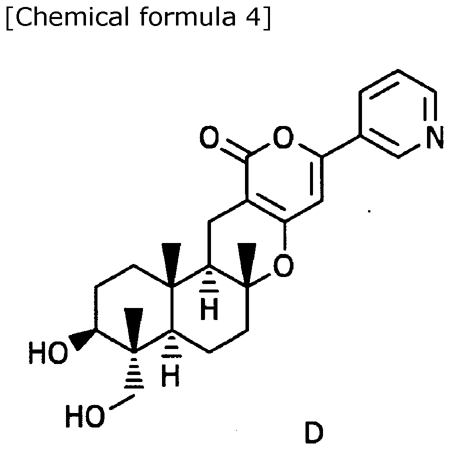 and isolating a compound represented by formula B according to claim 1.

6. The production method according to claim 5, further comprising a step of hydrolyzing the acetyl group at the 1 position and, when R' is an acetyl group, the acetyl group at the 11 position of a compound represented by the following formula C: 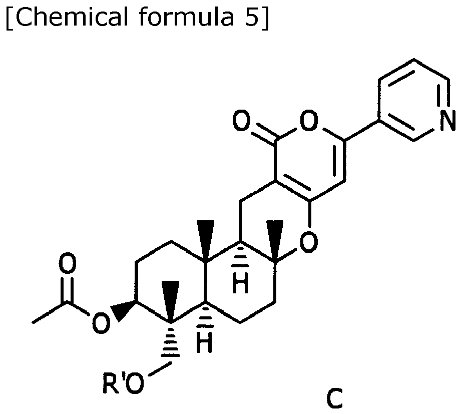 [wherein R' represents an acetyl group or a hydrogen atom],
and isolating a compound represented by formula D according to claim 5.

7. The production method according to claim 6, further comprising a step of culturing a microorganism into which at least one polynucleotide of (VI) to (VII) below or a recombinant vector comprising it/them is introduced with pyripyropene E and isolating a compound represented by formula C according to claim 6:
(VI) an isolated polynucleotide having a nucleotide sequence which encodes at least one amino acid sequence selected from SEQ ID NOs:269 and 275;
(VII) an isolated polynucleotide having at least one nucleotide sequence selected from the nucleotide sequences of the following (1) to (4):
(1) a nucleotide sequence of the following (a) and (b),
(a) a nucleotide sequence from 13266 to 15144 of the nucleotide sequence shown in SEQ ID NO:266, and
(b) a nucleotide sequence from 25824 to 27178 of the nucleotide sequence shown in SEQ ID NO:266,
(2) a nucleotide sequence which is capable of hybridizing with a sequence complementary to the nucleotide sequence of (1) under stringent conditions, and which encodes a protein substantially equivalent to the protein encoded by the each nucleotide sequence,
(3) a nucleotide sequence of (1) in which one or more nucleotides are deleted, substituted, inserted or added, which encodes a protein substantially equivalent to the protein encoded by the each nucleotide sequence, and
(4) a nucleotide sequence which has at least 90% identity to the polynucleotide of the nucleotide sequence of (1), and which encodes a protein substantially equivalent to the protein encoded by the each nucleotide sequence.

8. The production method according to claim 7, comprising a step of culturing a microorganism which comprises plasmid pPP2 or plasmid pPP9 with pyripyropene E and isolating a compound represented by formula C according to claim 6.

9. The production method according to claim 6, further comprising a step of culturing a microorganism into which at least one polynucleotide of (VIII) to (IX) below or a recombinant vector comprising it/them is introduced with deacetyl pyripyropene E and isolating a compound represented by formula C according to claim 6:
(VIII) an isolated polynucleotide having a nucleotide sequence which encodes at least one amino acid sequence selected from SEQ ID NOs:269, 274 and 275 or amino acid sequence substantially equivalent thereto; and
(IX) an isolated polynucleotide having at least one nucleotide sequence selected from the nucleotide sequences of the following (1) to (4):
(1) a nucleotide sequence of the following (a), (b) and (c):
(a) a nucleotide sequence from 13266 to 15144 of the nucleotide sequence shown in SEQ ID NO:266,
(b) a nucleotide sequence from 23205 to 24773 of the nucleotide sequence shown in SEQ ID NO:266, and
(c) a nucleotide sequence from 25824 to 27178 of the nucleotide sequence shown in SEQ ID NO:266,
(2) a nucleotide sequence which is capable of hybridizing with a sequence complementary to the nucleotide sequence of (1) under stringent conditions, and which encodes a protein substantially equivalent to the protein encoded by the each nucleotide sequence,
(3) a nucleotide sequence of (1) in which one or more nucleotides are deleted, substituted, inserted or added, which encodes a protein substantially equivalent to the protein encoded by the each nucleotide sequence, and
(4) a nucleotide sequence which has at least 90% identity to the polynucleotide of the nucleotide sequence of (1), and which encodes a protein substantially equivalent to the protein encoded by the each nucleotide sequence.

10. The production method according to claim 9, comprising a step of culturing a microorganism which comprises plasmid pPP2 or plasmid pPP9 and also comprises plasmid pPP7 with deacetyl pyripyropene E and isolating a compound represented by formula C according to claim 6.

11. Use of plasmid pCC1-PP1, plasmid pPP2, plasmid pPP3, plasmid pPP7 or plasmid pPP9, or one or more vectors selected from the group consisting of these plasmids, for producing a compound represented by formula A.

12. Use of a transformant comprising plasmid pCC1-PP1, plasmid pPP2, plasmid pPP3, plasmid pPP7 or plasmid pPP9, or one or more vector selected from the group consisting of these plasmids, for producing a compound represented by formula A.

13. A compound represented by formula B1 according to claim 2.

14. A compound represented by formula D according to claim 5.
